# EUROPEAN PATENT APPLICATION

(11) **EP 3 925 976 A1**
(43) Date of publication of application: **22.12.2021**
(21) Application number: 21164073.5
(22) Date of filing: 07.09.2018
(51) Int. Cl.: C07K 16/28, C07K 16/30, C12N 5/0783

(54) **PROTEINS BINDING NKG2D, CD16 AND A TUMOR-ASSOCIATED ANTIGEN**

(30) Priority: 07.09.2017 US 201762555110 P; 02.10.2017 US 201762566824 P
(62) Divisional of application: 18853095.0
(71) Applicant: Dragonfly Therapeutics, Inc., Waltham, MA 02451 (US)
(72) Inventor: CHANG, Gregory, Medford, MA 02155 (US); CHEUNG, Ann, Lincoln, MA 01773 (US); HANEY, William, Wayland, MA 01778 (US); LUNDE, Bradley, Lebanon, NH 03766 (US); PRINZ, Bianka, Lebanon, NH 03766 (US); GRINBERG, Asya, Lexington, MA 02421 (US)
(74) Representative: Haseltine Lake Kempner LLP

(57) **Abstract**

Multi-specific binding proteins that bind the NKG2D receptor, CD16, and a tumourassociated antigen are described, as well as pharmaceutical compositions and therapeutic methods useful for the treatment of cancer.

## Description

### CROSS-REFERENCE TO RELATED APPLICATIONS

This application claims the benefit of and priority to U.S. Provisional Patent Application No. 62/555,110, filed September 7, 2017, and U.S. Provisional Patent Application No. 62/566,824, filed on October 2, 2017, the entire disclosure of each of which is hereby incorporated by reference in its entirety for all purposes.

### SEQUENCE LISTING

The instant application contains a Sequence Listing which has been submitted electronically in ASCII format and is hereby incorporated by reference in its entirety. Said ASCII copy, created on September 6, 2018, is named DFY-038WO_SL.txt and is 321,395 bytes in size.

### FIELD OF THE INVENTION

The invention relates to multi-specific binding proteins that bind to NKG2D, CD16, and a tumor-associated antigen.

### BACKGROUND

Cancer continues to be a significant health problem despite the substantial research efforts and scientific advances reported in the literature for treating this disease. Some of the most frequently diagnosed cancers include prostate cancer, breast cancer, lung cancer, and colorectal cancer. Prostate cancer is the most common form of cancer in men. Breast cancer remains a leading cause of death in women. Blood and bone marrow cancers are also frequently diagnosed cancer types, including multiple myelomas, leukemia, and lymphomas. Current treatment options for these cancers are not effective for all patients and/or can have substantial adverse side effects. Other types of cancer also remain challenging to treat using existing therapeutic options.

Cancer immunotherapies are desirable because they are highly specific and can facilitate destruction of cancer cells using the patient's own immune system. Fusion proteins such as bi-specific T-cell engagers are cancer immunotherapies described in the literature that bind to tumor cells and T-cells to facilitate destruction of tumor cells. Antibodies that bind to certain tumor-associated antigens and to certain immune cells have been described in the literature. See, for example WO 2016/134371 and WO 2015/095412.

Natural killer (NK) cells are a component of the innate immune system and make up approximately 15% of circulating lymphocytes. NK cells infiltrate virtually all tissues and were originally characterized by their ability to kill tumor cells effectively without the need for prior sensitization. Activated NK cells kill target cells by means similar to cytotoxic T cells - *i.e.,* via cytolytic granules that contain perforin and granzymes as well as via death receptor pathways. Activated NK cells also secrete inflammatory cytokines such as IFN-gamma and chemokines that promote the recruitment of other leukocytes to the target tissue.

NK cells respond to signals through a variety of activating and inhibitory receptors on their surface. For example, when NK cells encounter healthy self-cells, their activity is inhibited through activation of the killer-cell immunoglobulin-like receptors (KIRs). Alternatively, when NK cells encounter foreign cells or cancer cells, they are activated via their activating receptors (*e.g*., Natural killer group 2 member D (NKG2D), NCRs, DNAM1). NK cells are also activated by the constant region of some immunoglobulins through CD16 receptors on their surface. The overall sensitivity of NK cells to activation depends on the sum of stimulatory and inhibitory signals.

Epithelial cell adhesion molecule (EpCAM) is a transmembrane glycoprotein mediating Ca²⁺-independent homotypic cell-cell adhesion in epithelia. EpCAM is also involved in cell signaling, migration, proliferation, and differentiation. Additionally, EpCAM has oncogenic potential via its capacity to upregulate c-myc, e-fabp, and cyclins A and E. Since EpCAM is expressed exclusively in epithelia and epithelial-derived neoplasms, EpCAM can be used as diagnostic marker for various cancers, such as head and neck cancer, ovarian cancer, bladder cancer, breast cancer, colorectal cancer, prostate cancer, gastric cancer, liver cancer, esophageal cancer, and lung cancer. It appears to play a role in tumorigenesis and metastasis of carcinomas, so it can also act as a potential prognostic marker and as a potential target for immunotherapeutic strategies.

CA125, also known as mucin 16, is a member of the mucin family glycoproteins. CA-125 has found application as a tumor marker or biomarker that may be elevated in the blood of some patients with specific types of cancers, including ovarian cancer, endometrial cancer, and pancreatic cancer. CA-125 has been shown to play a role in advancing tumorigenesis and tumor proliferation by several different mechanisms, including suppressing the response of natural killer cells, and thereby protecting cancer cells from the immune response; and by enabling cell growth and promoting cell motility.

Sodium-dependent phosphate transport protein 2b (NaPi2b) is involved in actively transporting phosphate into cells via Na+ co-transport. For example, it is the main phosphate transport protein in the intestinal brush border membrane, and has a role in the synthesis of surfactant in lungs' alveoli. NaPi2b is also an antigen expressed in a variety of cancer, such as lung cancer, ovarian cancer, and thyroid cancer.

Nectin4 is a member of the Nectin family, which is a family of cellular adhesion molecules involved in Ca²⁺-independent cellular adhesion. Nectins are ubiquitously expressed and have adhesive roles in a wide range of tissues such as the adherens junction of epithelia or the chemical synapse of the neuronal tissue. It is also a tumor associated antigen, and expressed in cancers such as bladder cancer, breast cancer, ovarian cancer, pancreatic cancer, colorectal cancer, and lung cancer.

Gangliosides have been implicated in many physiological processes, including growth, differentiation, migration, and apoptosis through modulating both cell signaling processes and cell-to-cell and cell-to-matrix interactions. GM1 is a ganglioside, and Fucosyl-GM1 is a ganglioside with a unique structure in which the terminal galactose is α-1,2-fucosylated at the non-reducing end. It is expressed in very few normal tissues but occurs in a variety of cancers such as in small cell lung cancer, neuroblastoma, liver cancer. Consequently, fucosyl-GM1 has been considered to be a candidate as a tumor marker and target antigen in antibody immunotherapy small cell lung cancer, neuroblastoma, liver cancer.

ADAM (a disintegrin and metalloproteinase) proteins have a predominant role in the protein ectodomain shedding of membrane-bound molecules. They have emerged as critical regulators of cell-cell signaling during development and homeostasis, and are believed to contribute to pathologies, such as cancer, where their regulation is altered. ADAM8, a member the ADAM family, is overexpressed in pancreatic cancer, breast cancer, lung cancer, and renal cancer. ADAM9 has been shown to cleave and release a number of molecules with important roles in tumorigenesis and angiogenesis, such as EGF, FGFR2iiib, Tie-2, Flk-1, EphB4, CD40, VCAM-1, and VE-cadherin. ADAM9 is overexpressed in renal cancer, breast cancer, lung cancer, liver cancer, pancreatic cancer, melanoma, cervical cancer, prostate cancer, osteosarcoma, and brain cancer.

SLC44A4, also known as CTL4, is a member of the family of solute carrier proteins known as choline transporter-like proteins (CTL1-5). SLC44A4 has not been shown to be involved in choline transport, but it has been linked with acetylcholine synthesis and transport as well as uptake of thiamine pyrophosphate, the phosphorylated form of vitamin B1. SLC44A4 is normally expressed on the apical surface of secretory epithelial cells, but it is markedly upregulated in a variety of epithelial tumors, most notably pancreatic cancer, prostate cancer, and gastric cancer.

CA19-9 is the common term for carbohydrate antigen sialyl Lewis a. It is overexpressed in cancer of the digestive organs such as pancreatic cancer, colorectal cancer, cholangiocarcinoma, and liver cancer. Therefore, it is the most frequently applied serum tumor marker for diagnosis of these above mentioned cancers.

### SUMMARY

The invention provides multi-specific binding proteins that bind to a tumor-associated antigen (selected from any one of the antigens provided in Table 11) and to the NKG2D receptor and CD16 receptor on natural killer cells. Such proteins can engage more than one kind of NK activating receptor, and may block the binding of natural ligands to NKG2D. In certain embodiments, the proteins can agonize NK cells in humans, and in other species such as rodents and cynomolgus monkeys. Various aspects and embodiments of the invention are described in further detail below.

Accordingly, one aspect of the invention provides a protein that incorporates a first antigen-binding site that binds NKG2D; a second antigen-binding site that binds an antigen selected from EpCAM, Cancer Antigen 125 (CA125), sodium/phosphate cotransporter 2B (NaPi2b), Nectin cell adhesion molecule 4 (Nectin4), Fucosyl-GM1 (monosialotetrahexosylganglioside), disintegrin and metalloproteinase domain-containing protein 8 (ADAM8), disintegrin and metalloproteinase domain-containing protein 9 (ADAM9), solute carrier family 44 member 4 (SLC44A4), and sialylated Lewis a antigen (CA19-9); and an antibody Fc domain, a portion thereof sufficient to bind CD16, or a third antigen-binding site that binds CD16. The antigen-binding sites may each incorporate an antibody heavy chain variable domain and an antibody light chain variable domain *(e.g.* arranged as in an antibody, or fused together to from an scFv), or one or more of the antigen-binding sites may be a single domain antibody, such as a V_{H}H antibody like a camelid antibody or a V_{NAR} antibody like those found in cartilaginous fish.

The invention provides multi-specific binding proteins that bind the NKG2D receptor, CD16, and an antigen selected from EpCAM, Cancer Antigen 125 (CA125), sodium/phosphate cotransporter 2B (NaPi2b), Nectin cell adhesion molecule 4 (Nectin4), Fucosyl-GM1 (monosialotetrahexosylganglioside), disintegrin and metalloproteinase domain-containing protein 8 (ADAM8), disintegrin and metalloproteinase domain-containing protein 9 (ADAM9), solute carrier family 44 member 4 (SLC44A4), and sialylated Lewis a antigen (CA19-9).

Some proteins of the present disclosure include an Fab fragment linked to the antibody Fc domain or a portion thereof sufficient to bind CD 16, or the third antigen-binding site that binds CD16.

Some proteins of the present disclosure include an Fab fragment, wherein the heavy chain portion of the Fab fragment comprises a heavy chain variable domain and a CH1 domain, and wherein the heavy chain variable domain is linked to the CH1 domain.

Some proteins of the present disclosure include an Fab fragment linked to the antibody Fc domain.

In one aspect, the invention provides a protein comprising (a) a first antigen-binding site comprising an Fab fragment that binds NKG2D; (b) a second antigen-binding site comprising a single-chain variable fragment (scFv) that binds EpCAM; and (c) an antibody Fc domain or a portion thereof sufficient to bind CD 16, or a third antigen-binding site that binds CD16. The present invention provides a protein in which the first antigen-binding site that binds NKG2D is an Fab fragment, and the second antigen-binding site that binds a tumor-associated antigen EpCAM is an scFv.

Certain proteins described in the present disclosure include an scFv-targeting EpCAM, comprising a heavy chain variable domain and a light chain variable domain, linked to an antibody Fc domain or a portion thereof sufficient to bind CD16, or the third antigen-binding site that binds CD16, via a hinge comprising Ala-Ser or Gly-Ala-Ser. Some proteins of the present disclosure includes an scFv-targeting EpCAM linked to an antibody Fc domain. Some proteins of the present disclosure includes a heavy chain variable domain of an scFv-targeting EpCAM, which forms a disulfide bridge with the light chain variable domain of the scFv.

Some proteins of the present disclosure include an scFv-targeting EpCAM, in which a disulfide bridge is formed between C44 from the heavy chain variable domain and C100 from the light chain variable domain.

Some proteins of the present disclosure include an scFv-targeting EpCAM linked to an antibody Fc domain, in which the light chain variable domain of the scFv is positioned at the N-terminus of the heavy chain variable domain of the scFv, and is linked to the heavy chain variable domain of the scFv via a flexible linker (GlyGlyGlyGlySer)₄(G4S)₄) (SEQ ID NO:206), and the Fab fragment that binds NKG2D is linked to the antibody Fc domain.

Some proteins of the present disclosure include an scFv-targeting EpCAM in which the heavy chain variable domain is positioned at the N-terminus or the C-terminus of the light chain variable domain of the scFv.

Some proteins of the present disclosure include an scFv-targeting EpCAM in which the light chain variable domain is positioned at the N-terminus of the heavy chain variable domain of the scFv.

In one aspect of the invention provides a protein comprising (a) a first antigen-binding site comprising a single-chain variable fragment (scFv) that binds NKG2D; (b) a second antigen-binding site that binds EpCAM; and (c) an antibody Fc domain or a portion thereof sufficient to bind CD 16, or a third antigen-binding site that binds CD 16. In certain embodiments, a protein of the present disclosure further comprises an additional antigen-binding site that binds EpCAM. In certain embodiments, the second antigen-binding site of a protein described in the present disclosure is an Fab fragment that binds EpCAM. In certain embodiments, the second and the additional antigen-binding site of a protein described in the present disclosure are Fab fragments that bind EpCAM.

In certain embodiments, the second and the additional antigen-binding site of a protein described in the present disclosure are scFvs that bind EpCAM. In certain embodiments, the heavy chain variable domain of the scFv that binds NKG2D is positioned at the N-terminus or the C-terminus of the light chain variable domain of the scFv. In certain embodiments, the light chain variable domain is positioned at the N-terminus of the heavy chain variable domain of the scFv that binds NKG2D.

In certain embodiments, the scFv that binds to NKG2D is linked to the antibody Fc domain or a portion thereof sufficient to bind CD16, or a third antigen-binding site that binds CD16. In certain embodiments, the scFv that binds to NKG2D is linked to the antibody Fc domain or a portion thereof sufficient to bind CD16, or a third antigen-binding site that binds CD16 *via* a hinge comprising Ala-Ser *(e.g.,* in a TriNKET that comprises an additional antigen-binding site that binds EpCAM, CA125, NaPi2b, Nectin4, Fucosyl-GM1, ADAM8, ADAM9, SLC44A4, or CA19-9) or Gly-Ala-Ser (*e.g.*, in a TriNKET that does not comprise an additional antigen-binding site that binds EpCAM, CA125, NaPi2b, Nectin4, Fucosyl-GM1, ADAM8, ADAM9, SLC44A4, or CA19-9). In certain embodiments, the scFv that binds to NKG2D is linked to the C-terminus of the antibody Fc domain or a portion thereof sufficient to bind CD 16, or a third antigen-binding site that binds CD 16 *via* a flexible linker comprising G4S. In certain embodiments, the C-terminus of the antibody Fc domain is linked to the N-terminus of the light chain variable domain of the scFv that binds NKG2D.

In certain embodiments, within the scFv that binds NKG2D, a disulfide bridge is formed between the heavy chain variable domain of the scFv and the light chain variable domain of the scFv. In certain embodiments, the disulfide bridge is formed between C44 from the heavy chain variable domain and C100 from the light chain variable domain.

Some proteins of the present disclosure include a sequence selected from SEQ ID NO:210 and SEQ ID NO:211.

Some proteins of the present disclosure include an scFv linked to an antibody Fc domain, wherein the scFv linked to the antibody Fc domain is represented by a sequence selected from SEQ ID NO:208 and SEQ ID NO:209.

Some proteins of the present disclosure include a sequence of SEQ ID NO:205 and SEQ ID NO:213.

Some proteins of the present disclosure include a sequence at least 90% identical to an amino acid sequence selected from SEQ ID NO:210 and SEQ ID NO:211.

Some proteins of the present disclosure include a sequence at least 95% identical to an amino acid sequence selected from SEQ ID NO:210 and SEQ ID NO:211.

Some proteins of the present disclosure include a sequence at least 99% identical to an amino acid sequence selected from SEQ ID NO:210 and SEQ ID NO:211.

Some proteins of the present disclosure include an amino acid sequence of SEQ ID NO:203.

Some proteins of the present disclosure include an amino acid sequence of SEQ ID NO:203 and SEQ ID NO:204.

Some proteins of the present disclosure include an amino acid sequence at least 90% identical to an amino acid sequence of SEQ ID NO:203. Some proteins of the present disclosure include an amino acid sequence at least 95% identical to an amino acid sequence of SEQ ID NO:203. Some proteins of the present disclosure include an amino acid sequence at least 99% identical to an amino acid sequence of SEQ ID NO:203.

The first antigen-binding site, which binds to NKG2D, in some embodiments, can incorporate a heavy chain variable domain related to SEQ ID NO:1, such as by having an amino acid sequence at least 90% *(e.g.,* 90%, 91%, 92%, 93%, 94%, 95%, 96%, 97%, 98%, 99%, or 100%) identical to SEQ ID NO:1, and/or incorporating amino acid sequences identical to the CDR1 (SEQ ID NO:105), CDR2 (SEQ ID NO:106), and CDR3 (SEQ ID NO: 107) sequences of SEQ ID NO:1. The heavy chain variable domain related to SEQ ID NO: 1 can be coupled with a variety of light chain variable domains to form an NKG2D binding site. For example, the first antigen-binding site that incorporates a heavy chain variable domain related to SEQ ID NO: 1 can further incorporate a light chain variable domain selected from any one of the sequences related to SEQ ID NOs:2, 4, 6, 8, 10, 12, 14, 16, 18, 20, 22, 24, 26, 28, 30, 32, 34, 36, 38, and 40. For example, the first antigen-binding site incorporates a heavy chain variable domain with amino acid sequences at least 90% (*e.g.,* 90%, 91%, 92%, 93%, 94%, 95%, 96%, 97%, 98%, 99%, or 100%) identical to SEQ ID NO: 1 and a light chain variable domain with amino acid sequences at least 90% (*e.g.*, 90%, 91%, 92%, 93%, 94%, 95%, 96%, 97%, 98%, 99%, or 100%) identical to any one of the sequences selected from SEQ ID NOs:2, 4, 6, 8, 10, 12, 14, 16, 18, 20, 22, 24, 26, 28, 30, 32, 34, 36, 38, and 40.

Alternatively, the first antigen-binding site can incorporate a heavy chain variable domain related to SEQ ID NO:41 and a light chain variable domain related to SEQ ID NO:42. For example, the heavy chain variable domain of the first antigen-binding site can be at least 90% *(e.g.,* 90%, 91%, 92%, 93%, 94%, 95%, 96%, 97%, 98%, 99%, or 100%) identical to SEQ ID NO:41, and/or incorporate amino acid sequences identical to the CDR1 (SEQ ID NO:43), CDR2 (SEQ ID NO:44), and CDR3 (SEQ ID NO:45) sequences of SEQ ID NO:41. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO:42, and/or incorporate amino acid sequences identical to the CDR1 (SEQ ID NO:46), CDR2 (SEQ ID NO:47), and CDR3 (SEQ ID NO:48) sequences of SEQ ID NO:42.

In other embodiments, the first antigen-binding site can incorporate a heavy chain variable domain related to SEQ ID NO:49 and a light chain variable domain related to SEQ ID NO:50. For example, the heavy chain variable domain of the first antigen-binding site can be at least 90% *(e.g.,* 90%, 91%, 92%, 93%, 94%, 95%, 96%, 97%, 98%, 99%, or 100%) identical to SEQ ID NO:49, and/or incorporate amino acid sequences identical to the CDR1 (SEQ ID NO:51), CDR2 (SEQ ID NO:52), and CDR3 (SEQ ID NO:53) sequences of SEQ ID NO:49. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO:50, and/or incorporate amino acid sequences identical to the CDR1 (SEQ ID NO:54), CDR2 (SEQ ID NO:55), and CDR3 (SEQ ID NO:56) sequences of SEQ ID NO:50.

Alternatively, the first antigen-binding site can incorporate a heavy chain variable domain related to SEQ ID NO:57 and a light chain variable domain related to SEQ ID NO:58, such as by having amino acid sequences at least 90% *(e.g.,* 90%, 91%, 92%, 93%, 94%, 95%, 96%, 97%, 98%, 99%, or 100%) identical to SEQ ID NO:57 and at least 90% (*e.g.,* 90%, 91%, 92%, 93%, 94%, 95%, 96%, 97%, 98%, 99%, or 100%) identical to SEQ ID NO:58, respectively.

In another embodiment, the first antigen-binding site can incorporate a heavy chain variable domain related to SEQ ID NO:59 and a light chain variable domain related to SEQ ID NO:60, For example, the heavy chain variable domain of the first antigen-binding site can be at least 90% *(e.g.,* 90%, 91%, 92%, 93%, 94%, 95%, 96%, 97%, 98%, 99%, or 100%) identical to SEQ ID NO:59, and/or incorporate amino acid sequences identical to the CDR1 (SEQ ID NO: 109), CDR2 (SEQ ID NO: 110), and CDR3 (SEQ ID NO:111) sequences of SEQ ID NO:59. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO:60, and/or incorporate amino acid sequences identical to the CDR1 (SEQ ID NO: 112), CDR2 (SEQ ID NO:113), and CDR3 (SEQ ID NO:114) sequences of SEQ ID NO:60.

The first antigen-binding site, which binds to NKG2D, in some embodiments, can incorporate a heavy chain variable domain related to SEQ ID NO:61 and a light chain variable domain related to SEQ ID NO:62. For example, the heavy chain variable domain of the first antigen-binding site can be at least 90% *(e.g.,* 90%, 91%, 92%, 93%, 94%, 95%, 96%, 97%, 98%, 99%, or 100%) identical to SEQ ID NO:61, and/or incorporate amino acid sequences identical to the CDR1 (SEQ ID NO:63), CDR2 (SEQ ID NO:64), and CDR3 (SEQ ID NO:65) sequences of SEQ ID NO:61. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO:62, and/or incorporate amino acid sequences identical to the CDR1 (SEQ ID NO:66), CDR2 (SEQ ID NO:67), and CDR3 (SEQ ID NO:68) sequences of SEQ ID NO:62.

In some embodiments, the first antigen-binding site can incorporate a heavy chain variable domain related to SEQ ID NO:69 and a light chain variable domain related to SEQ ID NO:70. For example, the heavy chain variable domain of the first antigen-binding site can be at least 90% *(e.g.,* 90%, 91%, 92%, 93%, 94%, 95%, 96%, 97%, 98%, 99%, or 100%) identical to SEQ ID NO:69, and/or incorporate amino acid sequences identical to the CDR1 (SEQ ID NO:71), CDR2 (SEQ ID NO:72), and CDR3 (SEQ ID NO:73) sequences of SEQ ID NO:69. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO:70, and/or incorporate amino acid sequences identical to the CDR1 (SEQ ID NO:74), CDR2 (SEQ ID NO:75), and CDR3 (SEQ ID NO:76) sequences of SEQ ID NO:70.

In some embodiments, the first antigen-binding site can incorporate a heavy chain variable domain related to SEQ ID NO:77 and a light chain variable domain related to SEQ ID NO:78. For example, the heavy chain variable domain of the first antigen-binding site can be at least 90% *(e.g.,* 90%, 91%, 92%, 93%, 94%, 95%, 96%, 97%, 98%, 99%, or 100%) identical to SEQ ID NO:77, and/or incorporate amino acid sequences identical to the CDR1 (SEQ ID NO:79), CDR2 (SEQ ID NO:80), and CDR3 (SEQ ID NO:81) sequences of SEQ ID NO:77. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO:78, and/or incorporate amino acid sequences identical to the CDR1 (SEQ ID NO:82), CDR2 (SEQ ID NO:83), and CDR3 (SEQ ID NO:84) sequences of SEQ ID NO:78.

In some embodiments, the first antigen-binding site can incorporate a heavy chain variable domain related to SEQ ID NO:85 and a light chain variable domain related to SEQ ID NO:86. For example, the heavy chain variable domain of the first antigen-binding site can be at least 90% *(e.g.,* 90%, 91%, 92%, 93%, 94%, 95%, 96%, 97%, 98%, 99%, or 100%) identical to SEQ ID NO:85, and/or incorporate amino acid sequences identical to the CDR1 (SEQ ID NO:87), CDR2 (SEQ ID NO:88), and CDR3 (SEQ ID NO:89) sequences of SEQ ID NO:85. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO:86, and/or incorporate amino acid sequences identical to the CDR1 (SEQ ID NO:90), CDR2 (SEQ ID NO:91), and CDR3 (SEQ ID NO:92) sequences of SEQ ID NO:86.

In some embodiments, the first antigen-binding site can incorporate a heavy chain variable domain related to SEQ ID NO:93 and a light chain variable domain related to SEQ ID NO:94. For example, the heavy chain variable domain of the first antigen-binding site can be at least 90% *(e.g.,* 90%, 91%, 92%, 93%, 94%, 95%, 96%, 97%, 98%, 99%, or 100%) identical to SEQ ID NO:93, and/or incorporate amino acid sequences identical to the CDR1 (SEQ ID NO:95), CDR2 (SEQ ID NO:96), and CDR3 (SEQ ID NO:97) sequences of SEQ ID NO:93. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO:94, and/or incorporate amino acid sequences identical to the CDR1 (SEQ ID NO:98), CDR2 (SEQ ID NO:99), and CDR3 (SEQ ID NO:100) sequences of SEQ ID NO:94.

In some embodiments, the first antigen-binding site can incorporate a heavy chain variable domain related to SEQ ID NO:101 and a light chain variable domain related to SEQ ID NO:102, such as by having amino acid sequences at least 90% *(e.g.,* 90%, 91%, 92%, 93%, 94%, 95%, 96%, 97%, 98%, 99%, or 100%) identical to SEQ ID NO:101 and at least 90% *(e.g.,* 90%, 91%, 92%, 93%, 94%, 95%, 96%, 97%, 98%, 99%, or 100%) identical to SEQ ID NO:102, respectively.

In some embodiments, the first antigen-binding site can incorporate a heavy chain variable domain related to SEQ ID NO:103 and a light chain variable domain related to SEQ ID NO:104, such as by having amino acid sequences at least 90% *(e.g.,* 90%, 91%, 92%, 93%, 94%, 95%, 96%, 97%, 98%, 99%, or 100%) identical to SEQ ID NO:103 and at least 90% *(e.g.,* 90%, 91%, 92%, 93%, 94%, 95%, 96%, 97%, 98%, 99%, or 100%) identical to SEQ ID NO:104, respectively.

In some embodiments, the second antigen-binding site can bind to EpCAM and can incorporate a heavy chain variable domain related to SEQ ID NO:115 and a light chain variable domain related to SEQ ID NO:119. For example, the heavy chain variable domain of the second antigen-binding site can be at least 90% *(e.g.,* 90%, 91%, 92%, 93%, 94%, 95%, 96%, 97%, 98%, 99%, or 100%) identical to SEQ ID NO:115, and/or incorporate amino acid sequences identical to the CDR1 (SEQ ID NO: 116), CDR2 (SEQ ID NO:117), and CDR3 (SEQ ID NO: 118) sequences of SEQ ID NO:115. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO:119, and/or incorporate amino acid sequences identical to the CDR1 (SEQ ID NO:120), CDR2 (SEQ ID NO:121), and CDR3 (SEQ ID NO: 122) sequences of SEQ ID NO:119.

In some embodiments, the second antigen-binding site can bind to EpCAM and can incorporate a heavy chain variable domain related to SEQ ID NO:123 and a light chain variable domain related to SEQ ID NO:127. For example, the heavy chain variable domain of the second antigen-binding site can be at least 90% *(e.g.,* 90%, 91%, 92%, 93%, 94%, 95%, 96%, 97%, 98%, 99%, or 100%) identical to SEQ ID NO:123, and/or incorporate amino acid sequences identical to the CDR1 (SEQ ID NO: 124), CDR2 (SEQ ID NO:125), and CDR3 (SEQ ID NO: 126) sequences of SEQ ID NO:123. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO:127, and/or incorporate amino acid sequences identical to the CDR1 (SEQ ID NO:128), CDR2 (SEQ ID NO:129), and CDR3 (SEQ ID NO: 130) sequences of SEQ ID NO:127.

In some embodiments, the second antigen-binding site can bind to EpCAM and can incorporate a heavy chain variable domain related to SEQ ID NO:131 and a light chain variable domain related to SEQ ID NO:135. For example, the heavy chain variable domain of the second antigen-binding site can be at least 90% *(e.g.,* 90%, 91%, 92%, 93%, 94%, 95%, 96%, 97%, 98%, 99%, or 100%) identical to SEQ ID NO:131, and/or incorporate amino acid sequences identical to the CDR1 (SEQ ID NO: 132), CDR2 (SEQ ID NO:133), and CDR3 (SEQ ID NO: 134) sequences of SEQ ID NO:131. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO:135, and/or incorporate amino acid sequences identical to the CDR1 (SEQ ID NO:136), CDR2 (SEQ ID NO:137), and CDR3 (SEQ ID NO: 138) sequences of SEQ ID NO:135.

In some embodiments, the second antigen-binding site can bind to EpCAM and can incorporate a heavy chain variable domain related to SEQ ID NO:139 and a light chain variable domain related to SEQ ID NO:143. For example, the heavy chain variable domain of the second antigen-binding site can be at least 90% *(e.g.,* 90%, 91%, 92%, 93%, 94%, 95%, 96%, 97%, 98%, 99%, or 100%) identical to SEQ ID NO:139, and/or incorporate amino acid sequences identical to the CDR1 (SEQ ID NO: 140), CDR2 (SEQ ID NO:141), and CDR3 (SEQ ID NO: 142) sequences of SEQ ID NO:139. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO:143, and/or incorporate amino acid sequences identical to the CDR1 (SEQ ID NO:144), CDR2 (SEQ ID NO:145), and CDR3 (SEQ ID NO: 146) sequences of SEQ ID NO:143.

In some embodiments, the second antigen-binding site can bind to CA125 and can incorporate a heavy chain variable domain related to SEQ ID NO:155 and a light chain variable domain related to SEQ ID NO:159. For example, the heavy chain variable domain of the second antigen-binding site can be at least 90% *(e.g.,* 90%, 91%, 92%, 93%, 94%, 95%, 96%, 97%, 98%, 99%, or 100%) identical to SEQ ID NO: 155, and/or incorporate amino acid sequences identical to the CDR1 (SEQ ID NO:156), CDR2 (SEQ ID NO:157), and CDR3 (SEQ ID NO: 158) sequences of SEQ ID NO: 155. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO:159, and/or incorporate amino acid sequences identical to the CDR1 (SEQ ID NO: 160), CDR2 (SEQ ID NO: 161), and CDR3 (SEQ ID NO: 162) sequences of SEQ ID NO:159.

In some embodiments, the second antigen-binding site can bind to CA125 and can incorporate a heavy chain variable domain related to SEQ ID NO: 163 and a light chain variable domain related to SEQ ID NO:167. For example, the heavy chain variable domain of the second antigen-binding site can be at least 90% *(e.g.,* 90%, 91%, 92%, 93%, 94%, 95%, 96%, 97%, 98%, 99%, or 100%) identical to SEQ ID NO: 163, and/or incorporate amino acid sequences identical to the CDR1 (SEQ ID NO: 164), CDR2 (SEQ ID NO: 165), and CDR3 (SEQ ID NO: 166) sequences of SEQ ID NO: 163. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO: 167, and/or incorporate amino acid sequences identical to the CDR1 (SEQ ID NO: 168), CDR2 (SEQ ID NO: 169), and CDR3 (SEQ ID NO: 170) sequences of SEQ ID NO: 167.

In some embodiments, the second antigen-binding site can bind to NaPi2b and can incorporate a heavy chain variable domain related to SEQ ID NO: 171 and a light chain variable domain related to SEQ ID NO: 175. For example, the heavy chain variable domain of the second antigen-binding site can be at least 90% *(e.g.,* 90%, 91%, 92%, 93%, 94%, 95%, 96%, 97%, 98%, 99%, or 100%) identical to SEQ ID NO: 171, and/or incorporate amino acid sequences identical to the CDR1 (SEQ ID NO: 172), CDR2 (SEQ ID NO: 173), and CDR3 (SEQ ID NO: 174) sequences of SEQ ID NO: 171. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO: 175, and/or incorporate amino acid sequences identical to the CDR1 (SEQ ID NO: 176), CDR2 (SEQ ID NO: 177), and CDR3 (SEQ ID NO: 178) sequences of SEQ ID NO: 175.

In some embodiments, the second antigen-binding site can bind to Nectin4 and can incorporate a heavy chain variable domain related to SEQ ID NO: 179 and a light chain variable domain related to SEQ ID NO:183. For example, the heavy chain variable domain of the second antigen-binding site can be at least 90% *(e.g.,* 90%, 91%, 92%, 93%, 94%, 95%, 96%, 97%, 98%, 99%, or 100%) identical to SEQ ID NO: 179, and/or incorporate amino acid sequences identical to the CDR1 (SEQ ID NO: 180), CDR2 (SEQ ID NO: 181), and CDR3 (SEQ ID NO: 182) sequences of SEQ ID NO: 179. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO: 183, and/or incorporate amino acid sequences identical to the CDR1 (SEQ ID NO: 184), CDR2 (SEQ ID NO: 185), and CDR3 (SEQ ID NO: 186) sequences of SEQ ID NO: 183.

In some embodiments, the second antigen-binding site can bind to fucosyl-GM1 and can incorporate a heavy chain variable domain related to SEQ ID NO: 187 and a light chain variable domain related to SEQ ID NO: 191. For example, the heavy chain variable domain of the second antigen-binding site can be at least 90% *(e.g.,* 90%, 91%, 92%, 93%, 94%, 95%, 96%, 97%, 98%, 99%, or 100%) identical to SEQ ID NO: 187, and/or incorporate amino acid sequences identical to the CDR1 (SEQ ID NO: 188), CDR2 (SEQ ID NO: 189), and CDR3 (SEQ ID NO: 190) sequences of SEQ ID NO: 187. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO:191, and/or incorporate amino acid sequences identical to the CDR1 (SEQ ID NO: 192), CDR2 (SEQ ID NO: 193), and CDR3 (SEQ ID NO: 194) sequences of SEQ ID NO: 191.

In some embodiments, the second antigen-binding site can bind to SLC44A4 and can incorporate a heavy chain variable domain related to SEQ ID NO: 195 and a light chain variable domain related to SEQ ID NO:199. For example, the heavy chain variable domain of the second antigen-binding site can be at least 90% *(e.g.,* 90%, 91%, 92%, 93%, 94%, 95%, 96%, 97%, 98%, 99%, or 100%) identical to SEQ ID NO: 195, and/or incorporate amino acid sequences identical to the CDR1 (SEQ ID NO: 196), CDR2 (SEQ ID NO: 197), and CDR3 (SEQ ID NO: 198) sequences of SEQ ID NO: 195. Similarly, the light chain variable domain of the second antigen-binding site can be at least 90% *(e.g.,* 90%, 91%, 92%, 93%, 94%, 95%, 96%, 97%, 98%, 99%, or 100%) identical to SEQ ID NO: 199, and/or incorporate amino acid sequences identical to the CDR1 (SEQ ID NO:200), CDR2 (SEQ ID NO:201), and CDR3 (SEQ ID NO:202) sequences of SEQ ID NO:199.

In some embodiments, the second antigen binding site incorporates a light chain variable domain having an amino acid sequence identical to the amino acid sequence of the light chain variable domain present in the first antigen binding site.

In some embodiments, the protein incorporates a portion of an antibody Fc domain sufficient to bind CD16, wherein the antibody Fc domain comprises hinge and CH2 domains, and/or amino acid sequences at least 90% identical to amino acid sequence 234-332 of a human IgG antibody.

Some proteins of the present disclosure bind to NKG2D with a K_{D} of 10 nM or weaker affinity.

Formulations containing one of these proteins; cells containing one or more nucleic acids expressing these proteins, and methods of enhancing tumor cell death using these proteins are also provided.

Another aspect of the invention provides a method of treating cancer in a patient. The method comprises administering to a patient in need thereof a therapeutically effective amount of the multi-specific binding protein described herein. Exemplary cancers for treatment using the multi-specific binding proteins include, for example, head and neck cancer, ovarian cancer, bladder cancer, breast cancer, colorectal cancer, prostate cancer, gastric cancer, liver cancer, esophageal cancer, and lung cancer.

### BRIEF DESCRIPTION OF THE DRAWINGS

**FIG. 1** is a representation of a heterodimeric, multi-specific antibody (a trispecific binding protein (TriNKET)). Each arm can represent either the NKG2D-binding domain, or the tumor associated antigen-binding domain. In some embodiments, the NKG2D- and the tumor associated antigen- binding domains can share a common light chain.
**FIG. 2** is a representation of a heterodimeric, multi-specific antibody. Either the NKG2D-binding domain or the tumor associated antigen-binding domain can take the scFv format (right arm).
**FIG. 3** are line graphs demonstrating the binding affinity of NKG2D-binding domains (listed as clones) to human recombinant NKG2D in an ELISA assay.
**FIG. 4** are line graphs demonstrating the binding affinity of NKG2D-binding domains (listed as clones) to cynomolgus recombinant NKG2D in an ELISA assay.
**FIG. 5** are line graphs demonstrating the binding affinity of NKG2D-binding domains (listed as clones) to mouse recombinant NKG2D in an ELISA assay.
**FIG. 6** are bar graphs demonstrating the binding of NKG2D-binding domains (listed as clones) to EL4 cells expressing human NKG2D by flow cytometry showing mean fluorescence intensity (MFI) fold over background (FOB).
**FIG. 7** are bar graphs demonstrating the binding of NKG2D-binding domains (listed as clones) to EL4 cells expressing mouse NKG2D by flow cytometry showing mean fluorescence intensity (MFI) fold over background (FOB).
**FIG. 8** are line graphs demonstrating specific binding affinity of NKG2D-binding domains (listed as clones) to recombinant human NKG2D-Fc by competing with natural ligand ULBP-6.
**FIG. 9** are line graphs demonstrating specific binding affinity of NKG2D-binding domains (listed as clones) to recombinant human NKG2D-Fc by competing with natural ligand MICA.
**FIG. 10** are line graphs demonstrating specific binding affinity of NKG2D-binding domains (listed as clones) to recombinant mouse NKG2D-Fc by competing with natural ligand Rae-1 delta.
**FIG. 11** are bar graphs showing activation of human NKG2D by NKG2D-binding domains (listed as clones) by quantifying the percentage of TNF-α positive cells, which express human NKG2D-CD3 zeta fusion proteins.
**FIG. 12** are bar graphs showing activation of mouse NKG2D by NKG2D-binding domains (listed as clones) by quantifying the percentage of TNF-α positive cells, which express mouse NKG2D-CD3 zeta fusion proteins.
**FIG. 13** are bar graphs showing activation of human NK cells by NKG2D-binding domains (listed as clones).
**FIG. 14** are bar graphs showing activation of human NK cells by NKG2D-binding domains (listed as clones).
**FIG. 15** are bar graphs showing activation of mouse NK cells by NKG2D-binding domains (listed as clones).
**FIG. 16** are bar graphs showing activation of mouse NK cells by NKG2D-binding domains (listed as clones).
**FIG. 17** are bar graphs showing the cytotoxic effect of NKG2D-binding domains (listed as clones) on tumor cells.
**FIG. 18** are bar graphs showing the melting temperature of NKG2D-binding domains (listed as clones) measured by differential scanning fluorimetry.
**FIGs. 19A-19C** are bar graphs of synergistic activation of NK cells using CD16 and NKG2D-binding. FIG. 19A demonstrates levels of CD107a; FIG. 19B demonstrates levels of IFN-γ; FIG. 19C demonstrates levels of CD107a and IFN-γ. Graphs indicate the mean (n = 2) ± SD. Data are representative of five independent experiments using five different healthy donors.

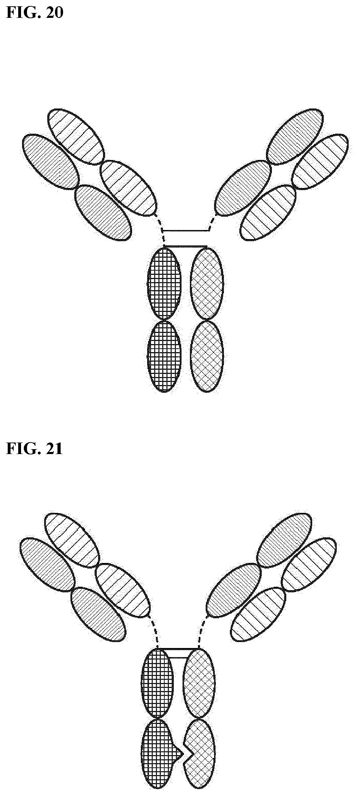

**FIG. 20** is a representation of a trispecific binding protein (TriNKET) in the Triomab form, which is a trifunctional, bispecific antibody that maintains an IgG-like shape. This chimera consists of two half antibodies, each with one light and one heavy chain, that originate from two parental antibodies. Triomab form may be a heterodimeric construct containing 1/2 of rat antibody and 1/2 of mouse antibody.
**FIG. 21** is a representation of a TriNKET in the KiH Common Light Chain form, which involves the knobs-into-holes (KIHs) technology. KiH is a heterodimer containing 2 Fab fragments binding to target 1 and 2, and an Fc stabilized by heterodimerization mutations. TriNKET in the KiH format may be a heterodimeric construct with 2 Fab fragments binding to target 1 and target 2, containing two different heavy chains and a common light chain that pairs with both heavy chains.

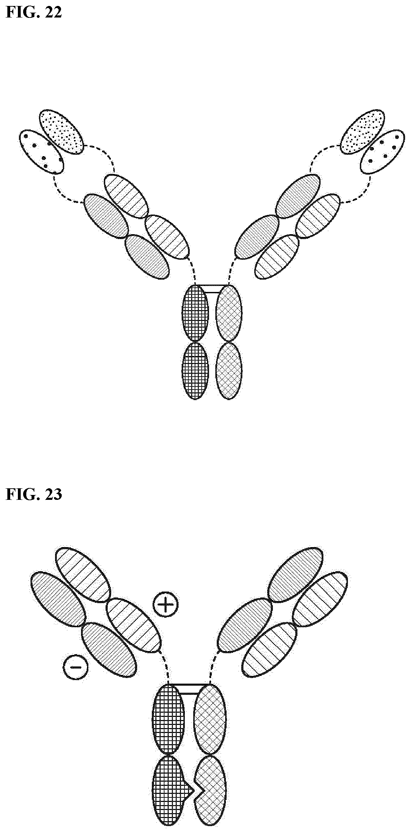

**FIG. 22** is a representation of a TriNKET in the dual-variable domain immunoglobulin (DVD-Ig^{™}) form, which combines the target-binding domains of two monoclonal antibodies via flexible naturally occurring linkers, and yields a tetravalent IgG-like molecule. DVD-Ig^{™} is a homodimeric construct where variable domain targeting antigen 2 is fused to the N-terminus of a variable domain of Fab fragment targeting antigen 1. DVD-Ig^{™} form contains normal Fc.
**FIG. 23** is a representation of a TriNKET in the Orthogonal Fab interface (Ortho-Fab) form, which is a heterodimeric construct that contains 2 Fab fragments binding to target 1 and target 2 fused to Fc. Light chain (LC)-heavy chain (HC) pairing is ensured by orthogonal interface. Heterodimerization is ensured by mutations in the Fc.

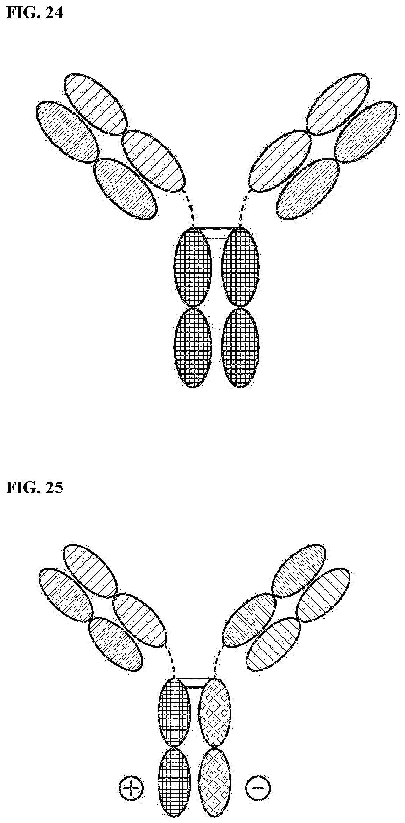

**FIG. 24** is a representation of a TriNKET in the 2-in-1 Ig format.
**FIG. 25** is a representation of a TriNKET in the ES form, which is a heterodimeric construct containing two different Fab fragments binding to target 1 and target 2 fused to the Fc. Heterodimerization is ensured by electrostatic steering mutations in the Fc.

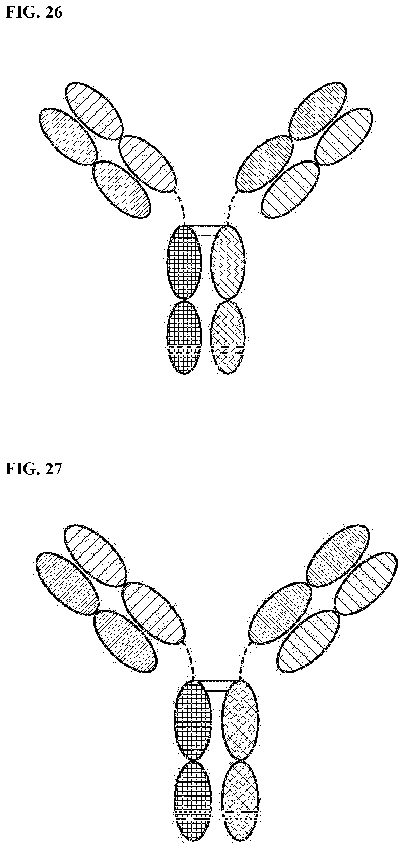

**FIG. 26** is a representation of a TriNKET in the Fab fragment Arm Exchange form: antibodies that exchange Fab arms by swapping a heavy chain and attached light chain (half-molecule) with a heavy-light chain pair from another molecule, resulting in bispecific antibodies. Fab Arm Exchange form (cFae) is a heterodimer containing 2 Fab fragments binding to target 1 and 2, and an Fc stabilized by heterodimerization mutations.
**FIG. 27** is a representation of a TriNKET in the SEED Body form, which is a heterodimer containing 2 Fab fragments binding to target 1 and 2, and an Fc stabilized by heterodimerization mutations.

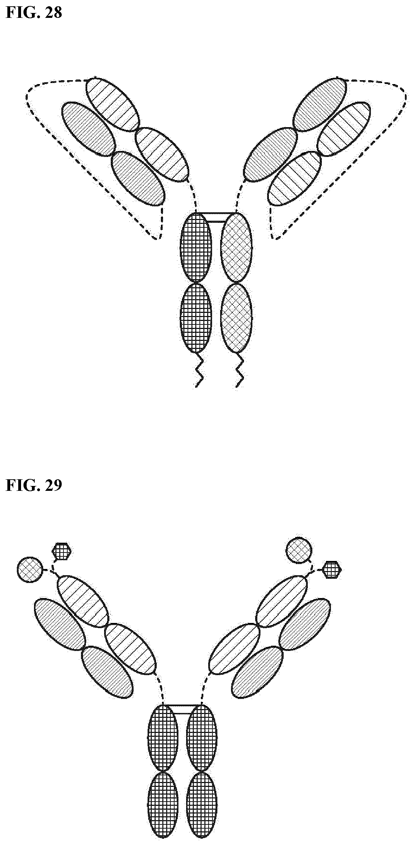

**FIG. 28** is a representation of a TriNKET in the LuZ-Y form, in which a leucine zipper is used to induce heterodimerization of two different HCs. The LuZ-Y form is a heterodimer containing two different scFabs binding to target 1 and 2, fused to Fc. Heterodimerization is ensured through leucine zipper motifs fused to C-terminus of Fc.
**FIG. 29** is a representation of a TriNKET in the Cov-X-Body form.
**FIGs. 30A and 30B** are representations of TriNKETs in the κλ-Body forms, which are heterodimeric constructs with two different Fab fragments fused to Fc stabilized by heterodimerization mutations: one Fab fragment targeting antigen 1 contains kappa LC, and the second Fab fragment targeting antigen 2 contains lambda LC. FIG. 30A is an exemplary representation of one form of a κλ-Body; FIG. 30B is an exemplary representation of another κλ-Body.

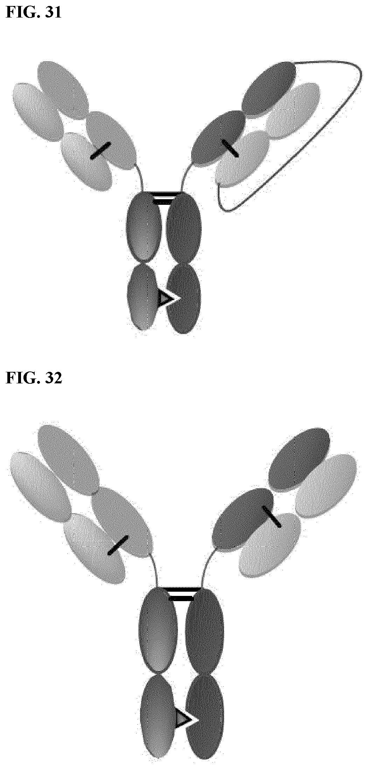

**FIG. 31** is an Oasc-Fab heterodimeric construct that includes Fab fragment binding to target 1 and scFab binding to target 2, both of which are fused to the Fc domain. Heterodimerization is ensured by mutations in the Fc domain.
**FIG. 32** is a DuetMab, which is a heterodimeric construct containing two different Fab fragments binding to antigens 1 and 2, and an Fc that is stabilized by heterodimerization mutations. Fab fragments 1 and 2 contain differential S-S bridges that ensure correct light chain and heavy chain pairing.

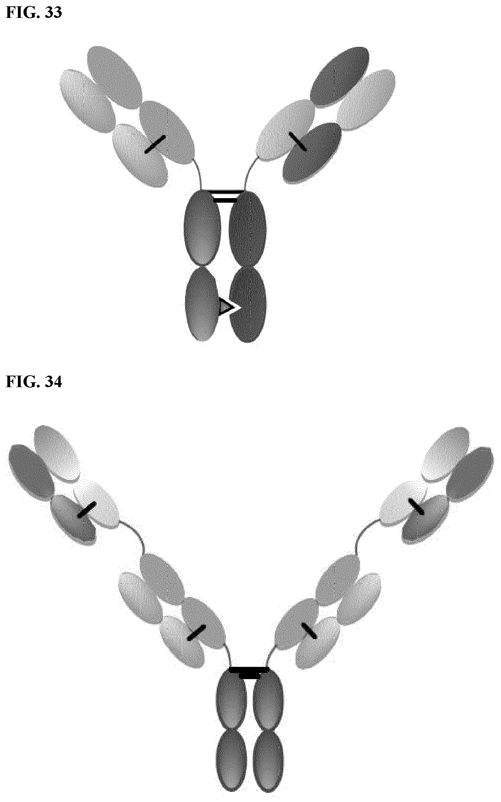

**FIG. 33** is a CrossmAb, which is a heterodimeric construct with two different Fab fragments binding to targets 1 and 2, and an Fc stabilized by heterodimerization mutations. CL and CH1 domains, and VH and VL domains are switched, *e.g.,* CH1 is fused in-line with VL, while CL is fused in-line with VH
**FIG. 34** is a Fit-Ig, which is a homodimeric construct where Fab fragment binding to antigen 2 is fused to the N-terminus of HC of Fab fragment that binds to antigen 1. The construct contains wild-type Fc.

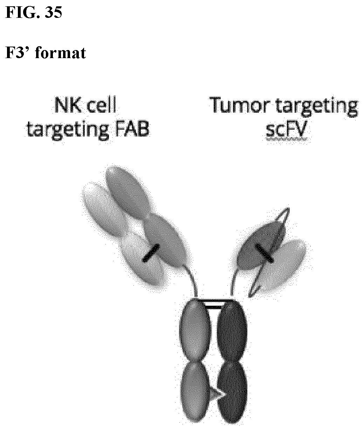

**FIG. 35** illustrates a trispecific antibody (TriNKET) that contains a tumor-associated antigen-binding scFv, a NKG2D-targeting Fab, and a heterodimerized antibody constant region/domain ("CD domain") that binds CD16 (scFv-Fab format). The antibody format is referred herein as F3'-TriNKET.
**FIG. 36** illustrates an exemplary trispecific antibodies (TriNKET), which includes an scFv first antigen-binding site that binds NKG2D, a second antigen-binding site that binds a tumor-associated antigen-binding (*e.g*., EpCAM), an additional tumor-associated antigen-binding site that binds a tumor-associated antigen-binding (*e.g*., EpCAM), and a heterodimerized antibody constant region that binds CD16. These antibody formats are referred herein as F4-TriNKET.
**FIG. 37** are line graphs demonstrating that TriNKETs and mAb bind to EpCAM expressed on H747 human colorectal cancer cells.
**FIG. 38** are line graphs demonstrating that TriNKETs and mAb bind to EpCAM expressed on HCC827 human lung cancer cells.
**FIG. 39** are line graphs demonstrating that TriNKETs and mAb bind to EPCAM expressed on HCT116 human colorectal cancer cells.
**FIG. 40A** and **FIG. 40B** are line graphs showing TriNKET-mediated killing of H747 cells with rested human NK cells from two different donors. The effector-to-target ratio was 10:1.
**FIG. 41A** and **FIG. 41B** are line graphs showing TriNKET-mediated killing of HCC827 cells with rested human NK cells from two different donors. The effector-to-target ratio was 10:1.
**FIG. 42A** and **FIG. 42B** are line graphs showing TriNKET-mediated killing of MCF7 cells with rested human NK cells from two different donors. The effector-to-target ratio was 10:1.
**FIG 43A** and **FIG. 43B** are line graphs showing TriNKET-mediated killing of HCT116 cells with rested human NK cells from two different donors. The effector-to-target ratio was 10:1.

### DETAILED DESCRIPTION

The invention provides multi-specific binding proteins that bind EPCAM on a cancer cell and the NKG2D receptor and CD16 receptor on natural killer cells to activate the natural killer cells, pharmaceutical compositions comprising such multi-specific binding proteins, and therapeutic methods using such multi-specific proteins and pharmaceutical compositions, including for the treatment of cancer. Various aspects of the invention are set forth below in sections; however, aspects of the invention described in one particular section are not to be limited to any particular section.

To facilitate an understanding of the present invention, a number of terms and phrases are defined below.

The terms "a" and "an" as used herein mean "one or more" and include the plural unless the context is inappropriate.

As used herein, the term "antigen-binding site" refers to the part of the immunoglobulin molecule that participates in antigen binding. In human antibodies,
the antigen binding site is formed by amino acid residues of the N-terminal variable ("V") regions of the heavy ("H") and light ("L") chains. Three highly divergent stretches within the V regions of the heavy and light chains are referred to as "hypervariable regions" which are interposed between more conserved flanking stretches known as "framework regions," or "FR". Thus the term "FR" refers to amino acid sequences which are naturally found between and adjacent to hypervariable regions in immunoglobulins. In a human antibody molecule, the three hypervariable regions of a light chain and the three hypervariable regions of a heavy chain are disposed relative to each other in three dimensional space to form an antigen-binding surface. The antigen-binding surface is complementary to the three-dimensional surface of a bound antigen, and the three hypervariable regions of each of the heavy and light chains are referred to as "complementarity-determining regions," or "CDRs." In certain animals, such as camels and cartilaginous fish, the antigen-binding site is formed by a single antibody chain providing a "single domain antibody." Antigen-binding sites can exist in an intact antibody, in an antigen-binding fragment of an antibody that retains the antigen-binding surface, or in a recombinant polypeptide such as an scFv, using a peptide linker to connect the heavy chain variable domain to the light chain variable domain in a single polypeptide.

The term "tumor associated antigen" as used herein means any antigen including but not limited to a protein, glycoprotein, ganglioside, carbohydrate, lipid that is associated with cancer. Such antigen can be expressed on malignant cells or in the tumor microenvironment such as on tumor-associated blood vessels, extracellular matrix, mesenchymal stroma, or immune infiltrates.

As used herein, the terms "subject" and "patient" refer to an organism to be treated by the methods and compositions described herein. Such organisms preferably include, but are not limited to, mammals (*e.g*., murines, simians, equines, bovines, porcines, canines, felines, and the like), and more preferably include humans.

As used herein, the term "effective amount" refers to the amount of a compound *(e.g.,* a compound of the present invention) sufficient to effect beneficial or desired results. An effective amount can be administered in one or more administrations, applications or dosages and is not intended to be limited to a particular formulation or administration route. As used herein, the term "treating" includes any effect, *e.g.,* lessening, reducing, modulating, ameliorating or eliminating, that results in the improvement of the condition, disease, disorder, and the like, or ameliorating a symptom thereof.

As used herein, the term "pharmaceutical composition" refers to the combination of an active agent with a carrier, inert or active, making the composition especially suitable for diagnostic or therapeutic use *in vivo* or *ex vivo.*

As used herein, the term "pharmaceutically acceptable carrier" refers to any of the standard pharmaceutical carriers, such as a phosphate buffered saline solution, water, emulsions (*e.g*., such as an oil/water or water/oil emulsions), and various types of wetting agents. The compositions also can include stabilizers and preservatives. For examples of carriers, stabilizers and adjuvants, *see e.g.,* Martin, Remington's Pharmaceutical Sciences, 15th Ed., Mack Publ. Co., Easton, PA [1975].

As used herein, the term "pharmaceutically acceptable salt" refers to any pharmaceutically acceptable salt *(e.g.,* acid or base) of a compound of the present invention which, upon administration to a subject, is capable of providing a compound of this invention or an active metabolite or residue thereof. As is known to those of skill in the art, "salts" of the compounds of the present invention may be derived from inorganic or organic acids and bases. Exemplary acids include, but are not limited to, hydrochloric, hydrobromic, sulfuric, nitric, perchloric, fumaric, maleic, phosphoric, glycolic, lactic, salicylic, succinic, toluene-p-sulfonic, tartaric, acetic, citric, methanesulfonic, ethanesulfonic, formic, benzoic, malonic, naphthalene-2-sulfonic, benzenesulfonic acid, and the like. Other acids, such as oxalic, while not in themselves pharmaceutically acceptable, may be employed in the preparation of salts useful as intermediates in obtaining the compounds of the invention and their pharmaceutically acceptable acid addition salts.

Exemplary bases include, but are not limited to, alkali metal (*e.g*., sodium) hydroxides, alkaline earth metal (*e.g*., magnesium) hydroxides, ammonia, and compounds of formula NW₄⁺, wherein W is C₁₋₄ alkyl, and the like.

Exemplary salts include, but are not limited to: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, flucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, palmoate, pectinate, persulfate, phenylpropionate, picrate, pivalate, propionate, succinate, tartrate, thiocyanate, tosylate, undecanoate, and the like. Other examples of salts include anions of the compounds of the present invention compounded with a suitable cation such as Na⁺, NH₄⁺, and NW₄⁺ (wherein W is a C₁₋₄ alkyl group), and the like.

For therapeutic use, salts of the compounds of the present invention are contemplated as being pharmaceutically acceptable. However, salts of acids and bases that are non-pharmaceutically acceptable may also find use, for example, in the preparation or purification of a pharmaceutically acceptable compound.

Throughout the description, where compositions are described as having, including, or comprising specific components, or where processes and methods are described as having, including, or comprising specific steps, it is contemplated that, additionally, there are compositions of the present invention that consist essentially of, or consist of, the recited components, and that there are processes and methods according to the present invention that consist essentially of, or consist of, the recited processing steps.

As a general matter, compositions specifying a percentage are by weight unless otherwise specified. Further, if a variable is not accompanied by a definition, then the previous definition of the variable controls.

### I. PROTEINS

The invention provides multi-specific binding proteins that bind to the NKG2D receptor and CD16 receptor on natural killer cells, and the tumor-associated antigen selected from any one of the antigens provided in Table 11. The multi-specific binding proteins are useful in the pharmaceutical compositions and therapeutic methods described herein. Binding of the multi-specific binding proteins to the NKG2D receptor and CD16 receptor on a natural killer cell enhances the activity of the natural killer cell toward destruction of tumor cells expressing the tumor-associated antigen selected from any one of the antigens provided in Table 11. Binding of the multi-specific binding proteins to tumor-associated antigen-expressing cells brings the cancer cells into proximity with the natural killer cell, which facilitates direct and indirect destruction of the cancer cells by the natural killer cell. Further description of some exemplary multi-specific binding proteins is provided below.

The first component of the multi-specific binding proteins binds to NKG2D receptor-expressing cells, which can include but are not limited to NK cells, γδ T cells and CD8⁺ αβ T cells. Upon NKG2D binding, the multi-specific binding proteins may block natural ligands, such as ULBP6 (UL16 binding protein 6) and MICA (Major Histocompatibility Complex Class I Chain-Related A), from binding to NKG2D and activating NKG2D receptors.

The second component of the multi-specific binding proteins binds a tumor-associated antigen selected from any one of the antigens provided in Table 11. The tumor-associated antigen-expressing cells, which may be found in leukemias such as, for example, acute myeloid leukemia and T-cell leukemia.

The third component for the multi-specific binding proteins binds to cells expressing CD16, an Fc receptor on the surface of leukocytes including natural killer cells, macrophages, neutrophils, eosinophils, mast cells, and follicular dendritic cells.

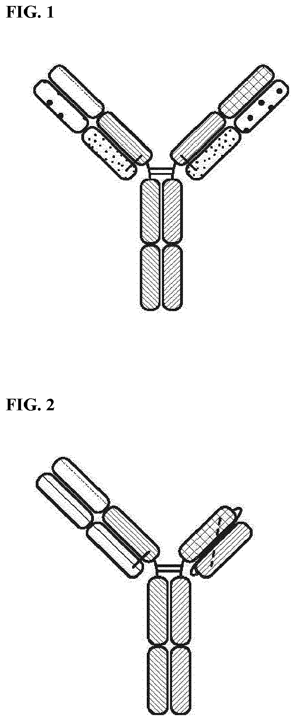

The multi-specific binding proteins described herein can take various formats. For example, one format is a heterodimeric, multi-specific antibody including a first immunoglobulin heavy chain, a first immunoglobulin light chain, a second immunoglobulin heavy chain and a second immunoglobulin light chain (FIG. 1). The first immunoglobulin heavy chain includes a first Fc (hinge-CH2-CH3) domain, a first heavy chain variable domain and optionally a first CH1 heavy chain domain. The first immunoglobulin light chain includes a first light chain variable domain and a first light chain constant domain. The first immunoglobulin light chain, together with the first immunoglobulin heavy chain, forms an antigen-binding site that binds NKG2D. The second immunoglobulin heavy chain comprises a second Fc (hinge-CH2-CH3) domain, a second heavy chain variable domain and optionally a second CH1 heavy chain domain. The second immunoglobulin light chain includes a second light chain variable domain and a second light chain constant domain. The second immunoglobulin light chain, together with the second immunoglobulin heavy chain, forms an antigen-binding site that binds a tumor-associated antigen selected from any one of the antigens provided in Table 11. The first Fc domain and second Fc domain together are able to bind to CD16 (FIG. 1). In some embodiments, the first immunoglobulin light chain is identical to the second immunoglobulin light chain.

Another exemplary format involves a heterodimeric, multi-specific antibody including a first immunoglobulin heavy chain, a second immunoglobulin heavy chain and an immunoglobulin light chain (FIG. 2). The first immunoglobulin heavy chain includes a first Fc (hinge-CH2-CH3) domain fused via either a linker or an antibody hinge to a single-chain variable fragment (scFv) composed of a heavy chain variable domain and light chain variable domain which pair and bind NKG2D, or bind a tumor-associated antigen selected from any one of the antigens provided in Table 11. The second immunoglobulin heavy chain includes a second Fc (hinge-CH2-CH3) domain, a second heavy chain variable domain and optionally a CH1 heavy chain domain. The immunoglobulin light chain includes a light chain variable domain and a light chain constant domain. The second immunoglobulin heavy chain pairs with the immunoglobulin light chain and binds to NKG2D or binds a tumor-associated antigen selected from any one of the antigens provided in Table 11. The first Fc domain and the second Fc domain together are able to bind to CD16 (FIG. 2).

One or more additional binding motifs may be fused to the C-terminus of the constant region CH3 domain, optionally via a linker sequence. In certain embodiments, the antigen-binding motif is a single-chain or disulfide-stabilized variable region (scFv) forming a tetravalent or trivalent molecule.

In some embodiments, the multi-specific binding protein is in the Triomab form, which is a trifunctional, bispecific antibody that maintains an IgG-like shape. This chimera consists of two half antibodies, each with one light and one heavy chain, that originate from two parental antibodies.

In some embodiments, the multi-specific binding protein is the KiH Common Light Chain (LC) form, which involves the knobs-into-holes (KIHs) technology. The KIH involves engineering C_{H}3 domains to create either a "knob" or a "hole" in each heavy chain to promote heterodimerization. The concept behind the "Knobs-into-Holes (KiH)" Fc technology was to introduce a "knob" in one CH3 domain (CH3A) by substitution of a small residue with a bulky one (*e.g*., T366W_{CH3A} in EU numbering). To accommodate the "knob," a complementary "hole" surface was created on the other CH3 domain (CH3B) by replacing the closest neighboring residues to the knob with smaller ones (*e.g.,* T366S/L368A/Y407V_{CH3B}). The "hole" mutation was optimized by structured-guided phage library screening (Atwell S, Ridgway JB, Wells JA, Carter P., Stable heterodimers from remodeling the domain interface of a homodimer using a phage display library, J. Mol. Biol. (1997) 270(1):26-35). X-ray crystal structures of KiH Fc variants (Elliott JM, Ultsch M, Lee J, Tong R, Takeda K, Spiess C, et al., Antiparallel conformation of knob and hole aglycosylated half-antibody homodimers is mediated by a CH2-CH3 hydrophobic interaction. J. Mol. Biol. (2014) 426(9):1947-57; Mimoto F, Kadono S, Katada H, Igawa T, Kamikawa T, Hattori K. Crystal structure of a novel asymmetrically engineered Fc variant with improved affinity for FcγRs. Mol. Immunol. (2014) 58(1): 132-8) demonstrated that heterodimerization is thermodynamically favored by hydrophobic interactions driven by steric complementarity at the inter-CH3 domain core interface, whereas the knob-knob and the hole-hole interfaces do not favor homodimerization owing to steric hindrance and disruption of the favorable interactions, respectively.

In some embodiments, the multi-specific binding protein is in the dual-variable domain immunoglobulin (DVD-Ig^{™}) form, which combines the target binding domains of two monoclonal antibodies via flexible naturally occurring linkers, and yields a tetravalent IgG-like molecule.

In some embodiments, the multi-specific binding protein is in the Orthogonal Fab interface (Ortho-Fab) form. In the ortho-Fab IgG approach (Lewis SM, Wu X, Pustilnik A, Sereno A, Huang F, Rick HL, et al., Generation of bispecific IgG antibodies by structure-based design of an orthogonal Fab interface. Nat. Biotechnol. (2014) 32(2):191-8), structure-based regional design introduces complementary mutations at the LC and HC_{VH-CH1} interface in only one Fab fragment, without any changes being made to the other Fab fragment.

In some embodiments, the multi-specific binding protein is in the 2-in-1 Ig format. In some embodiments, the multi-specific binding protein is in the ES form, which is a heterodimeric construct containing two different Fab fragments binding to targets 1 and target 2 fused to the Fc. Heterodimerization is ensured by electrostatic steering mutations in the Fc.

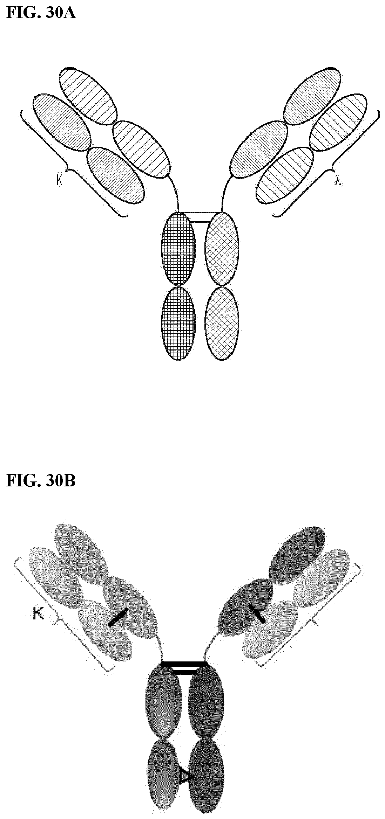

In some embodiments, the multi-specific binding protein is in the κλ-Body form, which is a heterodimeric construct with two different Fab fragments fused to Fc stabilized by heterodimerization mutations: Fab fragment1 targeting antigen 1 contains kappa LC, while second Fab fragment targeting antigen 2 contains lambda LC. FIG. 30A is an exemplary representation of one form of a κλ-Body; FIG. 30B is an exemplary representation of another κλ-Body.

In some embodiments, the multi-specific binding protein is in Fab Arm Exchange form (antibodies that exchange Fab arms by swapping a heavy chain and attached light chain (half-molecule) with a heavy-light chain pair from another molecule, which results in bispecific antibodies).

In some embodiments, the multi-specific binding protein is in the SEED Body form. The strand-exchange engineered domain (SEED) platform was designed to generate asymmetric and bispecific antibody-like molecules, a capability that expands therapeutic applications of natural antibodies. This protein engineered platform is based on exchanging structurally related sequences of immunoglobulin within the conserved CH3 domains. The SEED design allows efficient generation of AG/GA heterodimers, while disfavoring homodimerization of AG and GA SEED CH3 domains. (Muda M. et al., Protein Eng. Des. Sel. (2011, 24(5):447-54)).

In some embodiments, the multi-specific binding protein is in the LuZ-Y form, in which a leucine zipper is used to induce heterodimerization of two different HCs. (Wranik, BJ. et al., J. Biol. Chem. (2012), 287:43331-9).

In some embodiments, the multi-specific binding protein is in the Cov-X-Body form. In bispecific CovX-Bodies, two different peptides are joined together using a branched azetidinone linker and fused to the scaffold antibody under mild conditions in a site-specific manner. Whereas the pharmacophores are responsible for functional activities, the antibody scaffold imparts long half-life and Ig-like distribution. The pharmacophores can be chemically optimized or replaced with other pharmacophores to generate optimized or unique bispecific antibodies. (Doppalapudi VR et al., PNAS (2010), 107(52);22611-22616).

In some embodiments, the multi-specific binding protein is in an Oasc-Fab heterodimeric form that includes Fab fragment binding to target 1, and scFab binding to target 2 fused to Fc. Heterodimerization is ensured by mutations in the Fc.

In some embodiments, the multi-specific binding protein is in a DuetMab form, which is a heterodimeric construct containing two different Fab fragments binding to antigens 1 and 2, and Fc stabilized by heterodimerization mutations. Fab fragments 1 and 2 contain differential S-S bridges that ensure correct LC and HC pairing.

In some embodiments, the multi-specific binding protein is in a CrossmAb form, which is a heterodimeric construct with two different Fab fragments binding to targets 1 and 2, fused to Fc stabilized by heterodimerization. CL and CH1 domains and VH and VL domains are switched, *e.g.,* CH1 is fused in-line with VL, while CL is fused in-line with VH

In some embodiments, the multi-specific binding protein is in a Fit-Ig form, which is a homodimeric construct where Fab fragment binding to antigen 2 is fused to the N terminus of HC of Fab fragment that binds to antigen 1. The construct contains wild-type Fc.

Table 1 lists peptide sequences of heavy chain variable domains and light chain variable domains that, in combination, can bind to NKG2D. The NKG2D binding domains can vary in their binding affinity to NKG2D, nevertheless, they all activate human NKG2D and NK cells.

| Table 1 | | |
|---|---|---|
| Clone | Heavy chain variable region amino acid sequence | Light chain variable region amino acid sequence |
| ADI-27705 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:1) CDR1 (SEQ ID NO:105) - GSFSGYYWS CDR2 (SEQ ID NO:106) - EIDHSGSTNYNPSLKS CDR3 (SEQ ID NO:107) - ARARGPWSFDP | DIQMTQSPSTLSASVGDRVTIT CRASQSISSWLAWYQQKPGK APKLLIYKASSLESGVPSRFSG SGSGTEFTLTISSLQPDDFATY YCQQYNSYPITFGGGTKVEIK (SEQ ID NO:2) |
| ADI-27724 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:3) | EIVLTQSPGTLSLSPGERATLS CRASQSVSSSYLAWYQQKPG QAPRLLIYGASSRATGIPDRFS GSGSGTDFTLTISRLEPEDFAV YYCQQYGSSPITFGGGTKVEI K (SEQ ID NO:4) |
| ADI-27740 (A40) | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:5) | DIQMTQSPSTLSASVGDRVTIT CRASQSIGSWLAWYQQKPGK APKLLIYKASSLESGVPSRFSG SGSGTEFTLTISSLQPDDFATY YCQQYHSFYTFGGGTKVEIK (SEQ ID NO:6) |
| ADI-27741 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:7) | DIQMTQSPSTLSASVGDRVTIT CRASQSIGSWLAWYQQKPGK APKLLIYKASSLESGVPSRFSG SGSGTEFTLTISSLQPDDFATY YCQQSNSYYTFGGGTKVEIK (SEQ ID NO:8) |
| ADI-27743 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:9) | DIQMTQSPSTLSASVGDRVTIT CRASQSISSWLAWYQQKPGK APKLLIYKASSLESGVPSRFSG SGSGTEFTLTISSLQPDDFATY YCQQYNSYPTFGGGTKVEIK (SEQ ID NO:10) |
| ADI-28153 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWGFDPWGQGTLVTVSS | ELQMTQSPSSLSASVGDRVTIT CRTSQSISSYLNWYQQKPGQP PKLLIYWASTRESGVPDRFSGS GSGTDFTLTISSLQPEDSATYY CQQSYDIPYTFGQGTKLEIK |
| | (SEQ ID NO:11) | (SEQ ID NO:12) |
| ADI-28226 (C26) | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:13) | DIQMTQSPSTLSASVGDRVTIT CRASQSISSWLAWYQQKPGK APKLLIYKASSLESGVPSRFSG SGSGTEFTLTISSLQPDDFATY YCQQYGSFPITFGGGTKVEIK (SEQ ID NO:14) |
| ADI-28154 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:15) | DIQMTQSPSTLSASVGDRVTIT CRASQSISSWLAWYQQKPGK APKLLIYKASSLESGVPSRFSG SGSGTDFTLTISSLQPDDFATY YCQQSKEVPWTFGQGTKVEIK (SEQ ID NO:16) |
| ADI-29399 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:17) | DIQMTQSPSTLSASVGDRVTIT CRASQSISSWLAWYQQKPGK APKLLIYKASSLESGVPSRFSG SGSGTEFTLTISSLQPDDFATY YCQQYNSFPTFGGGTKVEIK (SEQ ID NO:18) |
| ADI-29401 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:19) | DIQMTQSPSTLSASVGDRVTIT CRASQSIGSWLAWYQQKPGK APKLLIYKASSLESGVPSRFSG SGSGTEFTLTISSLQPDDFATY YCQQYDIYPTFGGGTKVEIK (SEQ ID NO:20) |
| ADI-29403 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:21) | DIQMTQSPSTLSASVGDRVTIT CRASQSISSWLAWYQQKPGK APKLLIYKASSLESGVPSRFSG SGSGTEFTLTISSLQPDDFATY YCQQYDSYPTFGGGTKVEIK (SEQ ID NO:22) |
| ADI-29405 | QVQLQQWGAGLLKPSETLSLTCAV YGGSFSGYYWSWIRQPPGKGLEWI | DIQMTQSPSTLSASVGDRVTIT CRASQSISSWLAWYQQKPGK |
| | GEIDHSGSTNYNPSLKSRVTISVDTS KNQFSLKLSSVTAADTAVYYCARA RGPWSFDPWGQGTLVTVSS (SEQ ID NO:23) | APKLLIYKASSLESGVPSRFSG SGSGTEFTLTISSLQPDDFATY YCQQYGSFPTFGGGTKVEIK (SEQ ID NO:24) |
| ADI-29407 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:25) | DIQMTQSPSTLSASVGDRVTIT CRASQSISSWLAWYQQKPGK APKLLIYKASSLESGVPSRFSG SGSGTEFTLTISSLQPDDFATY YCQQYQSFPTFGGGTKVEIK (SEQ ID NO:26) |
| ADI-29419 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:27) | DIQMTQSPSTLSASVGDRVTIT CRASQSISSWLAWYQQKPGK APKLLIYKASSLESGVPSRFSG SGSGTEFTLTISSLQPDDFATY YCQQYSSFSTFGGGTKVEIK (SEQ ID NO:28) |
| ADI-29421 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:29) | DIQMTQSPSTLSASVGDRVTIT CRASQSISSWLAWYQQKPGK APKLLIYKASSLESGVPSRFSG SGSGTEFTLTISSLQPDDFATY YCQQYESYSTFGGGTKVEIK (SEQ ID NO:30) |
| ADI-29424 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:31) | DIQMTQSPSTLSASVGDRVTIT CRASQSISSWLAWYQQKPGK APKLLIYKASSLESGVPSRFSG SGSGTEFTLTISSLQPDDFATY YCQQYDSFITFGGGTKVEIK (SEQ ID NO:32) |
| ADI-29425 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS | DIQMTQSPSTLSASVGDRVTIT CRASQSISSWLAWYQQKPGK APKLLIYKASSLESGVPSRFSG SGSGTEFTLTISSLQPDDFATY YCQQYQSYPTFGGGTKVEIK |
| | (SEQ ID NO:33) | (SEQ ID NO:34) |
| ADI-29426 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:35) | DIQMTQSPSTLSASVGDRVTIT CRASQSIGSWLAWYQQKPGK APKLLIYKASSLESGVPSRFSG SGSGTEFTLTISSLQPDDFATY YCQQYHSFPTFGGGTKVEIK (SEQ ID NO:36) |
| ADI-29429 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:37) | DIQMTQSPSTLSASVGDRVTIT CRASQSIGSWLAWYQQKPGK APKLLIYKASSLESGVPSRFSG SGSGTEFTLTISSLQPDDFATY YCQQYELYSYTFGGGTKVEIK (SEQ ID NO:38) |
| ADI-29447 (F47) | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:39) | DIQMTQSPSTLSASVGDRVTIT CRASQSISSWLAWYQQKPGK APKLLIYKASSLESGVPSRFSG SGSGTEFTLTISSLQPDDFATY YCQQYDTFITFGGGTKVEIK (SEQ ID NO:40) |
| ADI-27727 | QVQLVQSGAEVKKPGSSVKVSCKA SGGTFSSYAISWVRQAPGQGLEWM GGIIPIFGTANYAQKFQGRVTITADE STSTAYMELSSLRSEDTAVYYCAR GDSSIRHAYYYYGMDVWGQGTTV TVSS (SEQ ID NO:41) CDR1 (SEQ ID NO:43) - GTFSSY AIS CDR2 (SEQ ID NO:44) - GIIPIFGTANYAQKFQG CDR3 (SEQ ID NO:45) - ARGDSSIRHAYYYYGMDV | DIVMTQSPDSLAVSLGERATIN CKSSQSVLYSSNNKNYLAWY QQKPGQPPKLLIYWASTRESG VPDRFSGSGSGTDFTLTISSLQ AEDVAVYYCQQYYSTPITFGG GTKVEIK (SEQ ID NO:42) CDR1 (SEQ ID NO:46) - KSSQSVLYSSNNKNYLA CDR2 (SEQ ID NO:47) - WASTRES CDR3 (SEQ ID NO:48) - QQYYSTPIT |
| ADI-29443 (F43) | QLQLQESGPGLVKPSETLSLTCTVS GGSISSSSYYWGWIRQPPGKGLEWI GSIYYSGSTYYNPSLKSRVTISVDTS KNQFSLKLSSVTAADTAVYYCARG SDRFHPYFDYWGQGTLVTVSS (SEQ ID NO:49) CDR1 (SEQ ID NO:51) - GSISSSSYYWG CDR2 (SEQ ID NO:52) - SIYYSGSTYYNPSLKS CDR3 (SEQ ID NO:53) - ARGSDRFHPYFDY | EIVLTQSPATLSLSPGERATLS CRASQSVSRYLAWYQQKPGQ APRLLIYDASNRATGIPARFSG SGSGTDFTLTISSLEPEDFAVY YCQQFDTWPPTFGGGTKVEIK (SEQ ID NO:50) CDR1 (SEQ ID NO:54) - RASQSVSRYLA CDR2 (SEQ ID NO:55) - DASNRAT CDR3 (SEQ ID NO:56) - QQFDTWPPT |
| ADI-29404 (F04) | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:57) | DIQMTQSPSTLSASVGDRVTIT CRASQSISSWLAWYQQKPGK APKLLIYKASSLESGVPSRFSG SGSGTEFTLTISSLQPDDFATY YCEQYDSYPTFGGGTKVEIK (SEQ ID NO:58) |
| ADI-28200 | QVQLVQSGAEVKKPGSSVKVSCKA SGGTFSSYAISWVRQAPGQGLEWM GGIIPIFGTANYAQKFQGRVTITADE STSTAYMELSSLRSEDTAVYYCAR RGRKASGSFYYYYGMDVWGQGTT VTVSS (SEQ ID NO:59) CDR1 (SEQ ID NO:109) - GTFSSY AIS CDR2 (SEQ ID NO:110) - GIIPIFGTANYAQKFQG CDR3 (SEQ ID NO:111) - ARRGRKASGSFYYYYGMDV |  (SEQ ID NO:60) CDR1 (SEQ ID NO:112) - ESSQSLLNSGNQKNYLT CDR2 (SEQ ID NO:113) - WASTRES CDR3 (SEQ ID NO:114) - QNDYSYPYT |
| ADI-29379 (E79) | QVQLVQSGAEVKKPGASVKVSCK ASGYTFTSYYMHWVRQAPGQGLE WMGIINPSGGSTSYAQKFQGRVTM TRDTSTSTVYMELSSLRSEDTAVYY CARGAPNYGDTTHDYYYMDVWG KGTTVTVSS (SEQ ID NO:61) CDR1 (SEQ ID NO:63) - YTFTSYYMH CDR2 (SEQ ID NO:64) - IINPSGGSTSYAQKFQG CDR3 (SEQ ID NO:65) - ARGAPNYGDTTHDYYYMDV | EIVMTQSPATLSVSPGERATLS CRASQSVSSNLAWYQQKPGQ APRLLIYGASTRATGIPARFSG SGSGTEFTLTISSLQSEDFAVY YCQQYDDWPFTFGGGTKVEI K (SEQ ID NO:62) CDR1 (SEQ ID NO:66) - RASQSVSSNLA CDR2 (SEQ ID NO:67) - GASTRAT CDR3 (SEQ ID NO:68) - QQYDDWPFT |
| ADI-29463 (F63) | QVQLVQSGAEVKKPGASVKVSCK ASGYTFTGYYMHWVRQAPGQGLE WMGWINPNSGGTNYAQKFQGRVT MTRDTSISTAYMELSRLRSDDTAV YYCARDTGEYYDTDDHGMDVWG QGTTVTVSS (SEQ ID NO:69) CDR1 (SEQ ID NO:71) - YTFTGYYMH CDR2 (SEQ ID NO:72) - WINPNSGGTNYAQKFQG CDR3 (SEQ ID NO:73) - ARDTGEYYDTDDHGMDV | EIVLTQSPGTLSLSPGERATLS CRASQSVSSNLAWYQQKPGQ APRLLIYGASTRATGIPARFSG SGSGTEFTLTISSLQSEDFAVY YCQQDDYWPPTFGGGTKVEI K (SEQ ID NO:70) CDR1 (SEQ ID NO:74) - RASQSVSSNLA CDR2 (SEQ ID NO:75) - GASTRAT CDR3 (SEQ ID NO:76) - QQDDYWPPT |
| ADI-27744 (A44) | EVQLLESGGGLVQPGGSLRLSCAAS GFTFSSYAMSWVRQAPGKGLEWV SAISGSGGSTYYADSVKGRFTISRD NSKNTLYLQMNSLRAEDTAVYYC AKDGGYYDSGAGDYWGQGTLVTV SS (SEQ ID NO:77) CDR1 (SEQ ID NO:79) - FTFSSYAMS CDR2 (SEQ ID NO:80) - AISGSGGSTYYADSVKG CDR3 (SEQ ID NO:81) - AKDGGYYDSGAGDY | DIQMTQSPSSVSASVGDRVTIT CRASQGIDSWLAWYQQKPGK APKLLIYAASSLQSGVPSRFSG SGSGTDFTLTISSLQPEDFATY YCQQGVSYPRTFGGGTKVEIK (SEQ ID NO:78) CDR1 (SEQ ID NO:82) - RASQGIDSWLA CDR2 (SEQ ID NO:83) - AASSLQS CDR3 (SEQ ID NO:84) - QQGVSYPRT |
| ADI-27749 (A49) | EVQLVESGGGLVKPGGSLRLSCAA SGFTFSSYSMNWVRQAPGKGLEW VSSISSSSSYIYYADSVKGRFTISRD NAKNSLYLQMNSLRAEDTAVYYC ARGAPMGAAAGWFDPWGQGTLVT VSS (SEQ ID NO:85) CDR1 (SEQ ID NO:87) - FTFSSYSMN CDR2 (SEQ ID NO:88) - SISSSSSYIYYADSVKG CDR3 (SEQ ID NO:89) - ARGAPMGAAAGWFDP | DIQMTQSPSSVSASVGDRVTIT CRASQGISSWLAWYQQKPGK APKLLIYAASSLQSGVPSRFSG SGSGTDFTLTISSLQPEDFATY YCQQGVSFPRTFGGGTKVEIK (SEQ ID NO:86) CDR1 (SEQ ID NO:90) - RASQGISSWLA CDR2 (SEQ ID NO:91) - AASSLQS CDR3 (SEQ ID NO:92) - QQGVSFPRT |
| ADI-29378 (E78) | QVQLVQSGAEVKKPGASVKVSCK ASGYTFTSYYMHWVRQAPGQGLE WMGIINPSGGSTSYAQKFQGRVTM TRDTSTSTVYMELSSLRSEDTAVYY CAREGAGFAYGMDYYYMDVWGK GTTVTVSS (SEQ ID NO:93) CDR1 (SEQ ID NO:95) - YTFTSYYMH CDR2 (SEQ ID NO:96) - IINPSGGSTSYAQKFQG CDR3 (SEQ ID NO:97) - AREGAGFAYGMDYYYMDV | EIVLTQSPATLSLSPGERATLS CRASQSVSSYLAWYQQKPGQ APRLLIYDASNRATGIPARFSG SGSGTDFTLTISSLEPEDFAVY YCQQSDNWPFTFGGGTKVEIK (SEQ ID NO:94) CDR1 (SEQ ID NO:98) - RASQSVSSYLA CDR2 (SEQ ID NO:99) - DASNRAT CDR3 (SEQ ID NO:100) - QQSDNWPFT |

Alternatively, a heavy chain variable domain represented by SEQ ID NO:101 can be paired with a light chain variable domain represented by SEQ ID NO:102 to form an antigen-binding site that can bind to NKG2D, as illustrated in US 9,273,136.

SEQ ID NO:101
QVQLVESGGGLVKPGGSLRLSCAASGFTFSSYGMHWVRQAPGKGLEWVAFI
RYDGSNKYYADSVKGRFTISRDNSKNTLYLQMNSLRAEDTAVYYCAKDRGL
GDGTYFDYWGQGTTVTVSS

SEQ ID NO:102
QSALTQPASVSGSPGQSITISCSGSSSNIGNNAVNWYQQLPGKAPKLLIYYDDL
LPSGVSDRFSGSKSGTSAFLAISGLQSEDEADYYCAAWDDSLNGPVFGGGTK
LTVL

Alternatively, a heavy chain variable domain represented by SEQ ID NO:103 can be paired with a light chain variable domain represented by SEQ ID NO:104 to form an antigen-binding site that can bind to NKG2D, as illustrated in US 7,879,985.

SEQ ID NO:103
QVHLQESGPGLVKPSETLSLTCTVSDDSISSYYWSWIRQPPGKGLEWIGHISYS
GSANYNPSLKSRVTISVDTSKNQFSLKLSSVTAADTAVYYCANWDDAFNIWG
QGTMVTVSS

SEQ ID NO:104
EIVLTQSPGTLSLSPGERATLSCRASQSVSSSYLAWYQQKPGQAPRLLIYGASS
RATGIPDRFSGSGSGTDFTLTISRLEPEDFAVYYCQQYGSSPWTFGQGTKVEIK

Table 2 lists peptide sequences of heavy chain variable domains and light chain variable domains that, in combination, can bind to EpCAM.

| Table 2 | | |
|---|---|---|
| Clones | Heavy chain variable domain amino acid sequence | Light chain variable domain amino acid sequence |
| Oportuzumab | EVQLVQSGPGLVQPGGSVRISCA ASGYTFTNYGMNWVKQAPGKGL EWMGWINTYTGESTYADSFKGRF TFSLDTSASAAYLQINSLRAEDTA VYYCARFAIKGDYWGQGTLLTVS SE (SEQ ID NO:115) CDR1 (SEQ ID NO:116) - GYTFTNY CDR2 (SEQ ID NO:117) - NTYTGE CDR3 (SEQ ID NO:118) - FAIKGDY | DIQMTQSPSSLSASVGDRVTITCRS TKSLLHSNGITYLYWYQQKPGKAP KLLIYQMSNLASGVPSRFSSSGSGT DFTLTISSLQPEDFATYYCAQNLEI PRTFGQGTKVELKR (SEQ ID NO:119) CDR1(SEQ ID NO:120) - KSLLHSNGITYLY CDR2 (SEQ ID NO:121) - QMSNLAS CDR3 (SEQ ID NO:122) - AQNLEIPRT |
| Adecatumumab | EVQLLESGGGVVQPGRSLRLSCA ASGFTFSSYGMHWVRQAPGKGL EWVAVISYDGSNKYYADSVKGR FTISRDNSKNTLYLQMNSLRAEDT AVYYCAKDMGWGSGWRPYYYY GMDVWGQGTTVTVSSA (SEQ ID NO:123) CDR1 (SEQ ID NO:124) - GFTFSSY CDR2 (SEQ ID NO:125) - SYDGSN CDR3 (SEQ ID NO:126) - DMGWGSGWRPYYYYGMDV | ELQMTQSPSSLSASVGDRVTITCRT SQSISSYLNWYQQKPGQPPKLLIY WASTRESGVPDRFSGSGSGTDFTL TISSLQPEDSATYYCQQSYDIPYTF GQGTKLEIKR (SEQ ID NO:127) CDR1 (SEQ ID NO:128) - QSISSYLN CDR2 (SEQ ID NO:129) - WASTRES CDR3 (SEQ ID NO:130) - QQSYDIPYT |
| Citatuzumab | EVQLVQSGPGLVQPGGSVRISCA ASGYTFTNYGMNWVKQAPGKGL EWMGWINTYTGESTYADSFKGRF TFSLDTSASAAYLQINSLRAEDTA VYYCARFAIKGDYWGQGTLLTVS SA (SEQ ID NO:131) CDR1 (SEQ ID NO:132) - GYTFTNY CDR2 (SEQ ID NO:133) - NTYTGE CDR3 (SEQ ID NO:134) - FAIKGDY | DIQMTQSPSSLSASVGDRVTITCRS TKSLLHSNGITYLYWYQQKPGKAP KLLIYQMSNLASGVPSRFSSSGSGT DFTLTISSLQPEDFATYYCAQNLEI PRTFGQGTKVELKR (SEQ ID NO:135) CDR1 (SEQ ID NO:136) - KSLLHSNGITYLY CDR2 (SEQ ID NO:137) - QMSNLAS CDR3 (SEQ ID NO:138) - AQNLEIPRT |
| Solitomab (MT110) | EVQLLEQSGAELVRPGTSVKISCK ASGYAFTNYWLGWVKQRPGHGL EWIGDIFPGSGNIHYNEKFKGKAT LTADKSSSTAYMQLSSLTFEDSAV YFCARLRNWDEPMDYWGQGTTV TVSS (SEQ ID NO:139) CDR1 (SEQ ID NO:140) - GYAFTNY CDR2 (SEQ ID NO:141) - FPGSGN CDR3 (SEQ ID NO:142) - LRNWDEPMDY | ELVMTQSPSSLTVTAGEKVTMSCK SSQSLLNSGNQKNYLTWYQQKPG QPPKLLIYWASTRESGVPDRFTGS GSGTDFTLTISSVQAEDLAVYYCQ NDYSYPLTFGAGTKLEIKG (SEQ ID NO:143) CDR1 (SEQ ID NO:144) - QSLLNSGNQKNYLT CDR2 (SEQ ID NO:145) - WASTRES CDR3 (SEQ ID NO:146) - QNDYSYPLT |

Alternatively, novel antigen-binding sites that can bind to EpCAM can be identified by screening for binding to the amino acid sequence defined by SEQ ID NO:147.

SEQ ID NO:147
MAPPQVLAFGLLLAAATATFAAAQEECVCENYKLAVNCFVNNNRQCQCTSVGAQN
TVICSKLAAKCLVMKAEMNGSKLGRRAKPEGALQNNDGLYDPDCDESGLFKAKQC
NGTSMCWCVNTAGVRRTDKDTEITCSERVRTYWIIIELKHKAREKPYDSKSLRTALQ
KEITTRYQLDPKFITSILYENNVITIDLVQNSSQKTQNDVDIADVAYYFEKDVKGESLF
HSKKMDLTVNGEQLDLDPGQTLIYYVDEKAPEFSMQGLKAGVIAVIVVVVIAVVAGI
VVLVISRKKRMAKYEKAEIKEMGEMHRELNA

Antigen-binding sites that can bind to tumor associated antigen CA125 can be identified by screening for binding to the amino acid sequence defined by SEQ ID NO:148.

SEQ ID NO:148

MLKPSGLPGSSSPTRSLMTGSRSTKATPEMDSGLTGATLSPKTSTGAIVVTEHTLPFTS
PDKTLASPTSSVVGRTTQSLGVMSSALPESTSRGMTHSEQRTSPSLSPQVNGTPSRNY
PATSMVSGLSSPRTRTSSTEGNFTKEASTYTLTVETTSGPVTEKYTVPTETSTTEGDST
ETPWDTRYIPVKITSPMKTFADSTASKENAPVSMTPAETTVTDSHTPGRTNPSFGTLY
SSFLDLSPKGTPNSRGETSLELILSTTGYPFSSPEPGSAGHSRISTSAPLSSSASVLDNKI
SETSIFSGQSLTSPLSPGVPEARASTMPNSAIPFSMTLSNAETSAERVRSTISSLGTPSIS
TKQTAETILTFHAFAETMDIPSTHIAKTLASEWLGSPGTLGGTSTSALTTTSPSTTLVSE
ETNTHHSTSGKETEGTLNTSMTPLETSAPGEESEMTATLVPTLGFTTLDSKIRSPSQVS
SSHPTRELRTTGSTSGRQSSSTAAHGSSDILRATTSSTSKASSWTSESTAQQFSEPQHT
QWVETSPSMKTERPPASTSVAAPITTSVPSVVSGFTTLKTSSTKGIWLEETSADTLIGE

STAGPTTHQFAVPTGISMTGGSSTRGSQGTTHLLTRATASSETSADLTLATNGVPVSV
SPAVSKTAAGSSPPGGTKPSYTMVSSVIPETSSLQSSAFREGTSLGLTPLNTRHPFSSPE
PDSAGHTKISTSIPLLSSASVLEDKVSATSTFSHHKATSSITTGTPEISTKTKPSSAVLSS
MTLSNAATSPERVRNATSPLTHPSPSGEETAGSVLTLSTSAETTDSPNIHPTGTLTSESS
ESPSTLSLPSVSGVKTTFSSSTPSTHLFTSGEETEETSNPSVSQPETSVSRVRTTLASTSV
PTPVFPTMDTWPTRSAQFSSSHLVSELRATSSTSVTNSTGSALPKISHLTGTATMSQT
NRDTFNDSAAPQSTTWPETSPRFKTGLPSATTTVSTSATSLSATVMVSKFTSPATSSM
EATSIREPSTTILTTETTNGPGSMAVASTNIPIGKGYITEGRLDTSHLPIGTTASSETSMD
FTMAKESVSMSVSPSQSMDAAGSSTPGRTSQFVDTFSDDVYHLTSREITIPRDGTSSA
LTPQMTATHPPSPDPGSARSTWLGILSSSPSSPTPKVTMSSTFSTQRVTTSMIMDTVET
SRWNMPNLPSTTSLTPSNIPTSGAIGKSTLVPLDTPSPATSLEASEGGLPTLSTYPESTN
TPSIHLGAHASSESPSTIKLTMASVVKPGSYTPLTFPSIETHIHVSTARMAYSSGSSPEM
TAPGETNTGSTWDPTTYITTTDPKDTSSAQVSTPHSVRTLRTTENHPKTESATPAAYS
GSPKISSSPNLTSPATKAWTITDTTEHSTQLHYTKLAEKSSGFETQSAPGPVSVVIPTSP
TIGSSTLELTSDVPGEPLVLAPSEQTTITLPMATWLSTSLTEEMASTDLDISSPSSPMST
FAIFPPMSTPSHELSKSEADTSAIRNTDSTTLDQHLGIRSLGRTGDLTTVPITPLTTTWT
SVIEHSTQAQDTLSATMSPTHVTQSLKDQTSIPASASPSHLTEVYPELGTQGRSSSEAT
TFWKPSTDTLSREIETGPTNIQSTPPMDNTTTGSSSSGVTLGIAHLPIGTSSPAETSTNM
ALERRSSTATVSMAGTMGLLVTSAPGRSISQSLGRVSSVLSESTTEGVTDSSKGSSPR
LNTQGNTALSSSLEPSYAEGSQMSTSIPLTSSPTTPDVEFIGGSTFWTKEVTTVMTSDI
SKSSARTESSSATLMSTALGSTENTGKEKLRTASMDLPSPTPSMEVTPWISLTLSNAP
NTTDSLDLSHGVHTSSAGTLATDRSLNTGVTRASRLENGSDTSSKSLSMGNSTHTSM
TYTEKSEVSSSIHPRPETSAPGAETTLTSTPGNRAISLTLPFSSIPVEEVISTGITSGPDIN
SAPMTHSPITPPTIVWTSTGTIEQSTQPLHAVSSEKVSVQTQSTPYVNSVAVSASPTHE
NSVSSGSSTSSPYSSASLESLDSTISRRNAITSWLWDLTTSLPTTTWPSTSLSEALSSGH
SGVSNPSSTTTEFPLFSAASTSAAKQRNPETETHGPQNTAASTLNTDASSVTGLSETPV
GASISSEVPLPMAITSRSDVSGLTSESTANPSLGTASSAGTKLTRTISLPTSESLVSFRM
NKDPWTVSIPLGSHPTTNTETSIPVNSAGPPGLSTVASDVIDTPSDGAESIPTVSFSPSP
DTEVTTISHFPEKTTHSFRTISSLTHELTSRVTPIPGDWMSSAMSTKPTGASPSITLGER
RTITSAAPTTSPIVLTASFTETSTVSLDNETTVKTSDILDARKTNELPSDSSSSSDLINTSI
ASSTMDVTKTASISPTSISGMTASSSPSLFSSDRPQVPTSTTETNTATSPSVSSNTYSLD
GGSNVGGTPSTLPPFTITHPVETSSALLAWSRPVRTFSTMVSTDTASGENPTSSNSVVT
SVPAPGTWTSVGSTTDLPAMGFLKTSPAGEAHSLLASTIEPATAFTPHLSAAVVTGSS
ATSEASLLTTSESKAIHSSPQTPTTPTSGANWETSATPESLLVVTETSDTTLTSKILVTD
TILFSTVSTPPSKFPSTGTLSGASFPTLLPDTPAIPLTATEPTSSLATSFDSTPLVTIASDS
LGTVPETTLTMSETSNGDALVLKTVSNPDRSIPGITIQGVTESPLHPSSTSPSKIVAPRN
TTYEGSITVALSTLPAGTTGSLVFSQSSENSETTALVDSSAGLERASVMPLTTGSQGM
ASSGGIRSGSTHSTGTKTFSSLPLTMNPGEVTAMSEITTNRLTATQSTAPKGIPVKPTS
AESGLLTPVSASSSPSKAFASLTTAPPTWGIPQSTLTFEFSEVPSLDTKSASLPTPGQSL
NTIPDSDASTASSSLSKSPEKNPRARMMTSTKAISASSFQSTGFTETPEGSASPSMAGH
EPRVPTSGTGDPRYASESMSYPDPSKASSAMTSTSLASKLTTLFSTGQAARSGSSSSPI
SLSTEKETSFLSPTASTSRKTSLFLGPSMARQPNILVHLQTSALTLSPTSTLNMSQEEPP
ELTSSQTIAEEEGTTAETQTLTFTPSETPTSLLPVSSPTEPTARRKSSPETWASSISVPAK
TSLVETTDGTLVTTIKMSSQAAQGNSTWPAPAEETGSSPAGTSPGSPEMSTTLKIMSS
KEPSISPEIRSTVRNSPWKTPETTVPMETTVEPVTLQSTALGSGSTSISHLPTGTTSPTK
SPTENMLATERVSLSPSPPEAWTNLYSGTPGGTRQSLATMSSVSLESPTARSITGTGQ
QSSPELVSKTTGMEFSMWHGSTGGTTGDTHVSLSTSSNILEDPVTSPNSVSSLTDKSK
HKTETWVSTTAIPSTVLNNKIMAAEQQTSRSVDEAYSSTSSWSDQTSGSDITLGASPD
VTNTLYITSTAQTTSLVSLPSGDQGITSLTNPSGGKTSSASSVTSPSIGLETLRANVSAV
KSDIAPTAGHLSQTSSPAEVSILDVTTAPTPGISTTITTMGTNSISTTTPNPEVGMSTMD

STPATERRTTSTEHPSTWSSTAASDSWTVTDMTSNLKVARSPGTISTMHTTSFLASST
ELDSMSTPHGRITVIGTSLVTPSSDASAVKTETSTSERTLSPSDTTASTPISTFSRVQRM
SISVPDILSTSWTPSSTEAEDVPVSMVSTDHASTKTDPNTPLSTFLFDSLSTLDWDTGR
SLSSATATTSAPQGATTPQELTLETMISPATSQLPFSIGHITSAVTPAAMARSSGVTFSR
PDPTSKKAEQTSTQLPTTTSAHPGQVPRSAATTLDVIPHTAKTPDATFQRQGQTALTT
EARATSDSWNEKEKSTPSAPWITEMMNSVSEDTIKEVTSSSSVLRTLNTLDINLESGT
TSSPSWKSSPYERIAPSESTTDKEAIHPSTNTVETTGWVTSSEHASHSTIPAHSASSKLT
SPVVTTSTREQAIVSMSTTTWPESTRARTEPNSFLTIELRDVSPYMDTSSTTQTSIISSP
GSTAITKGPRTEITSSKRISSSFLAQSMRSSDSPSEAITRLSNFPAMTESGGMILAMQTS
PPGATSLSAPTLDTSATASWTGTPLATTQRFTYSEKTTLFSKGPEDTSQPSPPSVEETS
SSSSLVPIHATTSPSNILLTSQGHSPSSTPPVTSVFLSETSGLGKTTDMSRISLEPGTSLPP
NLSSTAGEALSTYEASRDTKAIHHSADTAVTNMEATSSEYSPIPGHTKPSKATSPLVT
SHIMGDITSSTSVFGSSETTEIETVSSVNQGLQERSTSQVASSATETSTVITHVSSGDAT
THVTKTQATFSSGTSISSPHQFITSTNTFTDVSTNPSTSLIMTESSGVTITTQTGPTGAA
TQGPYLLDTSTMPYLTETPLAVTPDFMQSEKTTLISKGPKDVSWTSPPSVAETSYPSS
LTPFLVTTIPPATSTLQGQHTSSPVSATSVLTSGLVKTTDMLNTSMEPVTNSPQNLNN
PSNEILATLAATTDIETIHPSINKAVTNMGTASSAHVLHSTLPVSSEPSTATSPMVPASS
MGDALASISIPGSETTDIEGEPTSSLTAGRKENSTLQEMNSTTESNIILSNVSVGAITEA
TKMEVPSFDATFIPTPAQSTKFPDIFSVASSRLSNSPPMTISTHMTTTQTGSSGATSKIP
LALDTSTLETSAGTPSVVTEGFAHSKITTAMNNDVKDVSQTNPPFQDEASSPSSQAPV
LVTTLPSSVAFTPQWHSTSSPVSMSSVLTSSLVKTAGKVDTSLETVTSSPQSMSNTLD
DISVTSAATTDIETTHPSINTVVTNVGTTGSAFESHSTVSAYPEPSKVTSPNVTTSTME
DTTISRSIPKSSKTTRTETETTSSLTPKLRETSISQEITSSTETSTVPYKELTGATTEVSRT
DVTSSSSTSFPGPDQSTVSLDISTETNTRLSTSPIMTESAEITITTQTGPHGATSQDTFTM
DPSNTTPQAGIHSAMTHGFSQLDVTTLMSRIPQDVSWTSPPSVDKTSSPSSFLSSPAM
TTPSLISSTLPEDKLSSPMTSLLTSGLVKITDILRTRLEPVTSSLPNFSSTSDKILATSKDS
KDTKEIFPSINTEETNVKANNSGHESHSPALADSETPKATTQMVITTTVGDPAPSTSM
PVHGSSETTNIKREPTYFLTPRLRETSTSQESSFPTDTSFLLSKVPTGTITEVSSTGVNSS
SKISTPDHDKSTVPPDTFTGEIPRVFTSSIKTKSAEMTITTQASPPESASHSTLPLDTSTT
LSQGGTHSTVTQGFPYSEVTTLMGMGPGNVSWMTTPPVEETSSVSSLMSSPAMTSPS
PVSSTSPQSIPSSPLPVTALPTSVLVTTTDVLGTTSPESVTSSPPNLSSITHERPATYKDT
AHTEAAMHHSTNTAVTNVGTSGSGHKSQSSVLADSETSKATPLMSTTSTLGDTSVST
STPNISQTNQIQTEPTASLSPRLRESSTSEKTSSTTETNTAFSYVPTGAITQASRTEISSS
RTSISDLDRPTIAPDISTGMITRLFTSPIMTKSAEMTVTTQTTTPGATSQGILPWDTSTT
LFQGGTHSTVSQGFPHSEITTLRSRTPGDVSWMTTPPVEETSSGFSLMSPSMTSPSPVS
STSPESIPSSPLPVTALLTSVLVTTTNVLGTTSPEPVTSSPPNLSSPTQERLTTYKDTAH
TEAMHASMHTNTAVANVGTSISGHESQSSVPADSHTSKATSPMGITFAMGDTSVSTS
TPAFFETRIQTESTSSLIPGLRDTRTSEEINTVTETSTVLSEVPTTTTTEVSRTEVITSSRT
TISGPDHSKMSPYISTETITRLSTFPFVTGSTEMAITNQTGPIGTISQATLTLDTSSTASW
EGTHSPVTQRFPHSEETTTMSRSTKGVSWQSPPSVEETSSPSSPVPLPAITSHSSLYSAV
SGSSPTSALPVTSLLTSGRRKTIDMLDTHSELVTSSLPSASSFSGEILTSEASTNTETIHF
SENTAETNMGTTNSMHKLHSSVSIHSQPSGHTPPKVTGSMMEDAIVSTSTPGSPETKN
VDRDSTSPLTPELKEDSTALVMNSTTESNTVFSSVSLDAATEVSRAEVTYYDPTFMP
ASAQSTKSPDISPEASSSHSNSPPLTISTHKTIATQTGPSGVTSLGQLTLDTSTIATSAGT
PSARTQDFVDSETTSVMNNDLNDVLKTSPFSAEEANSLSSQAPLLVTTSPSPVTSTLQ
EHSTSSLVSVTSVPTPTLAKITDMDTNLEPVTRSPQNLRNTLATSEATTDTHTMHPSIN
TAVANVGTTSSPNEFYFTVSPDSDPYKATSAVVITSTSGDSIVSTSMPRSSAMKKIESE
TTFSLIFRLRETSTSQKIGSSSDTSTVFDKAFTAATTEVSRTELTSSSRTSIQGTEKPTMS
PDTSTRSVTMLSTFAGLTKSEERTIATQTGPHRATSQGTLTWDTSITTSQAGTHSAMT
HGFSQLDLSTLTSRVPEYISGTSPPSVEKTSSSSSLLSLPAITSPSPVPTTLPESRPSSPVH

LTSLPTSGLVKTTDMLASVASLPPNLGSTSHKIPTTSEDIKDTEKMYPSTNIAVTNVGT
TTSEKESYSSVPAYSEPPKVTSPMVTSFNIRDTIVSTSMPGSSEITRIEMESTFSLAHGL
KGTSTSQDPIVSTEKSAVLHKLTTGATETSRTEVASSRRTSIPGPDHSTESPDISTEVIPS
LPISLGITESSNMTIITRTGPPLGSTSQGTFTLDTPTTSSRAGTHSMATQEFPHSEMTTV
MNKDPEILSWTIPPSIEKTSFSSSLMPSPAMTSPPVSSTLPKTIHTTPSPMTSLLTPSLV
MTTDTLGTSPEPTTSSPPNLSSTSHEILTTDEDTTAIEAMHPSTSTAATNVETTSSGHGS
QSSVLADSEKTKATAPMDTTSTMGHTTVSTSMSVSSETTKIKRESTYSLTPGLRETSIS
QNASFSTDTSIVLSEVPTGTTAEVSRTEVTSSGRTSIPGPSQSTVLPEISTRTMTRLFASP
TMTESAEMTIPTQTGPSGSTSQDTLTLDTSTTKSQAKTHSTLTQRFPHSEMTTLMSRG
PGDMSWQSSPSLENPSSLPSLLSLPATTSPPPISSTLPVTISSSPLPVTSLLTSSPVTTTD
MLHTSPELVTSSPPKLSHTSDERLTTGKDTTNTEAVHPSTNTAASNVEIPSSGHESPSS
ALADSETSKATSPMFITSTQEDTTVAISTPHFLETSRIQKESISSLSPKLRETGSSVETSS
AIETSAVLSEVSIGATTEISRTEVTSSSRTSISGSAESTMLPEISTTRKIIKFPTSPILAE
SSEMTIKTQTSPPGSTSESTFTLDTSTTPSLVITHSTMTQRLPHSEITTLVSRGAGDVPR
PSSLPVEETSPPSSQLSLSAMISPSPVSSTLPASSHSSSASVTSLLTPGQVKTTEVLDAS
AEPETSSPPSLSSTSVEILATSEVTTDTEKIHPFSNTAVTKVGTSSSGHESPSSVLPDSE
TTKATSAMGTISIMGDTSVSTLTPALSNTRKIQSEPASSLTTRLRETSTSEETSLATEAN
TVLSKVSTGATTEVSRTEAISFSRTSMSGPEQSTMSQDISIGTIPRISASSVLTESAKMT
ITTQTGPSESTLESTLNLNTATTPSWVETHSIVIQGFPHPEMTTSMGRGPGGVSWPSPP
FVKETSPPSSPLSLPAVTSPHPVSTTFLAHIPPSPLPVTSLLTSGPATTTDILGTSTEPGT
SSSSSLSTTSHERLTTYKDTAHTEAVHPSTNTGGTNVATTSSGYKSQSSVLADSSPMC
TTSTMGDTSVLTSTPAFLETRRIQTELASSLTPGLRESSGSEGTSSGTKMSTVLSKVPT
GATTEISKEDVTSIPGPAQSTISPDISTRTVSWFSTSPVMTESAEITMNTHTSPLGATTQ
GTSTLDTSSTTSLTMTHSTISQGFSHSQMSTLMRRGPEDVSWMSPPLLEKTRPSFSLM
SSPATTSPSPVSSTLPESISSSPLPVTSLLTSGLAKTTDMLHKSSEPVTNSPANLSSTSVE
ILATSEVTTDTEKTHPSSNRTVTDVGTSSSGHESTSFVLADSQTSKVTSPMVITSTMED
TSVSTSTPGFFETSRIQTEPTSSLTLGLRKTSSSEGTSLATEMSTVLSGVPTGATAEVSR
TEVTSSSRTSISGFAQLTVSPETSTETITRLPTSSIMTESAEMMIKTQTDPPGSTPESTHT
VDISTTPNWVETHSTVTQRFSHSEMTTLVSRSPGDMLWPSQSSVEETSSASSLLSLPA
TTSPSPVSSTLVEDFPSASLPVTSLLNPGLVITTDRMGISREPGTSSTSNLSSTSHERLTT
LEDTVDTEDMQPSTHTAVTNVRTSISGHESQSSVLSDSETPKATSPMGTTYTMGETS
VSISTSDFFETSRIQIEPTSSLTSGLRETSSSERISSATEGSTVLSEVPSGATTEVSRTEVIS
SRGTSMSGPDQFTISPDISTEAITRLSTSPIMTESAESAITIETGSPGATSEGTLTLDTSTT
TFWSGTHSTASPGFSHSEMTTLMSRTPGDVPWPSLPSVEEASSVSSSLSSPAMTSTSFF
STLPESISSSPHPVTALLTLGPVKTTDMLRTSSEPETSSPPNLSSTSAEILATSEVTKDRE
KIHPSSNTPVVNVGTVIYKHLSPSSVLADLVTTKPTSPMATTSTLGNTSVSTSTPAFPE
TMMTQPTSSLTSGLREISTSQETSSATERSASLSGMPTGATTKVSRTEALSLGRTSTPG
PAQSTISPEISTETITRISTPLTTTGSAEMTITPKTGHSGASSQGTFTLDTSSRASWPGTH
SAATHRSPHSGMTTPMSRGPEDVSWPSRPSVEKTSPPSSLVSLSAVTSPSPLYSTPSES
SHSSPLRVTSLFTPVMMKTTDMLDTSLEPVTTSPPSMNITSDESLATSKATMETEAIQ
LSENTAVTQMGTISARQEFYSSYPGLPEPSKVTSPVVTSSTIKDIVSTTIPASSEITRIEM
ESTSTLTPTPRETSTSQEIHSATKPSTVPYKALTSATIEDSMTQVMSSSRGPSPDQSTM
SQDISTEVITRLSTSPIKTESTEMTITTQTGSPGATSRGTLTLDTSTTFMSGTHSTASQG
FSHSQMTALMSRTPGDVPWLSHPSVEEASSASFSLSSPVMTSSSPVSSTLPDSIHSSSLP
VTSLLTSGLVKTTELLGTSSEPETSSPPNLSSTSAEILAITEVTTDTEKLEMTNVVTSGY
THESPSSVLADSVTTKATSSMGITYPTGDTNVLTSTPAFSDTSRIQTKSKLSLTPGLME
TSISEETSSATEKSTVLSSVPTGATTEVSRTEAISSSRTSIPGPAQSTMSSDTSMETITRIS
TPLTRKESTDMAITPKTGPSGATSQGTFTLDSSSTASWPGTHSATTQRFPQSVVTTPM
SRGPEDVSWPSPLSVEKNSPPSSLVSSSSVTSPSPLYSTPSGSSHSSPVPVTSLFTSIMM
KATDMLDASLEPETTSAPNMNITSDESLAASKATTETEAIHVFENTAASHVETTSATE

ELYSSSPGFSEPTKVISPVVTSSSIRDNMVSTTMPGSSGITRIEIESMSSLTPGLRETRTS
QDITSSTETSTVLYKMPSGATPEVSRTEVMPSSRTSIPGPAQSTMSLDISDEVVTRLST
SPIMTESAEITITTQTGYSLATSQVTLPLGTSMTFLSGTHSTMSQGLSHSEMTNLMSRG
PESLSWTSPRFVETTRSSSSLTSLPLTTSLSPVSSTLLDSSPSSPLPVTSLILPGLVKTTEV
LDTSSEPKTSSSPNLSSTSVEIPATSEIMTDTEKIHPSSNTAVAKVRTSSSVHESHSSVL
ADSETTITIPSMGITSAVDDTTVFTSNPAFSETRRIPTEPTFSLTPGFRETSTSEETTSITE
TSAVLYGVPTSATTEVSMTEIMSSNRIHIPDSDQSTMSPDIITEVITRLSSSSMMSESTQ
MTITTQKSSPGATAQSTLTLATTTAPLARTHSTVPPRFLHSEMTTLMSRSPENPSWKS
SLFVEKTSSSSSLLSLPVTTSPSVSSTLPQSIPSSSFSVTSLLTPGMVKTTDTSTEPGTSLS
PNLSGTSVEILAASEVTTDTEKIHPSSSMAVTNVGTTSSGHELYSSVSIHSEPSKATYP
VGTPSSMAETSISTSMPANFETTGFEAEPFSHLTSGFRKTNMSLDTSSVTPTNTPSSPG
STHLLQSSKTDFTSSAKTSSPDWPPASQYTEIPVDIITPFNASPSITESTGITSFPESRFTM
SVTESTHHLSTDLLPSAETISTGTVMPSLSEAMTSFATTGVPRAISGSGSPFSRTESGPG
DATLSTIAESLPSSTPVPFSSSTFTTTDSSTIPALHEITSSSATPYRVDTSLGTESSTTEGR
LVMVSTLDTSSQPGRTSSSPILDTRMTESVELGTVTSAYQVPSLSTRLTRTDGIMEHIT
KIPNEAAHRGTIRPVKGPQTSTSPASPKGLHTGGTKRMETTTTALKTTTTALKTTSRA
TLTTSVYTPTLGTLTPLNASMQMASTIPTEMMITTPYVFPDVPETTSSLATSLGAETST
ALPRTTPSVFNRESETTASLVSRSGAERSPVIQTLDVSSSEPDTTASWVIHPAETIPTVS
KTTPNFFHSELDTVSSTATSHGADVSSAIPTNISPSELDALTPLVTISGTDTSTTFPTLTK
SPHETETRTTWLTHPAETSSTIPRTIPNFSHHESDATPSIATSPGAETSSAIPIMTVSPGA
EDLVTSQVTSSGTDRNMTIPTLTLSPGEPKTIASLVTHPEAQTSSAIPTSTISPAVSRLV
TSMVTSLAAKTSTTNRALTNSPGEPATTVSLVTHPAQTSPTVPWTTSIFFHSKSDTTPS
MTTSHGAESSSAVPTPTVSTEVPGVVTPLVTSSRAVISTTIPILTLSPGEPETTPSMATS
HGEEASSAIPTPTVSPGVPGVVTSLVTSSRAVTSTTIPILTFSLGEPETTPSMATSHGTE
AGSAVPTVLPEVPGMVTSLVASSRAVTSTTLPTLTLSPGEPETTPSMATSHGAEASST
VPTVSPEVPGVVTSLVTSSSGVNSTSIPTLILSPGELETTPSMATSHGAEASSAVPTPTV
SPGVSGVVTPLVTSSRAVTSTTIPILTLSSSEPETTPSMATSHGVEASSAVLTVSPEVPG
MVTSLVTSSRAVTSTTIPTLTISSDEPETTTSLVTHSEAKMISAIPTLAVSPTVQGLVTS
LVTSSGSETSAFSNLTVASSQPETIDSWVAHPGTEASSVVPTLTVSTGEPFTNISLVTH
PAESSSTLPRTTSRFSHSELDTMPSTVTSPEAESSSAISTTISPGIPGVLTSLVTSSGRDIS
ATFPTVPESPHESEATASWVTHPAVTSTTVPRTTPNYSHSEPDTTPSIATSPGAEATSD
FPTITVSPDVPDMVTSQVTSSGTDTSITIPTLTLSSGEPETTTSFITYSETHTSSAIPTLPV
SPGASKMLTSLVISSGTDSTTTFPTLTETPYEPETTAIQLIHPAETNTMVPRTTPKFSHS
KSDTTLPVAITSPGPEASSAVSTTTISPDMSDLVTSLVPSSGTDTSTTFPTLSETPYEPET
TATWLTHPAETSTTVSGTIPNFSHRGSDTAPSMVTSPGVDTRSGVPTTTIPPSIPGVVT
SQVTSSATDTSTAIPTLTPSPGEPETTASSATHPGTQTGFTVPIRTVPSSEPDTMASWV
THPPQTSTPVSRTTSSFSHSSPDATPVMATSPRTEASSAVLTTISPGAPEMVTSQITSSG
AATSTTVPTLTHSPGMPETTALLSTHPRTETSKTFPASTVFPQVSETTASLTIRPGAETS
TALPTQTTSSLFTLLVTGTSRVDLSPTASPGVSAKTAPLSTHPGTETSTMIPTSTLSLGL
LETTGLLATSSSAETSTSTLTLTVSPAVSGLSSASITTDKPQTVTSWNTETSPSVTSVGP
PEFSRTVTGTTMTLIPSEMPTPPKTSHGEGVSPTTILRTTMVEATNLATTGSSPTVAKT
TTTFNTLAGSLFTPLTTPGMSTLASESVTSRTSYNHRSWISTTSSYNRRYWTPATSTPV
TSTFSPGISTSSIPSSTAATVPFMVPFTLNFTITNLQYEEDMRHPGSRKFNATERELQGL
LKPLFRNSSLEYLYSGCRLASLRPEKDSSATAVDAICTHRPDPEDLGLDRERLYWELS
NLTNGIQELGPYTLDRNSLYVNGFTHRSSMPTTSTPGTSTVDVGTSGTPSSSPSPTTAG
PLLMPFTLNFTITNLQYEEDMRRTGSRKFNTMESVLQGLLKPLFKNTSVGPLYSGCR
LTLLRPEKDGAATGVDAICTHRLDPKSPGLNREQLYWELSKLTNDIEELGPYTLDRN
SLYVNGFTHQSSVSTTSTPGTSTVDLRTSGTPSSLSSPTIMAAGPLLVPFTLNFTITNLQ
YGEDMGHPGSRKFNTTERVLQGLLGPIFKNTSVGPLYSGCRLTSLRSEKDGAATGVD
AICIHHLDPKSPGLNRERLYWELSQLTNGIKELGPYTLDRNSLYVNGFTHRTSVPTSS

TPGTSTVDLGTSGTPFSLPSPATAGPLLVLFTLNFTITNLKYEEDMHRPGSRKFNTTER
VLQTLLGPMFKNTSVGLLYSGCRLTLLRSEKDGAATGVDAICTHRLDPKSPGVDREQ
LYWELSQLTNGIKELGPYTLDRNSLYVNGFTHWIPVPTSSTPGTSTVDLGSGTPSSLPS
PTTAGPLLVPFTLNFTITNLKYEEDMHCPGSRKFNTTERVLQSLLGPMFKNTSVGPLY
SGCRLTLLRSEKDGAATGVDAICTHRLDPKSPGVDREQLYWELSQLTNGIKELGPYT
LDRNSLYVNGFTHQTSAPNTSTPGTSTVDLGTSGTPSSLPSPTSAGPLLVPFTLNFTIT
NLQYEEDMHHPGSRKFNTTERVLQGLLGPMFKNTSVGLLYSGCRLTLLRPEKNGAA
TGMDAICSHRLDPKSPGLNREQLYWELSQLTHGIKELGPYTLDRNSLYVNGFTHRSS
VAPTSTPGTSTVDLGTSGTPSSLPSPTTAVPLLVPFTLNFTITNLQYGEDMRHPGSRKF
NTTERVLQGLLGPLFKNSSVGPLYSGCRLISLRSEKDGAATGVDAICTHHLNPQSPGL
DREQLYWQLSQMTNGIKELGPYTLDRNSLYVNGFTHRSSGLTTSTPWTSTVDLGTSG
TPSPVPSPTTTGPLLVPFTLNFTITNLQYEENMGHPGSRKFNITESVLQGLLKPLFKSTS
VGPLYSGCRLTLLRPEKDGVATRVDAICTHRPDPKIPGLDRQQLYWELSQLTHSITEL
GPYTLDRDSLYVNGFTQRSSVPTTSTPGTFTVQPETSETPSSLPGPTATGPVLLPFTLN
FTITNLQYEEDMRRPGSRKFNTTERVLQGLLMPLFKNTSVSSLYSGCRLTLLRPEKDG
AATRVDAVCTHRPDPKSPGLDRERLYWKLSQLTHGITELGPYTLDRHSLYVNGFTH
QSSMTTTRTPDTSTMHLATSRTPASLSGPMTASPLLVLFTINFTITNLRYEENMHHPG
SRKFNTTERVLQGLLRPVFKNTSVGPLYSGCRLTLLRPKKDGAATKVDAICTYRPDP
KSPGLDREQLYWELSQLTHSITELGPYTLDRDSLYVNGFTQRSSVPTTSIPGTPTVDLG
TSGTPVSKPGPSAASPLLVLFTLNFTITNLRYEENMQHPGSRKFNTTERVLQGLLRSLF
KSTSVGPLYSGCRLTLLRPEKDGTATGVDAICTHHPDPKSPRLDREQLYWELSQLTH
NITELGPYALDNDSLFVNGFTHRSSVSTTSTPGTPTVYLGASKTPASIFGPSAASHLLIL
FTLNFTITNLRYEENMWPGSRKFNTTERVLQGLLRPLFKNTSVGPLYSGCRLTLLRPE
KDGEATGVDAICTHRPDPTGPGLDREQLYLELSQLTHSITELGPYTLDRDSLYVNGFT
HRSSVPTTSTGVVSEEPFTLNFTINNLRYMADMGQPGSLKFNITDNVMQHLLSPLFQR
SSLGARYTGCRVIALRSVKNGAETRVDLLCTYLQPLSGPGLPIKQVFHELSQQTHGIT
RLGPYSLDKDSLYLNGYNEPGPDEPPTTPKPATTFLPPLSEATTAMGYHLKTLTLNFT
ISNLQYSPDMGKGSATFNSTEGVLQHLLRPLFQKSSMGPFYLGCQLISLRPEKDGAAT
GVDTTCTYHPDPVGPGLDIQQLYWELSQLTHGVTQLGFYVLDRDSLFINGYAPQNLS
IRGEYQINFHIVNWNLSNPDPTSSEYITLLRDIQDKVTTLYKGSQLHDTFRFCLVTNLT
MDSVLVTVKALFSSNLDPSLVEQVFLDKTLNASFHWLGSTYQLVDIHVTEMESSVY
QPTSSSSTQHFYLNFTITNLPYSQDKAQPGTTNYQRNKRNIEDALNQLFRNSSIKSYFS
DCQVSTFRSVPNRHHTGVDSLCNFSPLARRVDRVAIYEEFLRMTRNGTQLQNFTLDR
SSVLVDGYSPNRNEPLTGNSDLPFWAVILIGLAGLLGVITCLICGVLVTTRRRKKEGE
YNVQQQCPGYYQSHLDLEDLQ

Antigen-binding sites that can bind to tumor associated antigen NaPi2b can be identified by screening for binding to the amino acid sequence defined by SEQ ID NO:149.

SEQ ID NO:149

MAPWPELGDAQPNPDKYLEGAAGQQPTAPDKSKETNKTDNTEAPVTKIELLPSYST
ATLIDEPTEVDDPWNLPTLQDSGIKWSERDTKGKILCFFQGIGRLILLLGFLYFFVCSL
DILSSAFQLVGGKMAGQFFSNSSIMSNPLLGLVIGVLVTVLVQSSSTSTSIVVSMVSSS
LLTVRAAIPIIMGANIGTSITNTIVALMQVGDRSEFRRAFAGATVHDFFNWLSVLVLL
PVEVATHYLEIITQLIVESFHFKNGEDAPDLLKVITKPFTKLIVQLDKKVISQIAMNDE
KAKNKSLVKIWCKTFTNKTQINVTVPSTANCTSPSLCWTDGIQNWTMKNVTYKENI
AKCQHIFVNFHLPDLAVGTILLILSLLVLCGCLIMIVKILGSVLKGQVATVIKKTINTDF
PFPFAWLTGYLAILVGAGMTFIVQSSSVFTSALTPLIGIGVITIERAYPLTLGSNIGTTTT
AILAALASPGNALRSSLQIALCHFFFNISGILLWYPIPFTRLPIRMAKGLGNISAKYRWF

AVFYLIIFFFLIPLTVFGLSLAGWRVLVGVGVPVVFIIILVLCLRLLQSRCPRVLPKKLQ
NWNFLPLWMRSLKPWDAVVSKFTGCFQMRCCCCCRVCCRACCLLCDCPKCCRCSK
CCEDLEEAQEGQDVPVKAPETFDNITISREAQGEVPASDSKTECTAL

Antigen-binding sites that can bind to tumor associated antigen Nectin4 can be identified by screening for binding to the amino acid sequence defined by SEQ ID NO:150.

SEQ ID NO:150

MPLSLGAEMWGPEAWLLLLLLLASFTGRCPAGELETSDVVTVVLGQDAKLPCFYRG
DSGEQVGQVAWARVDAGEGAQELALLHSKYGLHVSPAYEGRVEQPPPPRNPLDGS
VLLRNAVQADEGEYECRVSTFPAGSFQARLRLRVLVPPLPSLNPGPALEEGQGLTLA
ASCTAEGSPAPSVTWDTEVKGTTSSRSFKHSRSAAVTSEFHLVPSRSMNGQPLTCVV
SHPGLLQDQRITHILHVSFLAEASVRGLEDQNLWHIGREGAMLKCLSEGQPPPSYNW
TRLDGPLPSGVRVDGDTLGFPPLTTEHSGIYVCHVSNEFSSRDSQVTVDVLDPQEDSG
KQVDLVSASVVVVGVIAALLFCLLVVVVVLMSRYHRRKAQQMTQKYEEELTLTRE
NSIRRLHSHHTDPRSQPEESVGLRAEGHPDSLKDNSSCSVMSEEPEGRSYSTLTTVREI
ETQTELLSPGSGRAEEEEDQDEGIKQAMNHFVQENGTLRAKPTGNGIYINGRGHLV

Antigen-binding sites that can bind to tumor associated antigen Fucosyl-GM1 can be identified by screening for binding to monosialotetrahexosylganglioside.

Antigen-binding sites that can bind to tumor associated antigen ADAM8 can be identified by screening for binding to the amino acid sequence defined by SEQ ID NO:151.

SEQ ID NO:151

LGATGHNFTLHLRKNRDLLGSGYTETYTAANGSEVTEQPRGQDHCFYQGHVEGYPD
SAASLSTCAGLRGFFQVGSDLHLIEPLDEGGEGGRHAVYQAEHLLQTAGTCGVSDDS
LGSLLGPRTAAVFRPRPGDSLPSRETRYVELYVVVDNAEFQMLGSEAAVRHRVLEV
VNHVDKLYQKLNFRVVLVGLEIWNSQDRFHVSPDPSVTLENLLTWQARQRTRRHLH
DNVQLITGVDFTGTTVGFARVSAMCSHSSGAVNQDHSKNPVGVACTMAHEMGHNL
GMDHDENVQGCRCQERFEAGRCIMAGSIGSSFPRMFSDCSQAYLESFLERPQSVCLA
NAPDLSHLVGGPVCGNLFVERGEQCDCGPPEDCRNRCCNSTTCQLAEGAQCAHGTC
CQECKVKPAGELCRPKKDMCDLEEFCDGRHPECPEDAFQENGTPCSGGYCYNGACP
TLAQQCQAFWGPGGQAAEESCFSYDILPGCKASRYRADMCGVLQCKGGQQPLGRAI
CIVDVCHALTTEDGTAYEPVPEGTRCGPEKVCWKGRCQDLHVYRSSNCSAQCHNH
GVCNHKQECHCHAGWAPPHCAKLLTEVHAASGSLPVFVVVVLVLLAVVLVTLAGII
VYRKARSRILSRNVAPKTTMGRSNPLFHQAASRVPAKGGAPAPSRGPQELVPTTHPG
QPARHPASSVALKRPPPAPPVTVSSPPFPVPVYTRQAPKQVIKPTFAPPVPPVKPGAG
AANPGPAEGAVGPKVALKPPIQRKQGAGAPTAP

Antigen-binding sites that can bind to tumor associated antigen ADAM9 can be identified by screening for binding to the amino acid sequence defined by SEQ ID NO:152.

SEQ ID NO:152

MGSGARFPSGTLRVRWLLLLGLVGPVLGAARPGFQQTSHLSSYEIITPWRLTRERRE
APRPYSKQVSYVIQAEGKEHIIHLERNKDLLPEDFVVYTYNKEGTLITDHPNIQNHCH
YRGYVEGVHNSSIALSDCFGLRGLLHLENASYGIEPLQNSSHFEHIIYRMDDVYKEPL
KCGVSNKDIEKETAKDEEEEPPSMTQLLRRRRAVLPQTRYVELFIVVDKERYDMMG

RNQTAVREEMILLANYLDSMYIMLNIRIVLVGLEIWTNGNLINIVGGAGDVLGNFVQ
WREKFLITRRRHDSAQLVLKKGFGGTAGMAFVGTVCSRSHAGGINVFGQITVETFAS
IVAHELGHNLGMNHDDGRDCSCGAKSCIMNSGASGSRNFSSCSAEDFEKLTLNKGG
NCLLNIPKPDEAYSAPSCGNKLVDAGEECDCGTPKECELDPCCEGSTCKLKSFAECA
YGDCCKDCRFLPGGTLCRGKTSECDVPEYCNGSSQFCQPDVFIQNGYPCQNNKAYC
YNGMCQYYDAQCQVIFGSKAKAAPKDCFIEVNSKGDRFGNCGFSGNEYKKCATGN
ALCGKLQCENVQEIPVFGIVPAIIQTPSRGTKCWGVDFQLGSDVPDPGMVNEGTKCG
AGKICRNFQCVDASVLNYDCDVQKKCHGHGVCNSNKNCHCENGWAPPNCETKGY
GGSVDSGPTYNEMNTALRDGLLVFFFLIVPLIVCAIFIFIKRDQLWRSYFRKKRSQTYE
SDGKNQANPSRQPGSVPRHVSPVTPPREVPIYANRFAVPTYAAKQPQQFPSRPPPPQP
KVSSQGNLIPARPAPAPPLYSSLT

Antigen-binding sites that can bind to tumor associated antigen SLC44A4 can be identified by screening for binding to the amino acid sequence defined by SEQ ID NO:153.

SEQ ID NO:153

MGGKQRDEDDEAYGKPVKYDPSFRGPIKNRSCTDVICCVLFLLFILGYIVVGIVAWL
YGDPRQVLYPRNSTGAYCGMGENKDKPYLLYFNIFSCILSSNIISVAENGLQCPTPQV
CVSSCPEDPWTVGKNEFSQTVGEVFYTKNRNFCLPGVPWNMTVITSLQQELCPSFLL
PSAPALGRCFPWTNVTPPALPGITNDTTIQQGISGLIDSLNARDISVKIFEDFAQSWYW
ILVALGVALVLSLLFILLLRLVAGPLVLVLILGVLGVLAYGIYYCWEEYRVLRDKGAS
ISQLGFTTNLSAYQSVQETWLAALIVLAVLEAILLLMLIFLRQRIRIAIALLKEASKAV
GQMMSTMFYPLVTFVLLLICIAYWAMTALYLATSGQPQYVLWASNISSPGCEKVPIN
TSCNPTAHLVNSSCPGLMCVFQGYSSKGLIQRSVFNLQIYGVLGLFWTLNWVLALG
QCVLAGAFASFYWAFHKPQDIPTFPLISAFIRTLRYHTGSLAFGALILTLVQIARVILEY
IDHKLRGVQNPVARCIMCCFKCCLWCLEKFIKFLNRNAYIMIAIYGKNFCVSAKNAF
MLLMRNIVRVVVLDKVTDLLLFFGKLLVVGGVGVLSFFFFSGRIPGLGKDFKSPHLN
YYWLPIMTSILGAYVIASGFFSVFGMCVDTLFLCFLEDLERNNGSLDRPYYMSKSLL
KILGKKNEAPPDNKKRKK

Antigen-binding sites that can bind to tumor associated antigen CA19-9 can be identified by screening for binding to the amino acid sequence defined by SEQ ID NO:154.

SEQ ID NO:154

MACSRPPSQCEPTSLPPGPPAGRRHLPLSRRRREMSSNKEQRSAVFVILFALITILILYS
SNSANEVFHYGSLRGRSRRPVNLKKWSITDGYVPILGNKTLPSRCHQCVIVSSSSHLL
GTKLGPEIERAECTIRMNDAPTTGYSADVGNKTTYRVVAHSSVFRVLRRPQEFVNRT
PETVFIFWGPPSKMQKPQGSLVRVIQRAGLVFPNMEAYAVSPGRMRQFDDLFRGET
GKDREKSHSWLSTGWFTMVIAVELCDHVHVYGMVPPNYCSQRPRLQRMPYHYYEP
KGPDECVTYIQNEHSRKGNHHRFITEKRVFSSWAQLYGITFSHPSWT

Alternatively, Table 3 lists peptide sequences of heavy chain variable domains and light chain variable domains that, in combination, can bind to CA125 (abagovomab, sofituzumab), NaPi2b (lifastuzumab), Nectin4 (enfortumab), Fucosyl-GM1 (described in US Patent Application Publication No.: 20130142789, specific sequences are incorporated by reference herein), or SLC44A4 (described in International Application Publication No.: WO2010111018, specific sequences are incorporated by reference herein).

| Table 3 | | |
|---|---|---|
| Clones | Heavy chain variable domain amino acid sequence | Light chain variable domain amino acid sequence |
| abagovomab | QVKLQESGAELARPGASVKLSC KASGYTFTNYWMQWVKQRPGQ GLDWIGAIYPGDGNTRYTHKFK GKATLTADKSSSTAYMQLSSLAS EDSGVYYCARGEGNYAWFAYW GQGTTVTVSSA (SEQ ID NO:155) CDR1 (SEQ ID NO:156) - GYTFTNY CDR2 (SEQ ID NO:157) - YPGDGN CDR3 (SEQ ID NO:158) - GEGNYAWFAY | DIELTQSPASLSASVGETVTITCQA SENIYSYLAWHQQKQGKSPQLLV YNAKTLAGGVSSRFSGSGSGTHFS LKIKSLQPEDFGIYYCQHHYGILPT FGGGTKLEIKR (SEQ ID NO:159) CDR1(SEQ ID NO:160) - ENIYSYLA CDR2 (SEQ ID NO:161) - NAKTLAG CDR3 (SEQ ID NO:162) - QHHYGILPT |
| sofituzumab | EVQLVESGGGLVQPGGSLRLSCA ASGYSITNDYAWNWVRQAPGK GLEWVGYISYSGYTTYNPSLKSR FTISRDTSKNTLYLQMNSLRAED TAVYYCARWTSGLDYWGQGTL VTVSSA (SEQ ID NO:163) CDR1 (SEQ ID NO:164) - GYSITNDY CDR2 (SEQ ID NO:165) - SYSGY CDR3 (SEQ ID NO:166) - WTSGLDY | DIQMTQSPSSLSASVGDRVTITCKA SDLIHNWLAWYQQKPGKAPKLLI YGATSLETGVPSRFSGSGSGTDFTL TISSLQPEDFATYYCQQYWTTPFTF GQGTKVEIKR (SEQ ID NO:167) CDR1 (SEQ ID NO:168) - DLIHNWLA CDR2 (SEQ ID NO:169) - GATSLET CDR3 (SEQ ID NO:170) - QQYWTTPFT |
| | | |
| lifastuzumab | EVQLVESGGGLVQPGGSLRLSCA ASGFSFSDFAMSWVRQAPGKGL EWVATIGRVAFHTYYPDSMKGR FTISRDNSKNTLYLQMNSLRAED TAVYYCARHRGFDVGHFDFWG QGTLVTVSSA (SEQ ID NO:171) CDR1 (SEQ ID NO:172) - GFSFSDF CDR2 (SEQ ID NO:173) - GRVAFH CDR3 (SEQ ID NO:174) - HRGFDVGHFDF | DIQMTQSPSSLSASVGDRVTITCRS SETLVHSSGNTYLEWYQQKPGKA PKLLIYRVSNRFSGVPSRFSGSGSG TDFTLTISSLQPEDFATYYCFQGSF NPLTFGQGTKVEIKR(SEQ ID NO:175) CDR1 (SEQ ID NO:176) - ETLVHSSGNTYLE CDR2 (SEQ ID NO:177) - RVSNRFS CDR3 (SEQ ID NO:178) - FQGSFNPLT |
| enfortumab | EVQLVESGGGLVQPGGSLRLSCA ASGFTFSSYNMNWVRQAPGKGL EWVSYISSSSSTIYYADSVKGRFT ISRDNAKNSLSLQMNSLRDEDTA VYYCARAYYYGMDVWGQGTTV TVSSA (SEQ ID NO:179) CDR1 (SEQ ID NO:180) - GFTFSSY CDR2 (SEQ ID NO:181) - SSSSST CDR3 (SEQ ID NO:182) - AYYYGMDV | DIQMTQSPSSVSASVGDRVTITCRA SQGISGWLAWYQQKPGKAPKFLIY AASTLQSGVPSRFSGSGSGTDFTLT ISSLQPEDFATYYCQQANSFPPTFG GGTKVEIKR (SEQ ID NO:183) CDR1(SEQ ID NO:184) - QGISGWLA CDR2 (SEQ ID NO:185) - AASTLQS CDR3 (SEQ ID NO:186) - QQANSFPPT |
| Anti- Fucosyl-GM1 | EVQLVESGGGSVQPGESLRLSCV ASGFTFSRYKMNWVRQAPGKGL EWVSYISRSGRDIYYADSVKGRF TISRDNAKNSLYLQMNSLRDEDT AVYYCAGTVTTYYYDFGMDVW GQGTTVTVSS (SEQ ID NO:187) CDR1 (SEQ ID NO:188) - GFTFSRY CDR2 (SEQ ID NO:189) - SRSGRD CDR3 (SEQ ID NO:190) - TVTTYYYDFGMDV | DIQMTQSPSSLSASVGDRVTITCRA SQGISSWLAWYQQKPEKAPKSLIY AASSLQSGVPSRFSGSGSGTDFTLT ISSLQPEDFATYYCQQYNSYPPTFG GGTKVEIK (SEQ ID NO:191) CDR1 (SEQ ID NO:192)-QGISSWLA CDR2 (SEQ ID NO:193) - AASSLQS CDR3 (SEQ ID NO:194) - QQYNSYPPT |
| Anti-SLC44A4 | QVQLVESGGGVVQPGRSLRLSC AASGFTFSSYGMHWVRQAPGKG LEWVAVMSYDGSKKFYTDSVK GRFTISRDNSKNTLYLQMNSLRA EDTAVYYCARDGGDYVRYHYY GMDVWGQGTTVTVSSA (SEQ ID NO:195) CDR1 (SEQ ID NO:196) - GFTFSSY CDR2 (SEQ ID NO:197) - SYDGSK CDR3 (SEQ ID NO:198) - DGGDYVRYHYYGMDV | DIQMTQSPSTLSASIGDRVTITCRA SQGISYYLAWYQQKPGKIPKLLIY DTSSLQSGVPSRFSGSRSGTDLSLTI SSLQPEDVATYYCQRYDSAPLTFG GGTKVEIKR (SEQ ID NO:199) CDR1 (SEQ ID NO:200) - QGISYYLA CDR2 (SEQ ID NO:201) - DTSSLQS CDR3 (SEQ ID NO:202) - QRYDSAPLT |

Listed below are examples of the scFv linked to an antibody constant region that also includes mutations that enable heterodimerization of two polypeptide chains. The scFv containing a heavy chain variable domain (V_{H}) and a light chain variable domain (V_{L}) from an anti-NKG2D antibody is used in preparing a multispecific protein of the present disclosure. Each sequence represents V_{L}-(G4S)₄-V_{H}-hinge (AS or GAS)-Fc containing heterodimerization mutations (underlined). V_{L} and V_{H} contain 100V_{L} - 44V_{H} S-S bridge (underlined), and can be from any tumor targeting or NKG2D binding antibody. The Ala-Ser (AS, bolded & underlined) is included at the elbow hinge region sequence to balance between flexibility and optimal geometry. In certain embodiments, an additional Gly can be added to the N-terminus of the AS sequence, generating a hinge having the sequence of Gly-Ala-Ser (GAS, bolded & underlined). In certain embodiments, an additional sequence Thr-Lys-Gly can be added to the AS sequence at the hinge. (G4S)₄ linker is underlined in the sequences listed in the paragraph below.

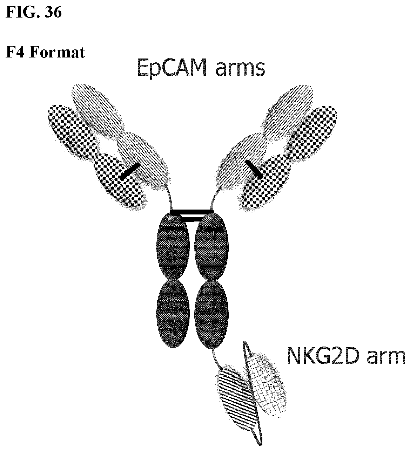

A TriNKET of the present disclosure is NKG2D-binding-F4-TriNKET-EpCAM comprising a first polypeptide comprising the sequence of SEQ ID NO:203 (F4- EpCAMFc-AJchainB-NKG2D-binding scFv), and a second polypeptide comprising the sequence of SEQ ID NO:204 (Anti-EpCAM HC-hinge-Fc). The NKG2D-binding-F4-TriNKET-EpCAM also comprises two EpCAM-targeting light chains each comprising an anti-EpCAM V_{L}- Constant domain comprising the sequence of SEQ ID NO:214. For example, in the structure of FIG. 36, when the Fab fragments target EpCAM, the NKG2D-binding-F4-TriNKET-EpCAM includes SEQ ID NO:203 and SEQ ID NO:214 forming one arm of the TriNKET, and SEQ ID NO:204 and SEQ ID NO:214 forming the second arm of the TriNKET.

Each of the arms comprises an EpCAM-binding Fab fragment, which comprises a heavy chain portion comprising a heavy chain variable domain and a CH1 domain, in which the heavy chain variable domain is connected to the CH1 domain; and a light chain portion comprising a light chain variable domain and a light chain constant domain (SEQ ID NO:214). In the first arm *(e.g.,* in F4- EpCAMFc-AJchainB-NKG2D-binding scFv) the CH1 domain is connected to the Fc domain, which is connected to an scFv-targeting NKG2D, forming a polypeptide comprising the sequence of SEQ ID NO:203. In the second arm, the CH1 domain is connected to the Fc domain, forming a polypeptide comprising the sequence of SEQ ID NO:204.

For example, F4-EpCAMFc-AJchainB-NKG2D-binding scFv (SEQ ID NO:203) comprises a EpCAM-targeting heavy chain variable domain (V_{H}) (SEQ ID NO: 139) and a CH1 domain connected to an Fc domain (hinge-CH2-CH3), which at the C-terminus of the Fc is linked to a single-chain variable fragment (scFv) that binds NKG2D. The Fc domain in SEQ ID NO:203 comprises a S354C substitution, which forms a disulfide bond with a Y349C substitution in another Fc domain (SEQ ID NO:204, described below). The Fc domain in SEQ ID NO:203 includes Q347R, D399V, and F405T substitutions. The scFv that binds NKG2D is represented by the amino acid sequence of SEQ ID NO:205, and includes a light chain variable domain (V_{L}) linked to an heavy chain variable domain (V_{H}) *via* a (G4S)₄ linker, GGGGSGGGGSGGGGSGGGGS (SEQ ID NO:206). The V_{L} and V_{H} comprised within SEQ ID NO:205 are connected as V_{L}-(G4S)₄-V_{H}; V_{L} and V_{H} contain 100V_{L} - 44V_{H} S-S bridge (resulting from G100C and G44C substitutions, respectively) (cysteine residues are bold-italics-underlined). As represented in SEQ ID NO:203, the C-terminus of the Fc domain is linked to the N-terminus of the scFv (SEQ ID NO:205) *via* a short SGSGGGGS linker (SEQ ID NO:207).

### NKG2D-binding scFv

DIQMTQSPSSVSASVGDRVTITCRASQGISSWLAWYQQKPGKAPKLLIYAASSLQSG
VPSRFSGSGSGTDFTLTISSLQPEDFATYYCQQGVSFPRTFGCGTKVEIKGGGGSGGG
GSGGGGSGGGGSEVQLVESGGGLVKPGGSLRLSCAASGFTFSSYSMNWVRQAPGKC
LEWVSSISSSSSYIYYADSVKGRFTISRDNAKNSLYLQMNSLRAEDTAVYYCARGAP
MGAAAGWFDPWGQGTLVTVSS (SEQ ID NO:205)

### F4-EpCAMFc-AJchainB-NKG2D-binding scFv

EVQLLEQSGAELVRPGTSVKISCKASGYAFTNYWLGWVKQRPGHGLEWIGDIFPGSG
NIHYNEKFKGKATLTADKSSSTAYMQLSSLTFEDSAVYFCARLRNWDEPMDYWGQ
GTTVTVSSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKVEPKSCDKT
HTCPPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIEKTI
SKAKGQPREPRVYTLPPCRDELTKNQVSLTCLVKGFYPSDIAVEWESNGQPENNYKT
TPPVLVSDGSFTLYSKLTVDKSRWQQGNVFSCSVMHEALHNHYTQKSLSLSPG
SGSGGGGSDIQMTQSPSSVSASVGDRVTITCRASQGISSWLAWYQQKPGKAPKLLIY
AASSLQSGVPSRFSGSGSGTDFTLTISSLQPEDFATYYCQQGVSFPRTFGCGTKVEIKG
GGGSGGGGSGGGGSGGGGSEVQLVESGGGLVKPGGSLRLSCAASGFTFSSYSMNWV
RQAPGKCLEWVSSISSSSSYIYYADSVKGRFTISRDNAKNSLYLQMNSLRAEDTAVY
YCARGAPMGAAAGWFDPWGQGTLVTVSS (SEQ ID NO:203)

Anti-EpCAM HC-hinge-Fc (SEQ ID NO:204) includes a EpCAM-targeting heavy chain variable domain and a CH1 domain connected to an Fc domain (hinge-CH2-CH3). The Fc domain in SEQ ID NO:204 includes a Y349C substitution, which forms a disulfide bond with an S354C substitution in the CH3 domain of the Fc linked to the NKG2D-binding scFv (SEQ ID NO:203). In SEQ ID NO:204, the Fc domain also includes K360E and K409W substitutions.

### Anti-EpCAM V_{H}-CH1-Fc

EVQLLEQSGAELVRPGTSVKISCKASGYAFTNYWLGWVKQRPGHGLEWIGDIFPGSG
NIHYNEKFKGKATLTADKSSSTAYMQLSSLTFEDSAVYFCARLRNWDEPMDYWGQ
GTTVTVSSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKVEPKSCDKT
HTCPPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIEKTI
SKAKGQPREPQVCTLPPSRDELTENQVSLTCLVKGFYPSDIAVEWESNGQPENNYKT
TPPVLDSDGSFFLYSWLTVDKSRWQQGNVFSCSVMHEALHNHYTQKSLSLSPG
(SEQ ID NO:204)

Anti-EpCAM V_{L}- Constant domain (SEQ ID NO:214) includes a EpCAM-targeting light chain portion comprising a light chain variable domain and a light chain constant domain.

### Anti-EpCAM V_{L}- Constant domain

ELVMTQSPSSLTVTAGEKVTMSCKSSQSLLNSGNQKNYLTWYQQKPGQPPKLLIYW
ASTRESGVPDRFTGSGSGTDFTLTISSVQAEDLAVYYCQNDYSYPLTFGAGTKLEIKG
RTVAAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC (SEQ ID
NO:214)

In an exemplary embodiment, the Fc domain linked to the NKG2D-binding scFv fragment comprises the mutations of K360E and K409W, and the Fc domain linked to the EPCAM Fab fragment comprises matching mutations Q347R, D399V, and F405T for forming a heterodimer.

In an exemplary embodiment, the Fc domain linked to the NKG2D-binding scFv includes a Y349C substitution in the CH3 domain, which forms a disulfide bond with a S354C substitution on the Fc domain that is not linked to an NKG2D-binding scFv.

Another TriNKET of the present disclosure is NKG2D-binding-F3'-TriNKET-EPCAM, sequences of which are described below (CDRs (Kabat numbering) are underlined).

Some TriNKETs of the present disclosure are in the form A49-F3'-TriNKET-EPCAM, sequences of which are provided below (CDRs (Kabat numbering) are underlined).

An A49-F3'-TriNKET-EPCAM includes a single-chain variable fragment (scFv) that binds EPCAM (SEQ ID NOs:208 and 209 are exemplary sequences of such EPCAM-binding scFv polypeptides), linked to an Fc domain via a hinge comprising Gly-Ala-Ser (for example, in SEQ ID NO:210 and SEQ ID NO:211); and an NKG2D-binding Fab fragment ("A49") including a heavy chain portion comprising an heavy chain variable domain (SEQ ID NO:85) and a CH1 domain, and a light chain portion comprising a light chain variable domain (SEQ ID NO:86) and a light chain constant domain, wherein the heavy chain variable domain is connected to the CH1 domain, and the CH1 domain is connected to the Fc domain. The Fc domain linked to the EpCAM-targeting Fab comprises Q347R, D399V, and F405T substitutions for forming a heterodimer with an Fab comprising K360E and K409W substitutions (*see, e.g.,* SEQ ID NO:212 described below).

An EPCAM-binding scFv of the present disclosure can include a heavy chain variable domain connected to a light chain variable domain with a (G4S)₄ linker (represented as V_{L}(G4S)₄V_{H} or LH where V_{L} is N-terminal to V_{H}, and represented as V_{H}(G4S)₄V_{L} or HL where V_{H} is N-terminal to V_{L}). SEQ ID NOs:208 and 209 are exemplary sequences of such EPCAM-binding scFv polypeptides. The V_{L} and V_{H} comprised within the scFv (SEQ ID NOs:208 or 209) contain 100V_{L} - 44V_{H} S-S bridge (resulting from G100C and G44C substitutions, respectively) (cysteine residues are in bold-italics-underlined in the sequences below). (G4S)₄ is the bolded-underlined sequence GGGGSGGGGSGGGGSGGGGS (SEQ ID NO:206) in SEQ ID NO:208 and SEQ ID NO:209.

### EPCAM (MT110LH) scFv

ELVMTQSPSSLTVTAGEKVTMSCKSSQSLLNSGNQKNYLTWYQQKPGQPPKLLIYW
ASTRESGVPDRFTGSGSGTDFTLTISSVQAEDLAVYYCQNDYSYPLTFGCGTKLEIK
GGGGSGGGGSGGGGSGGGGSEVQLLEQSGAELVRPGTSVKISCKASGYAFTNYW
LGWVKQRPGHCLEWIGDIFPGSGNIHYNEKFKGKATLTADKSSSTAYMQLSSLTFED
SAVYFCARLRNWDEPMDYWGQGTTVTVSS (SEQ ID NO:208)

### EPCAM (MT100HL) scFv

EVQLLEQSGAELVRPGTSVKISCKASGYAFTNYWLGWVKQRPGHCLEWIGDIFPGSG
NIHYNEKFKGKATLTADKSSSTAYMQLSSLTFEDSAVYFCARLRNWDEPMDYWGQ
GTTVTVSSGGGGSGGGGSGGGGSGGGGSELVMTQSPSSLTVTAGEKVTMSCKSSQ
SLLNSGNQKNYLTWYQQKPGQPPKLLIYWASTRESGVPDRFTGSGSGTDFTLTISSV
QAEDLAVYYCQNDYSYPLTFGCGTKLEIK (SEQ ID NO:209)

SEQ ID NO:210 and SEQ ID NO:211 represent two sequences of an EPCAM-binding scFv, which can be linked to an Fc domain via a hinge comprising Gly-Ala-Ser (bold-underlined). The Fc domain linked to the scFv includes Q347R, D399V, and F405T substitutions.

### EPCAM (MT110LH) scFv-Fc

ELVMTQSPSSLTVTAGEKVTMSCKSSQSLLNSGNQKNYLTWYQQKPGQPPKLLIYW
ASTRESGVPDRFTGSGSGTDFTLTISSVQAEDLAVYYCQNDYSYPLTFGCGTKLEIK
GGGGSGGGGSGGGGSGGGGSEVQLLEQSGAELVRPGTSVKISCKASGYAFTNYW
LGWVKQRPGHCLEWIGDIFPGSGNIHYNEKFKGKATLTADKSSSTAYMQLSSLTFED
SAVYFCARLRNWDEPMDYWGQGTTVTVSSGASDKTHTCPPCPAPELLGGPSVFLFP
PKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPRVYTLPPCRDE
LTKNQVSLTCLVKGFYPSDIAVEWESNGQPENNYKTTPPVLVSDGSFTLYSKLTVDK
SRWQQGNVFSCSVMHEALHNHYTQKSLSLSPG (SEQ ID NO:210)

### EPCAM (MT110HL) scFv-Fc

EVQLLEQSGAELVRPGTSVKISCKASGYAFTNYWLGWVKQRPGHCLEWIGDIFPGSG
NIHYNEKFKGKATLTADKSSSTAYMQLSSLTFEDSAVYFCARLRNWDEPMDYWGQ
GTTVTVSSGGGGSGGGGSGGGGSGGGGSELVMTQSPSSLTVTAGEKVTMSCKSSQ
SLLNSGNQKNYLTWYQQKPGQPPKLLIYWASTRESGVPDRFTGSGSGTDFTLTISSV
QAEDLAVYYCQNDYSYPLTFGCGTKLEIKGASDKTHTCPPCPAPELLGGPSVFLFPPK
PKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPRVYTLPPCRDELT
KNQVSLTCLVKGFYPSDIAVEWESNGQPENNYKTTPPVLVSDGSFTLYSKLTVDKSR
WQQGNVFSCSVMHEALHNHYTQKSLSLSPG (SEQ ID NO:211)

SEQ ID NO:212 represents the heavy chain portion of a Fab fragment, which comprises an heavy chain variable domain (SEQ ID NO:85) of an NKG2D-binding site and a CH1 domain, connected to an Fc domain. The Fc domain in SEQ ID NO:212 includes a Y349C substitution in the CH3 domain, which forms a disulfide bond with a S354C substitution on the Fc linked to the EpCAM-binding scFv *(e.g.,* SEQ ID NO:210 and SEQ ID NO:211). In SEQ ID NO:212, the Fc domain also includes K360E and K409W substitutions.

### A49 - V_{H}

EVQLVESGGGLVKPGGSLRLSCAASGFTFSSYSMNWVRQAPGKGLEWVSSISSSSSYI
YYADSVKGRFTISRDNAKNSLYLQMNSLRAEDTAVYYCARGAPMGAAAGWFDPW
GQGTLVTVSS (SEQ ID NO:85)

### A49 V_{H}-CH1-Fc

EVQLVESGGGLVKPGGSLRLSCAASGFTFSSYSMNWVRQAPGKGLEWVSSISSSSSYI
YYADSVKGRFTISRDNAKNSLYLQMNSLRAEDTAVYYCARGAPMGAAAGWFDPW
GQGTLVTVSSASTKGPSVFPLAPSSKSTSGGTAALGCLVKDYFPEPVTVSWNSGALT
SGVHTFPAVLQSSGLYSLSSVVTVPSSSLGTQTYICNVNHKPSNTKVDKKVEPKSCD
KTHTCPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEK
TISKAKGQPREPQVCTLPPSRDELTENQVSLTCLVKGFYPSDIAVEWESNGQPENNY
KTTPPVLDSDGSFFLYSWLTVDKSRWQQGNVFSCSVMHEALHNHYTQKSLSLSPG
(SEQ ID NO:212)

SEQ ID NO:213 represents the light chain portion of a Fab fragment comprising a light chain variable domain (SEQ ID NO:86) of an NKG2D-binding site and a light chain constant domain.

### A49 - V_{L}

DIQMTQSPSSVSASVGDRVTITCRASQGISSWLAWYQQKPGKAPKLLIYAASSLQSG
VPSRFSGSGSGTDFTLTISSLQPEDFATYYCQQGVSFPRTFGGGTKVEIK (SEQ ID
NO:86)

### A49 LC V_{L} - Constant domain

DIQMTQSPSSVSASVGDRVTITCRASQGISSWLAWYQQKPGKAPKLLIYAASSLQSG
VPSRFSGSGSGTDFTLTISSLQPEDFATYYCQQGVSFPRTFGGGTKVEIK
RTVAAPSPPSDEQLKSGTASVVCLLNNFYPREAKVQWKVDNALQSGNSQESVTEQD
SKDSTYSLSSTLTLSKADYEKHKVYACEVTHQGLSSPVTKSFNRGEC (SEQ ID
NO:213)

In an exemplary embodiment, the Fc domain linked to the NKG2D-binding Fab fragment includes the mutations of Q347R, D399V, and F405T, and the Fc domain linked to the EPCAM scFv comprises matching mutations K360E and K409W for forming a heterodimer. In an exemplary embodiment, the Fc domain linked to the NKG2D-binding Fab fragment includes a S354C substitution in the CH3 domain, which forms a disulfide bond with a Y349C substitution on the Fc linked to the EPCAM-binding scFv.

Within the Fc domain, CD16 binding is mediated by the hinge region and the CH2 domain. For example, within human IgG1, the interaction with CD16 is primarily focused on amino acid residues Asp 265 - Glu 269, Asn 297 - Thr 299, Ala 327 - Ile 332, Leu 234 - Ser 239, and carbohydrate residue N-acetyl-D-glucosamine in the CH2 domain (see, Sondermann et al., Nature, 406 (6793):267-273). Based on the known domains, mutations can be selected to enhance or reduce the binding affinity to CD 16, such as by using phage-displayed libraries or yeast surface-displayed cDNA libraries, or can be designed based on the known three-dimensional structure of the interaction.

The assembly of heterodimeric antibody heavy chains can be accomplished by expressing two different antibody heavy chain sequences in the same cell, which may lead to the assembly of homodimers of each antibody heavy chain as well as assembly of heterodimers. Promoting the preferential assembly of heterodimers can be accomplished by incorporating different mutations in the CH3 domain of each antibody heavy chain constant region as shown in US13/494870, US16/028850, US11/533709, US12/875015, US13/289934, US14/773418, US12/811207, US13/866756, US14/647480, and US14/830336. For example, mutations can be made in the CH3 domain based on human IgG1 and incorporating distinct pairs of amino acid substitutions within a first polypeptide and a second polypeptide that allow these two chains to selectively heterodimerize with each other. The positions of amino acid substitutions illustrated below are all numbered according to the EU index as in Kabat.

In one scenario, an amino acid substitution in the first polypeptide replaces the original amino acid with a larger amino acid, selected from arginine (R), phenylalanine (F), tyrosine (Y) or tryptophan (W), and at least one amino acid substitution in the second polypeptide replaces the original amino acid(s) with a smaller amino acid(s), chosen from alanine (A), serine (S), threonine (T), or valine (V), such that the larger amino acid substitution (a protuberance) fits into the surface of the smaller amino acid substitutions (a cavity). For example, one polypeptide can incorporate a T366W substitution, and the other can incorporate three substitutions including T366S, L368A, and Y407V.

An antibody heavy chain variable domain of the invention can optionally be coupled to an amino acid sequence at least 90% identical to an antibody constant region, such as an IgG constant region including hinge, CH2 and CH3 domains with or without CH1 domain. In some embodiments, the amino acid sequence of the constant region is at least 90% identical to a human antibody constant region, such as an human IgG1 constant region, an IgG2 constant region, IgG3 constant region, or IgG4 constant region. In some other embodiments, the amino acid sequence of the constant region is at least 90% identical to an antibody constant region from another mammal, such as rabbit, dog, cat, mouse, or horse. One or more mutations can be incorporated into the constant region as compared to human IgG1 constant region, for example at Q347, Y349, L351, S354, E356, E357, K360, Q362, S364, T366, L368, K370, N390, K392, T394, D399, S400, D401, F405, Y407, K409, T411 and/or K439. Exemplary substitutions include, for example, Q347E, Q347R, Y349S, Y349K, Y349T, Y349D, Y349E, Y349C, T350V, L351K, L351D, L351Y, S354C, E356K, E357Q, E357L, E357W, K360E, K360W, Q362E, S364K, S364E, S364H, S364D, T366V, T366I, T366L, T366M, T366K, T366W, T366S, L368E, L368A, L368D, K370S, N390D, N390E, K392L, K392M, K392V, K392F, K392D, K392E, T394F, T394W, D399R, D399K, D399V, S400K, S400R, D401K, F405A, F405T, Y407A, Y407I, Y407V, K409F, K409W, K409D, T411D, T411E, K439D, and K439E.

In certain embodiments, mutations that can be incorporated into the CH1 of a human IgG1 constant region may be at amino acid V125, F126, P127, T135, T139, A140, F170, P171, and/or V173. In certain embodiments, mutations that can be incorporated into the Cκ of a human IgG1 constant region may be at amino acid E123, F116, S176, V163, S174, and/or T164.

Alternatively, amino acid substitutions could be selected from the following sets of substitutions shown in Table 4.

| Table 4 | | |
|---|---|---|
| | First Polypeptide | Second Polypeptide |
| Set 1 | S364E/F405A | Y349K/T394F |
| Set 2 | S364H/D401K | Y349T/T411E |
| Set 3 | S364H/T394F | Y349T/F405A |
| Set 4 | S364E/T394F | Y349K/F405A |
| Set 5 | S364E/T411E | Y349K/D401K |
| Set 6 | S364D/T394F | Y349K/F405A |
| Set 7 | S364H/F405A | Y349T/T394F |
| Set 8 | S364K/E357Q | L368D/K370S |
| Set 9 | L368D/K370S | S364K |
| Set 10 | L368E/K370S | S364K |
| Set 11 | K360E/Q362E | D401K |
| Set 12 | L368D/K370S | S364K/E357L |
| Set 13 | K370S | S364K/E357Q |
| Set 14 | F405L | K409R |
| Set 15 | K409R | F405L |

Alternatively, amino acid substitutions could be selected from the following sets of substitutions shown in Table 5.

| Table 5 | | |
|---|---|---|
| | First Polypeptide | Second Polypeptide |
| Set 1 | K409W | D399V/F405T |
| Set 2 | Y349S | E357W |
| Set 3 | K360E | Q347R |
| Set 4 | K360E/K409W | Q347R/D399V/F405T |
| Set 5 | Q347E/K360E/K409W | Q347R/D399V/F405T |
| Set 6 | Y349S/K409W | E357W/D399V/F405T |

Alternatively, amino acid substitutions could be selected from the following set of substitutions shown in Table 6.

| Table 6 | | |
|---|---|---|
| | First Polypeptide | Second Polypeptide |
| Set 1 | T366K/L351K | L351D/L368E |
| Set 2 | T366K/L351K | L351D/Y349E |
| Set 3 | T366K/L351K | L351D/Y349D |
| Set 4 | T366K/L351K | L351D/Y349E/L368E |
| Set 5 | T366K/L351K | L351D/Y349D/L368E |
| Set 6 | E356K/D399K | K392D/K409D |

Alternatively, at least one amino acid substitution in each polypeptide chain could be selected from Table 7.

| Table 7 | |
|---|---|
| First Polypeptide | Second Polypeptide |
| L351Y, D399R, D399K, S400K, S400R, Y407A, Y407I, Y407V | T366V, T366I, T366L, T366M, N390D, N390E, K392L, K392M, K392V, K392F K392D, K392E, K409F, K409W, T411D and T411E |

Alternatively, at least one amino acid substitutions could be selected from the following set of substitutions in Table 8, where the position(s) indicated in the First Polypeptide column is replaced by any known negatively-charged amino acid, and the position(s) indicated in the Second Polypeptide Column is replaced by any known positively-charged amino acid.

| Table 8 | |
|---|---|
| First Polypeptide | Second Polypeptide |
| K392, K370, K409, or K439 | D399, E356, or E357 |

Alternatively, at least one amino acid substitutions could be selected from the following set of in Table 9, where the position(s) indicated in the First Polypeptide column is replaced by any known positively-charged amino acid, and the position(s) indicated in the Second Polypeptide Column is replaced by any known negatively-charged amino acid.

| Table 9 | |
|---|---|
| First Polypeptide | Second Polypeptide |
| D399, E356, or E357 | K409, K439, K370, or K392 |

Alternatively, amino acid substitutions could be selected from the following set in Table 10.

| Table 10 | |
|---|---|
| First Polypeptide | Second Polypeptide |
| T350V, L351Y, F405A, and Y407V | T350V, T366L, K392L, and T394W |

Alternatively, or in addition, the structural stability of a hetero-multimeric protein may be increased by introducing S354C on either of the first or second polypeptide chain, and Y349C on the opposing polypeptide chain, which forms an artificial disulfide bridge within the interface of the two polypeptides.

In some embodiments, the amino acid sequence of one polypeptide chain of the antibody constant region differs from the amino acid sequence of an IgG1 constant region at position T366, and wherein the amino acid sequence of the other polypeptide chain of the antibody constant region differs from the amino acid sequence of an IgG1 constant region at one or more positions selected from the group consisting of T366, L368 and Y407.

In some embodiments, the amino acid sequence of one polypeptide chain of the antibody constant region differs from the amino acid sequence of an IgG1 constant region at one or more positions selected from the group consisting of T366, L368 and Y407, and wherein the amino acid sequence of the other polypeptide chain of the antibody constant region differs from the amino acid sequence of an IgG1 constant region at position T366.

In some embodiments, the amino acid sequence of one polypeptide chain of the antibody constant region differs from the amino acid sequence of an IgG1 constant region at one or more positions selected from the group consisting of E357, K360, Q362, S364, L368, K370, T394, D401, F405, and T411 and wherein the amino acid sequence of the other polypeptide chain of the antibody constant region differs from the amino acid sequence of an IgG1 constant region at one or more positions selected from the group consisting of Y349, E357, S364, L368, K370, T394, D401, F405 and T411.

In some embodiments, the amino acid sequence of one polypeptide chain of the antibody constant region differs from the amino acid sequence of an IgG1 constant region at one or more positions selected from the group consisting of Y349, E357, S364, L368, K370, T394, D401, F405 and T411 and wherein the amino acid sequence of the other polypeptide chain of the antibody constant region differs from the amino acid sequence of an IgG1 constant region at one or more positions selected from the group consisting of E357, K360, Q362, S364, L368, K370, T394, D401, F405, and T411.

In some embodiments, the amino acid sequence of one polypeptide chain of the antibody constant region differs from the amino acid sequence of an IgG1 constant region at one or more positions selected from the group consisting of L351, D399, S400 and Y407 and wherein the amino acid sequence of the other polypeptide chain of the antibody constant region differs from the amino acid sequence of an IgG1 constant region at one or more positions selected from the group consisting of T366, N390, K392, K409 and T411.

In some embodiments, the amino acid sequence of one polypeptide chain of the antibody constant region differs from the amino acid sequence of an IgG1 constant region at one or more positions selected from the group consisting of T366, N390, K392, K409 and T411 and wherein the amino acid sequence of the other polypeptide chain of the antibody constant region differs from the amino acid sequence of an IgG1 constant region at one or more positions selected from the group consisting of L351, D399, S400 and Y407.

In some embodiments, the amino acid sequence of one polypeptide chain of the antibody constant region differs from the amino acid sequence of an IgG1 constant region at one or more positions selected from the group consisting of Q347, Y349, K360, and K409, and wherein the amino acid sequence of the other polypeptide chain of the antibody constant region differs from the amino acid sequence of an IgG1 constant region at one or more positions selected from the group consisting of Q347, E357, D399 and F405.

In some embodiments, the amino acid sequence of one polypeptide chain of the antibody constant region differs from the amino acid sequence of an IgG1 constant region at one or more positions selected from the group consisting of Q347, E357, D399 and F405, and wherein the amino acid sequence of the other polypeptide chain of the antibody constant region differs from the amino acid sequence of an IgG1 constant region at one or more positions selected from the group consisting of Y349, K360, Q347 and K409.

In some embodiments, the amino acid sequence of one polypeptide chain of the antibody constant region differs from the amino acid sequence of an IgG1 constant region at one or more positions selected from the group consisting of K370, K392, K409 and K439, and wherein the amino acid sequence of the other polypeptide chain of the antibody constant region differs from the amino acid sequence of an IgG1 constant region at one or more positions selected from the group consisting of D356, E357 and D399.

In some embodiments, the amino acid sequence of one polypeptide chain of the antibody constant region differs from the amino acid sequence of an IgG1 constant region at one or more positions selected from the group consisting of D356, E357 and D399, and wherein the amino acid sequence of the other polypeptide chain of the antibody constant region differs from the amino acid sequence of an IgG1 constant region at one or more positions selected from the group consisting of K370, K392, K409 and K439.

In some embodiments, the amino acid sequence of one polypeptide chain of the antibody constant region differs from the amino acid sequence of an IgG1 constant region at one or more positions selected from the group consisting of L351, E356, T366 and D399, and wherein the amino acid sequence of the other polypeptide chain of the antibody constant region differs from the amino acid sequence of an IgG1 constant region at one or more positions selected from the group consisting of Y349, L351, L368, K392 and K409.

In some embodiments, the amino acid sequence of one polypeptide chain of the antibody constant region differs from the amino acid sequence of an IgG1 constant region at one or more positions selected from the group consisting of Y349, L351, L368, K392 and K409, and wherein the amino acid sequence of the other polypeptide chain of the antibody constant region differs from the amino acid sequence of an IgG1 constant region at one or more positions selected from the group consisting of L351, E356, T366 and D399.

In some embodiments, the amino acid sequence of one polypeptide chain of the antibody constant region differs from the amino acid sequence of an IgG1 constant region by an S354C substitution and wherein the amino acid sequence of the other polypeptide chain of the antibody constant region differs from the amino acid sequence of an IgG1 constant region by a Y349C substitution.

In some embodiments, the amino acid sequence of one polypeptide chain of the antibody constant region differs from the amino acid sequence of an IgG1 constant region by a Y349C substitution and wherein the amino acid sequence of the other polypeptide chain of the antibody constant region differs from the amino acid sequence of an IgG1 constant region by an S354C substitution.

In some embodiments, the amino acid sequence of one polypeptide chain of the antibody constant region differs from the amino acid sequence of an IgG1 constant region by K360E and K409W substitutions and wherein the amino acid sequence of the other polypeptide chain of the antibody constant region differs from the amino acid sequence of an IgG1 constant region by O347R, D399V and F405T substitutions.

In some embodiments, the amino acid sequence of one polypeptide chain of the antibody constant region differs from the amino acid sequence of an IgG1 constant region by O347R, D399V and F405T substitutions and wherein the amino acid sequence of the other polypeptide chain of the antibody constant region differs from the amino acid sequence of an IgG1 constant region by K360E and K409W substitutions.

In some embodiments, the amino acid sequence of one polypeptide chain of the antibody constant region differs from the amino acid sequence of an IgG1 constant region by a T366W substitutions and wherein the amino acid sequence of the other polypeptide chain of the antibody constant region differs from the amino acid sequence of an IgG1 constant region by T366S, T368A, and Y407V substitutions.

In some embodiments, the amino acid sequence of one polypeptide chain of the antibody constant region differs from the amino acid sequence of an IgG1 constant region by T366S, T368A, and Y407V substitutions and wherein the amino acid sequence of the other polypeptide chain of the antibody constant region differs from the amino acid sequence of an IgG1 constant region by a T366W substitution.

In some embodiments, the amino acid sequence of one polypeptide chain of the antibody constant region differs from the amino acid sequence of an IgG1 constant region by T350V, L351Y, F405A, and Y407V substitutions and wherein the amino acid sequence of the other polypeptide chain of the antibody constant region differs from the amino acid sequence of an IgG1 constant region by T350V, T366L, K392L, and T394W substitutions.

In some embodiments, the amino acid sequence of one polypeptide chain of the antibody constant region differs from the amino acid sequence of an IgG1 constant region by T350V, T366L, K392L, and T394W substitutions and wherein the amino acid sequence of the other polypeptide chain of the antibody constant region differs from the amino acid sequence of an IgG1 constant region by T350V, L351Y, F405A, and Y407V substitutions.

The multi-specific proteins described above can be made using recombinant DNA technology well known to a skilled person in the art. For example, a first nucleic acid sequence encoding the first immunoglobulin heavy chain can be cloned into a first expression vector; a second nucleic acid sequence encoding the second immunoglobulin heavy chain can be cloned into a second expression vector; a third nucleic acid sequence encoding the immunoglobulin light chain can be cloned into a third expression vector; and the first, second, and third expression vectors can be stably transfected together into host cells to produce the multimeric proteins.

To achieve the highest yield of the multi-specific protein, different ratios of the first, second, and third expression vector can be explored to determine the optimal ratio for transfection into the host cells. After transfection, single clones can be isolated for cell bank generation using methods known in the art, such as limited dilution, ELISA, FACS, microscopy, or Clonepix.

Clones can be cultured under conditions suitable for bio-reactor scale-up and maintained expression of the multi-specific protein. The multispecific proteins can be isolated and purified using methods known in the art including centrifugation, depth filtration, cell lysis, homogenization, freeze-thawing, affinity purification, gel filtration, ion exchange chromatography, hydrophobic interaction exchange chromatography, and mixed-mode chromatography.

### II. CHARACTERISTICS OF THE MULTI-SPECIFIC PROTEINS

The multi-specific proteins described herein include an NKG2D-binding site, a CD16-binding site, and a tumor-associated antigen selected from any one of the antigens provided in Table 11. In some embodiments, the multi-specific proteins bind simultaneously to cells expressing NKG2D and/or CD16, such as NK cells, and to tumor cells expressing a tumor-associated antigen selected from any one of the antigens provided in Table 11. Binding of the multi-specific proteins to NK cells can enhance the activity of the NK cells toward destruction of the tumor cells.

**Table 11**

| **Type of Antigen** | **Biological Name** |
|---|---|
| Transmembrane glycoprotein mediating Ca²⁺-independent homotypic cell-cell adhesion in epithelia | Epithelial cell adhesion molecule (EpCAM) |
| Mucin family glycoproteins | Cancer Antigen 125 (CA125) |
| Phosphate transport protein involved in transporting phosphate into cells via Na+ co-transport | sodium/phosphate cotransporter 2B (NaPi2b) |
| Cellular adhesion molecules involved in Ca²⁺-independent cellular adhesion | Nectin cell adhesion molecule 4 (Nectin4) |
| Gangliosides | Fucosyl-GM1 (monosialotetrahexosylganglioside) |
| ADAM (a disintegrin and metalloproteinase) protein | disintegrin and metalloproteinase domain-containing protein 8 (ADAM8) |
| ADAM (a disintegrin and metalloproteinase) protein | disintegrin and metalloproteinase domain-containing protein 9 (ADAM9) |
| Solute carrier proteins known as choline transporter-like proteins (CTL1-5) | solute carrier family 44 member 4 (SLC44A4) |
| Carbohydrate antigen sialyl Lewis a | sialylated Lewis a antigen (CA19-9) |

In some embodiments, the multi-specific proteins bind to a tumor-associated antigen selected from any one of the antigens provided in Table 11 with a similar affinity to the corresponding monoclonal antibody *(i.e.,* a monoclonal antibody containing the same a tumor-associated antigen-binding site as the one incorporated in the multi-specific proteins (selected from any one of the antigens provided in Table 11)). In some embodiments, the multi-specific proteins are more effective in killing the tumor cells expressing a tumor-associated antigen selected from any one of the antigens provided in Table 11 than the corresponding monoclonal antibodies.

In certain embodiments, the multi-specific proteins described herein, which include an NKG2D-binding site and a binding site for a tumor-associated antigen selected from any one of the antigens provided in Table 11, activate primary human NK cells when co-culturing with cells expressing the tumor-associated antigen. NK cell activation is marked by the increase in CD107a degranulation and IFN-γ cytokine production. Furthermore, compared to a corresponding monoclonal antibody for a tumor-associated antigen selected from any one of the antigens provided in Table 11, the multi-specific proteins may show superior activation of human NK cells in the presence of cells expressing the tumor-associated antigen.

In certain embodiments, the multi-specific proteins described herein, which include an NKG2D-binding site and a binding site for a tumor-associated antigen selected from any one of the antigens provided in Table 11, enhance the activity of rested and IL-2-activated human NK cells co-culturing with cells expressing the tumor-associated antigen.

In certain embodiments, compared to a corresponding monoclonal antibody that binds to a tumor-associated antigen selected from any one of the antigens provided in Table 11, the multi-specific proteins offer an advantage in targeting tumor cells that express the tumor-associated antigen. The multi-specific binding proteins described herein may be more effective in reducing tumor growth and killing cancer cells.

In certain embodiments, EpCAM-targeting F4-TriNKET *(e.g.,* NKG2D-binding-F4-TriNKET-EpCAM) killed target cells more effectively than the parental mAb targeting EpCAM. In certain embodiments, the F4-TriNKET also killed target cells more potently than F3'-TriNKET *(e.g.,* NKG2D-binding-F3'-TriNKET-EpCAM), which may be a reflection of the stronger binding of F4-TriNKET to target cells.

### III. THERAPEUTIC APPLICATIONS

The invention provides methods for treating cancer using a multi-specific binding protein described herein and/or a pharmaceutical composition described herein. The methods may be used to treat a variety of cancers which express EPCAM by administering to a patient in need thereof a therapeutically effective amount of a multi-specific binding protein described herein.

The therapeutic method can be characterized according to the cancer to be treated. For example, in certain embodiments, the cancer is acute myeloid leukemia, multiple myeloma, diffuse large B cell lymphoma, thymoma, adenoid cystic carcinoma, gastrointestinal cancer, renal cancer, breast cancer, glioblastoma, lung cancer, ovarian cancer, brain cancer, prostate cancer, pancreatic cancer, or melanoma.

In certain other embodiments, the cancer is a solid tumor. In certain other embodiments, the cancer is colon cancer, bladder cancer, cervical cancer, endometrial cancer, esophageal cancer, leukemia, liver cancer, rectal cancer, stomach cancer, testicular cancer, or uterine cancer. In yet other embodiments, the cancer is a vascularized tumor, squamous cell carcinoma, adenocarcinoma, small cell carcinoma, melanoma, glioma, neuroblastoma, sarcoma *(e.g.,* an angiosarcoma or chondrosarcoma), larynx cancer, parotid cancer, bilary tract cancer, thyroid cancer, acral lentiginous melanoma, actinic keratoses, acute lymphocytic leukemia, acute myeloid leukemia, adenoid cycstic carcinoma, adenomas, adenosarcoma, adenosquamous carcinoma, anal canal cancer, anal cancer, anorectum cancer, astrocytic tumor, bartholin gland carcinoma, basal cell carcinoma, biliary cancer, bone cancer, bone marrow cancer, bronchial cancer, bronchial gland carcinoma, carcinoid, cholangiocarcinoma, chondosarcoma, choriod plexus papilloma/carcinoma, chronic lymphocytic leukemia, chronic myeloid leukemia, clear cell carcinoma, connective tissue cancer, cystadenoma, digestive system cancer, duodenum cancer, endocrine system cancer, endodermal sinus tumor, endometrial hyperplasia, endometrial stromal sarcoma, endometrioid adenocarcinoma, endothelial cell cancer, ependymal cancer, epithelial cell cancer, Ewing's sarcoma, eye and orbit cancer, female genital cancer, focal nodular hyperplasia, gallbladder cancer, gastric antrum cancer, gastric fundus cancer, gastrinoma, glioblastoma, glucagonoma, heart cancer, hemangiblastomas, hemangioendothelioma, hemangiomas, hepatic adenoma, hepatic adenomatosis, hepatobiliary cancer, hepatocellular carcinoma, Hodgkin's disease, ileum cancer, insulinoma, intaepithelial neoplasia, interepithelial squamous cell neoplasia, intrahepatic bile duct cancer, invasive squamous cell carcinoma, jejunum cancer, joint cancer, Kaposi's sarcoma, pelvic cancer, large cell carcinoma, large intestine cancer, leiomyosarcoma, lentigo maligna melanomas, lymphoma, male genital cancer, malignant melanoma, malignant mesothelial tumors, medulloblastoma, medulloepithelioma, meningeal cancer, mesothelial cancer, metastatic carcinoma, mouth cancer, mucoepidermoid carcinoma, multiple myeloma, muscle cancer, nasal tract cancer, nervous system cancer, neuroepithelial adenocarcinoma nodular melanoma, non-epithelial skin cancer, non-Hodgkin's lymphoma, oat cell carcinoma, oligodendroglial cancer, oral cavity cancer, osteosarcoma, papillary serous adenocarcinoma, penile cancer, pharynx cancer, pituitary tumors, plasmacytoma, pseudosarcoma, pulmonary blastoma, rectal cancer, renal cell carcinoma, respiratory system cancer, retinoblastoma, rhabdomyosarcoma, sarcoma, serous carcinoma, sinus cancer, skin cancer, small cell carcinoma, small intestine cancer, smooth muscle cancer, soft tissue cancer, somatostatin-secreting tumor, spine cancer, squamous cell carcinoma, striated muscle cancer, submesothelial cancer, superficial spreading melanoma, T cell leukemia, tongue cancer, undifferentiated carcinoma, ureter cancer, urethra cancer, urinary bladder cancer, urinary system cancer, uterine cervix cancer, uterine corpus cancer, uveal melanoma, vaginal cancer, verrucous carcinoma, VIPoma, vulva cancer, well differentiated carcinoma, or Wilms tumor.

In certain other embodiments, the cancer is non-Hodgkin's lymphoma, such as a B-cell lymphoma or a T-cell lymphoma. In certain embodiments, the non-Hodgkin's lymphoma is a B-cell lymphoma, such as a diffuse large B-cell lymphoma, primary mediastinal B-cell lymphoma, follicular lymphoma, small lymphocytic lymphoma, mantle cell lymphoma, marginal zone B-cell lymphoma, extranodal marginal zone B-cell lymphoma, nodal marginal zone B-cell lymphoma, splenic marginal zone B-cell lymphoma, Burkitt lymphoma, lymphoplasmacytic lymphoma, hairy cell leukemia, or primary central nervous system (CNS) lymphoma. In certain other embodiments, the non-Hodgkin's lymphoma is a T-cell lymphoma, such as a precursor T-lymphoblastic lymphoma, peripheral T-cell lymphoma, cutaneous T-cell lymphoma, angioimmunoblastic T-cell lymphoma, extranodal natural killer/T-cell lymphoma, enteropathy type T-cell lymphoma, subcutaneous panniculitis-like T-cell lymphoma, anaplastic large cell lymphoma, or peripheral T-cell lymphoma.

The cancer to be treated can be characterized according to the presence of a particular antigen expressed on the surface of the cancer cell. In certain embodiments, the cancer cell can express one or more of the following in addition to EpCAM: CD2, CD19, CD20, CD30, CD38, CD40, CD52, CD70, EGFR/ERBB1, IGF1R, HER3/ERBB3, HER4/ERBB4, MUC1, TROP2, cMET, SLAMF7, PSCA, MICA, MICB, TRAILR1, TRAILR2, MAGE-A3, B7.1, B7.2, CTLA4, and PD1.

In some other embodiments, when the second binding site binds EpCAM, the cancer to be treated is selected from head and neck cancer, ovarian cancer, bladder cancer, breast cancer, colorectal cancer, prostate cancer, gastric cancer, liver cancer, esophageal cancer, and lung cancer. In some other embodiments, when the second binding site binds an antigen selected from Cancer Antigen 125 (CA125), sodium/phosphate cotransporter 2B (NaPi2b), Nectin cell adhesion molecule 4 (Nectin4), Fucosyl-GM1 (monosialotetrahexosylganglioside), disintegrin and metalloproteinase domain-containing protein 8 (ADAM8), disintegrin and metalloproteinase domain-containing protein 9 (ADAM9), solute carrier family 44 member 4 (SLC44A4), and sialylated Lewis a antigen (CA19-9), the cancer to be treated is selected from ovarian cancer, endometrial cancer, pancreatic cancer, lung cancer, thyroid cancer, bladder cancer, breast cancer, colorectal cancer, small cell lung cancer, neuroblastoma, liver cancer, renal cancer, melanoma, cervical cancer, prostate cancer, osteosarcoma, brain cancer, gastric cancer, cholangiocarcinoma.

### IV. COMBINATION THERAPY

Another aspect of the invention provides for combination therapy. A multi-specific binding protein described herein can be used in combination with additional therapeutic agents to treat the cancer.

Exemplary therapeutic agents that may be used as part of a combination therapy in treating cancer, include, for example, radiation, mitomycin, tretinoin, ribomustin, gemcitabine, vincristine, etoposide, cladribine, mitobronitol, methotrexate, doxorubicin, carboquone, pentostatin, nitracrine, zinostatin, cetrorelix, letrozole, raltitrexed, daunorubicin, fadrozole, fotemustine, thymalfasin, sobuzoxane, nedaplatin, cytarabine, bicalutamide, vinorelbine, vesnarinone, aminoglutethimide, amsacrine, proglumide, elliptinium acetate, ketanserin, doxifluridine, etretinate, isotretinoin, streptozocin, nimustine, vindesine, flutamide, drogenil, butocin, carmofur, razoxane, sizofilan, carboplatin, mitolactol, tegafur, ifosfamide, prednimustine, picibanil, levamisole, teniposide, improsulfan, enocitabine, lisuride, oxymetholone, tamoxifen, progesterone, mepitiostane, epitiostanol, formestane, interferon-alpha, interferon-2 alpha, interferon-beta, interferon-gamma (IFN-γ), colony stimulating factor-1, colony stimulating factor-2, denileukin diftitox, interleukin-2, luteinizing hormone releasing factor and variations of the aforementioned agents that may exhibit differential binding to its cognate receptor, and increased or decreased serum half-life.

An additional class of agents that may be used as part of a combination therapy in treating cancer is immune checkpoint inhibitors. Exemplary immune checkpoint inhibitors include agents that inhibit one or more of (i) cytotoxic T lymphocyte-associated antigen 4 (CTLA4), (ii) programmed cell death protein 1 (PD1), (iii) PDL1, (iv) LAG3, (v) B7-H3, (vi) B7-H4, and (vii) TIM3. The CTLA4 inhibitor ipilimumab has been approved by the United States Food and Drug Administration for treating melanoma.

Yet other agents that may be used as part of a combination therapy in treating cancer are monoclonal antibody agents that target non-checkpoint targets *(e.g.,* herceptin) and non-cytotoxic agents *(e.g.,* tyrosine-kinase inhibitors).

Yet other categories of anti-cancer agents include, for example: (i) an inhibitor selected from an ALK Inhibitor, an ATR Inhibitor, an A2A Antagonist, a Base Excision Repair Inhibitor, a Bcr-Abl Tyrosine Kinase Inhibitor, a Bruton's Tyrosine Kinase Inhibitor, a CDC7 Inhibitor, a CHK1 Inhibitor, a Cyclin-Dependent Kinase Inhibitor, a DNA-PK Inhibitor, an Inhibitor of both DNA-PK and mTOR, a DNMT1 Inhibitor, a DNMT1 Inhibitor plus 2-chloro-deoxyadenosine, an HDAC Inhibitor, a Hedgehog Signaling Pathway Inhibitor, an IDO Inhibitor, a JAK Inhibitor, a mTOR Inhibitor, a MEK Inhibitor, a MELK Inhibitor, a MTH1 Inhibitor, a PARP Inhibitor, a Phosphoinositide 3-Kinase Inhibitor, an Inhibitor of both PARP1 and DHODH, a Proteasome Inhibitor, a Topoisomerase-II Inhibitor, a Tyrosine Kinase Inhibitor, a VEGFR Inhibitor, and a WEE1 Inhibitor; (ii) an agonist of OX40, CD137, CD40, GITR, CD27, HVEM, TNFRSF25, or ICOS; and (iii) a cytokine selected from IL-12, IL-15, GM-CSF, and G-CSF.

Proteins of the invention can also be used as an adjunct to surgical removal of the primary lesion.

The amount of multi-specific binding protein and additional therapeutic agent and the relative timing of administration may be selected in order to achieve a desired combined therapeutic effect. For example, when administering a combination therapy to a patient in need of such administration, the therapeutic agents in the combination, or a pharmaceutical composition or compositions comprising the therapeutic agents, may be administered in any order such as, for example, sequentially, concurrently, together, simultaneously and the like. Further, for example, a multi-specific binding protein may be administered during a time when the additional therapeutic agent(s) exerts its prophylactic or therapeutic effect, or *vice versa.*

### V. PHARMACEUTICAL COMPOSITIONS

The present disclosure also features pharmaceutical compositions that contain a therapeutically effective amount of a protein described herein. The composition can be formulated for use in a variety of drug delivery systems. One or more physiologically acceptable excipients or carriers can also be included in the composition for proper formulation. Suitable formulations for use in the present disclosure are found in Remington's Pharmaceutical Sciences, Mack Publishing Company, Philadelphia, Pa., 17th ed., 1985. For a brief review of methods for drug delivery, *see, e.g.,* Langer (Science 249:1527-1533, 1990).

Pharmaceutical compositions can contain a therapeutically effective amount of a multi-specific binding protein comprising an antigen (listed in Table 11) site.

The intravenous drug delivery formulation of the present disclosure may be contained in a bag, a pen, or a syringe. In certain embodiments, the bag may be connected to a channel comprising a tube and/or a needle. In certain embodiments, the formulation may be a lyophilized formulation or a liquid formulation. In certain embodiments, the formulation may freeze-dried (lyophilized) and contained in about 12-60 vials. In certain embodiments, the formulation may be freeze-dried and 45 mg of the freeze-dried formulation may be contained in one vial. In certain embodiments, the about 40 mg - about 100 mg of freeze-dried formulation may be contained in one vial. In certain embodiments, freeze dried formulation from 12, 27, or 45 vials are combined to obtained a therapeutic dose of the protein in the intravenous drug formulation. In certain embodiments, the formulation may be a liquid formulation and stored as about 250 mg/vial to about 1000 mg/vial. In certain embodiments, the formulation may be a liquid formulation and stored as about 600 mg/vial. In certain embodiments, the formulation may be a liquid formulation and stored as about 250 mg/vial.

The protein could exist in a liquid aqueous pharmaceutical formulation including a therapeutically effective amount of the protein in a buffered solution forming a formulation.

These compositions may be sterilized by conventional sterilization techniques, or may be sterile filtered. The resulting aqueous solutions may be packaged for use as-is, or lyophilized, the lyophilized preparation being combined with a sterile aqueous carrier prior to administration. The pH of the preparations typically will be between 3 and 11, more preferably between 5 and 9 or between 6 and 8, and most preferably between 7 and 8, such as 7 to 7.5. The resulting compositions in solid form may be packaged in multiple single dose units, each containing a fixed amount of the above-mentioned agent or agents. The composition in solid form can also be packaged in a container for a flexible quantity.

In certain embodiments, the present disclosure provides a formulation with an extended shelf life including the protein of the present disclosure, in combination with mannitol, citric acid monohydrate, sodium citrate, disodium phosphate dihydrate, sodium dihydrogen phosphate dihydrate, sodium chloride, polysorbate 80, water, and sodium hydroxide.

In certain embodiments, an aqueous formulation is prepared including the protein of the present disclosure in a pH-buffered solution. The buffer of this invention may have a pH ranging from about 4 to about 8, *e.g.,* from about 4.5 to about 6.0, or from about 4.8 to about 5.5, or may have a pH of about 5.0 to about 5.2. Ranges intermediate to the above recited pH's are also intended to be part of this disclosure. For example, ranges of values using a combination of any of the above recited values as upper and/or lower limits are intended to be included. Examples of buffers that will control the pH within this range include acetate *(e.g.,* sodium acetate), succinate (such as sodium succinate), gluconate, histidine, citrate and other organic acid buffers.

In certain embodiments, the formulation includes a buffer system which contains citrate and phosphate to maintain the pH in a range of about 4 to about 8. In certain embodiments the pH range may be from about 4.5 to about 6.0, or from about pH 4.8 to about 5.5, or in a pH range of about 5.0 to about 5.2. In certain embodiments, the buffer system includes citric acid monohydrate, sodium citrate, disodium phosphate dihydrate, and/or sodium dihydrogen phosphate dihydrate. In certain embodiments, the buffer system includes about 1.3 mg/mL of citric acid *(e.g.,* 1.305 mg/mL), about 0.3 mg/mL of sodium citrate *(e.g.,* 0.305 mg/mL), about 1.5 mg/mL of disodium phosphate dihydrate *(e.g.,* 1.53 mg/mL), about 0.9 mg/mL of sodium dihydrogen phosphate dihydrate *(e.g.,* 0.86), and about 6.2 mg/mL of sodium chloride *(e.g.,* 6.165 mg/mL). In certain embodiments, the buffer system includes 1-1.5 mg/mL of citric acid, 0.25 to 0.5 mg/mL of sodium citrate, 1.25 to 1.75 mg/mL of disodium phosphate dihydrate, 0.7 to 1.1 mg/mL of sodium dihydrogen phosphate dihydrate, and 6.0 to 6.4 mg/mL of sodium chloride. In certain embodiments, the pH of the formulation is adjusted with sodium hydroxide.

A polyol, which acts as a tonicifier and may stabilize the antibody, may also be included in the formulation. The polyol is added to the formulation in an amount which may vary with respect to the desired isotonicity of the formulation. In certain embodiments, the aqueous formulation may be isotonic. The amount of polyol added may also be altered with respect to the molecular weight of the polyol. For example, a lower amount of a monosaccharide *(e.g.,* mannitol) may be added, compared to a disaccharide (such as trehalose). In certain embodiments, the polyol which may be used in the formulation as a tonicity agent is mannitol. In certain embodiments, the mannitol concentration may be about 5 to about 20 mg/mL. In certain embodiments, the concentration of mannitol may be about 7.5 to 15 mg/mL. In certain embodiments, the concentration of mannitol may be about 10-14 mg/mL. In certain embodiments, the concentration of mannitol may be about 12 mg/mL. In certain embodiments, the polyol sorbitol may be included in the formulation.

A detergent or surfactant may also be added to the formulation. Exemplary detergents include nonionic detergents such as polysorbates *(e.g.,* polysorbates 20, 80 etc.) or poloxamers *(e.g.,* poloxamer 188). The amount of detergent added is such that it reduces aggregation of the formulated antibody and/or minimizes the formation of particulates in the formulation and/or reduces adsorption. In certain embodiments, the formulation may include a surfactant which is a polysorbate. In certain embodiments, the formulation may contain the detergent polysorbate 80 or Tween 80. Tween 80 is a term used to describe polyoxyethylene (20) sorbitanmonooleate *(see* Fiedler, Lexikon der Hifsstoffe, Editio Cantor Verlag Aulendorf, 4th ed., 1996). In certain embodiments, the formulation may contain between about 0.1 mg/mL and about 10 mg/mL of polysorbate 80, or between about 0.5 mg/mL and about 5 mg/mL. In certain embodiments, about 0.1% polysorbate 80 may be added in the formulation.

In embodiments, the protein product of the present disclosure is formulated as a liquid formulation. The liquid formulation may be presented at a 10 mg/mL concentration in either a USP / Ph Eur type I 50R vial closed with a rubber stopper and sealed with an aluminum crimp seal closure. The stopper may be made of elastomer complying with USP and Ph Eur. In certain embodiments vials may be filled with 61.2 mL of the protein product solution in order to allow an extractable volume of 60 mL. In certain embodiments, the liquid formulation may be diluted with 0.9% saline solution.

In certain embodiments, the liquid formulation of the disclosure may be prepared as a 10 mg/mL concentration solution in combination with a sugar at stabilizing levels. In certain embodiments the liquid formulation may be prepared in an aqueous carrier. In certain embodiments, a stabilizer may be added in an amount no greater than that which may result in a viscosity undesirable or unsuitable for intravenous administration. In certain embodiments, the sugar may be disaccharides, *e.g*., sucrose. In certain embodiments, the liquid formulation may also include one or more of a buffering agent, a surfactant, and a preservative.

In certain embodiments, the pH of the liquid formulation may be set by addition of a pharmaceutically acceptable acid and/or base. In certain embodiments, the pharmaceutically acceptable acid may be hydrochloric acid. In certain embodiments, the base may be sodium hydroxide.

In addition to aggregation, deamidation is a common product variant of peptides and proteins that may occur during fermentation, harvest/cell clarification, purification, drug substance/drug product storage and during sample analysis. Deamidation is the loss of NH3 from a protein forming a succinimide intermediate that can undergo hydrolysis. The succinimide intermediate results in a 17 dalton mass decrease of the parent peptide. The subsequent hydrolysis results in an 18 dalton mass increase. Isolation of the succinimide intermediate is difficult due to instability under aqueous conditions. As such, deamidation is typically detectable as 1 dalton mass increase. Deamidation of an asparagine results in either aspartic or isoaspartic acid. The parameters affecting the rate of deamidation include pH, temperature, solvent dielectric constant, ionic strength, primary sequence, local polypeptide conformation and tertiary structure. The amino acid residues adjacent to Asn in the peptide chain affect deamidation rates. Gly and Ser following an Asn in protein sequences results in a higher susceptibility to deamidation.

In certain embodiments, the liquid formulation of the present disclosure may be preserved under conditions of pH and humidity to prevent deamination of the protein product.

The aqueous carrier of interest herein is one which is pharmaceutically acceptable (safe and non-toxic for administration to a human) and is useful for the preparation of a liquid formulation. Illustrative carriers include sterile water for injection (SWFI), bacteriostatic water for injection (BWFI), a pH buffered solution *(e.g.,* phosphate-buffered saline), sterile saline solution, Ringer's solution or dextrose solution.

A preservative may be optionally added to the formulations herein to reduce bacterial action. The addition of a preservative may, for example, facilitate the production of a multi-use (multiple-dose) formulation.

Intravenous (IV) formulations may be the preferred administration route in particular instances, such as when a patient is in the hospital after transplantation receiving all drugs via the IV route. In certain embodiments, the liquid formulation is diluted with 0.9% Sodium Chloride solution before administration. In certain embodiments, the diluted drug product for injection is isotonic and suitable for administration by intravenous infusion.

In certain embodiments, a salt or buffer components may be added in an amount of 10 mM - 200 mM. The salts and/or buffers are pharmaceutically acceptable and are derived from various known acids (inorganic and organic) with "base forming" metals or amines. In certain embodiments, the buffer may be phosphate buffer. In certain embodiments, the buffer may be glycinate, carbonate, citrate buffers, in which case, sodium, potassium or ammonium ions can serve as counterion.

A preservative may be optionally added to the formulations herein to reduce bacterial action. The addition of a preservative may, for example, facilitate the production of a multi-use (multiple-dose) formulation.

The aqueous carrier of interest herein is one which is pharmaceutically acceptable (safe and non-toxic for administration to a human) and is useful for the preparation of a liquid formulation. Illustrative carriers include sterile water for injection (SWFI), bacteriostatic water for injection (BWFI), a pH buffered solution *(e.g.,* phosphate-buffered saline), sterile saline solution, Ringer's solution or dextrose solution.

The protein of the present disclosure could exist in a lyophilized formulation including the proteins and a lyoprotectant. The lyoprotectant may be sugar, *e.g.,* disaccharides. In certain embodiments, the lyoprotectant may be sucrose or maltose. The lyophilized formulation may also include one or more of a buffering agent, a surfactant, a bulking agent, and/or a preservative.

The amount of sucrose or maltose useful for stabilization of the lyophilized drug product may be in a weight ratio of at least 1:2 protein to sucrose or maltose. In certain embodiments, the protein to sucrose or maltose weight ratio may be of from 1:2 to 1:5.

In certain embodiments, the pH of the formulation, prior to lyophilization, may be set by addition of a pharmaceutically acceptable acid and/or base. In certain embodiments the pharmaceutically acceptable acid may be hydrochloric acid. In certain embodiments, the pharmaceutically acceptable base may be sodium hydroxide.

Before lyophilization, the pH of the solution containing the protein of the present disclosure may be adjusted between 6 to 8. In certain embodiments, the pH range for the lyophilized drug product may be from 7 to 8.

In certain embodiments, a salt or buffer components may be added in an amount of 10 mM - 200 mM. The salts and/or buffers are pharmaceutically acceptable and are derived from various known acids (inorganic and organic) with "base forming" metals or amines. In certain embodiments, the buffer may be phosphate buffer. In certain embodiments, the buffer may be glycinate, carbonate, citrate buffers, in which case, sodium, potassium or ammonium ions can serve as counterion.

In certain embodiments, a "bulking agent" may be added. A "bulking agent" is a compound which adds mass to a lyophilized mixture and contributes to the physical structure of the lyophilized cake *(e.g.,* facilitates the production of an essentially uniform lyophilized cake which maintains an open pore structure). Illustrative bulking agents include mannitol, glycine, polyethylene glycol and sorbitol. The lyophilized formulations of the present invention may contain such bulking agents.

A preservative may be optionally added to the formulations herein to reduce bacterial action. The addition of a preservative may, for example, facilitate the production of a multi-use (multiple-dose) formulation.

In certain embodiments, the lyophilized drug product may be constituted with an aqueous carrier. The aqueous carrier of interest herein is one which is pharmaceutically acceptable (e.g., safe and non-toxic for administration to a human) and is useful for the preparation of a liquid formulation, after lyophilization. Illustrative diluents include sterile water for injection (SWFI), bacteriostatic water for injection (BWFI), a pH buffered solution (e.g., phosphate-buffered saline), sterile saline solution, Ringer's solution or dextrose solution.

In certain embodiments, the lyophilized drug product of the current disclosure is reconstituted with either Sterile Water for Injection, USP (SWFI) or 0.9% Sodium Chloride Injection, USP. During reconstitution, the lyophilized powder dissolves into a solution.

In certain embodiments, the lyophilized protein product of the instant disclosure is constituted to about 4.5 mL water for injection and diluted with 0.9% saline solution (sodium chloride solution).

Actual dosage levels of the active ingredients in the pharmaceutical compositions of this invention may be varied so as to obtain an amount of the active ingredient which is effective to achieve the desired therapeutic response for a particular patient, composition, and mode of administration, without being toxic to the patient.

The specific dose can be a uniform dose for each patient, for example, 50-5000 mg of protein. Alternatively, a patient's dose can be tailored to the approximate body weight or surface area of the patient. Other factors in determining the appropriate dosage can include the disease or condition to be treated or prevented, the severity of the disease, the route of administration, and the age, sex and medical condition of the patient. Further refinement of the calculations necessary to determine the appropriate dosage for treatment is routinely made by those skilled in the art, especially in light of the dosage information and assays disclosed herein. The dosage can also be determined through the use of known assays for determining dosages used in conjunction with appropriate dose-response data. An individual patient's dosage can be adjusted as the progress of the disease is monitored. Blood levels of the targetable construct or complex in a patient can be measured to see if the dosage needs to be adjusted to reach or maintain an effective concentration. Pharmacogenomics may be used to determine which targetable constructs and/or complexes, and dosages thereof, are most likely to be effective for a given individual (Schmitz et al., Clinica Chimica Acta 308: 43-53, 2001; Steimer et al., Clinica Chimica Acta 308: 33-41, 2001).

In general, dosages based on body weight are from about 0.01 µg to about 100 mg per kg of body weight, such as about 0.01 µg to about 100 mg/kg of body weight, about 0.01 µg to about 50 mg/kg of body weight, about 0.01 µg to about 10 mg/kg of body weight, about 0.01 µg to about 1 mg/kg of body weight, about 0.01 µg to about 100 µg/kg of body weight, about 0.01 µg to about 50 µg/kg of body weight, about 0.01 µg to about 10 µg/kg of body weight, about 0.01 µg to about 1 µg/kg of body weight, about 0.01 µg to about 0.1 µg/kg of body weight, about 0.1 µg to about 100 mg/kg of body weight, about 0.1 µg to about 50 mg/kg of body weight, about 0.1 µg to about 10 mg/kg of body weight, about 0.1 µg to about 1 mg/kg of body weight, about 0.1 µg to about 100 µg/kg of body weight, about 0.1 µg to about 10 µg/kg of body weight, about 0.1 µg to about 1 µg/kg of body weight, about 1 µg to about 100 mg/kg of body weight, about 1 µg to about 50 mg/kg of body weight, about 1 µg to about 10 mg/kg of body weight, about 1 µg to about 1 mg/kg of body weight, about 1 µg to about 100 µg/kg of body weight, about 1 µg to about 50 µg/kg of body weight, about 1 µg to about 10 µg/kg of body weight, about 10 µg to about 100 mg/kg of body weight, about 10 µg to about 50 mg/kg of body weight, about 10 µg to about 10 mg/kg of body weight, about 10 µg to about 1 mg/kg of body weight, about 10 µg to about 100 µg/kg of body weight, about 10 µg to about 50 µg/kg of body weight, about 50 µg to about 100 mg/kg of body weight, about 50µg to about 50 mg/kg of body weight, about 50 µg to about 10 mg/kg of body weight, about 50 µg to about 1 mg/kg of body weight, about 50 µg to about 100 µg/kg of body weight, about 100 µg to about 100 mg/kg of body weight, about 100 µg to about 50 mg/kg of body weight, about 100 µg to about 10 mg/kg of body weight, about 100 µg to about 1 mg/kg of body weight, about 1 mg to about 100 mg/kg of body weight, about 1 mg to about 50 mg/kg of body weight, about 1 mg to about 10 mg/kg of body weight, about 10 mg to about 100 mg/kg of body weight, about 10 mg to about 50 mg/kg of body weight, about 50 mg to about 100 mg/kg of body weight.

Doses may be given once or more times daily, weekly, monthly or yearly, or even once every 2 to 20 years. Persons of ordinary skill in the art can easily estimate repetition rates for dosing based on measured residence times and concentrations of the targetable construct or complex in bodily fluids or tissues. Administration of the present invention could be intravenous, intraarterial, intraperitoneal, intramuscular, subcutaneous, intrapleural, intrathecal, intracavitary, by perfusion through a catheter or by direct intralesional injection. This may be administered once or more times daily, once or more times weekly, once or more times monthly, and once or more times annually.

The description above describes multiple aspects and embodiments of the invention. The patent application specifically contemplates all combinations and permutations of the aspects and embodiments.

### EXAMPLES

The invention now being generally described, will be more readily understood by reference to the following examples, which are included merely for purposes of illustration of certain aspects and embodiments of the present invention, and which are not intended to limit the invention.

### Example 1 - NKG2D binding domains bind to NKG2D

### NKG2D-binding domains bind to purified recombinant NKG2D

The nucleic acid sequences of human, mouse, or cynomolgus NKG2D ectodomains were fused with nucleic acid sequences encoding human IgG1 Fc domains and introduced into mammalian cells to be expressed. After purification, NKG2D-Fc fusion proteins were adsorbed to wells of microplates. After blocking the wells with bovine serum albumin to prevent non-specific binding, NKG2D-binding domains were titrated and added to the wells pre-adsorbed with NKG2D-Fc fusion proteins. Primary antibody binding was detected using a secondary antibody which was conjugated to horseradish peroxidase and specifically recognizes a human kappa light chain to avoid Fc cross-reactivity. 3,3',5,5'-Tetramethylbenzidine (TMB), a substrate for horseradish peroxidase, was added to the wells to visualize the binding signal, whose absorbance was measured at 450 nM and corrected at 540 nM. An NKG2D-binding domain clone, an isotype control or a positive control (comprising heavy chain and light chain variable domains selected from SEQ ID NOs: 101-104, or anti-mouse NKG2D clones MI-6 and CX-5 available at eBioscience) was added to each well.

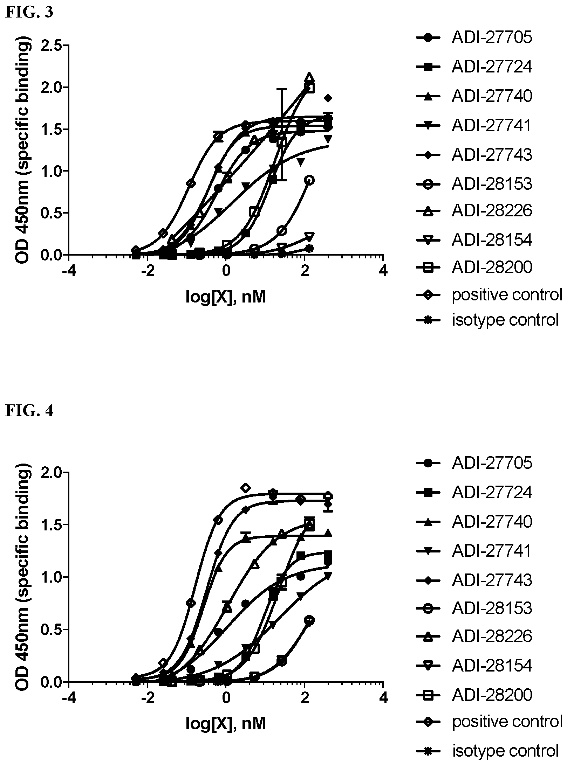

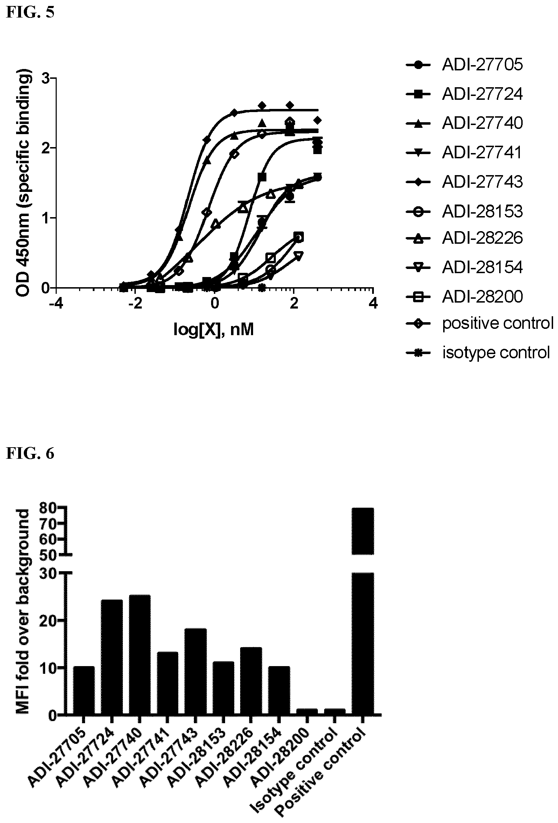

The isotype control showed minimal binding to recombinant NKG2D-Fc proteins, while the positive control bound strongest to the recombinant antigens. NKG2D-binding domains produced by all clones demonstrated binding across human, mouse, and cynomolgus recombinant NKG2D-Fc proteins, although with varying affinities from clone to clone. Generally, each anti-NKG2D clone bound to human (FIG. 3) and cynomolgus (FIG. 4) recombinant NKG2D-Fc with similar affinity, but with lower affinity to mouse (FIG. 5) recombinant NKG2D-Fc.

### NKG2D-binding domains bind to cells expressing NKG2D

EL4 mouse lymphoma cell lines were engineered to express human or mouse NKG2D-CD3 zeta signaling domain chimeric antigen receptors. An NKG2D-binding clone, an isotype control, or a positive control was used at a 100 nM concentration to stain extracellular NKG2D expressed on the EL4 cells. The antibody binding was detected using fluorophore-conjugated anti-human IgG secondary antibodies. Cells were analyzed by flow cytometry, and fold-over-background (FOB) was calculated using the mean fluorescence intensity (MFI) of NKG2D-expressing cells compared to parental EL4 cells.

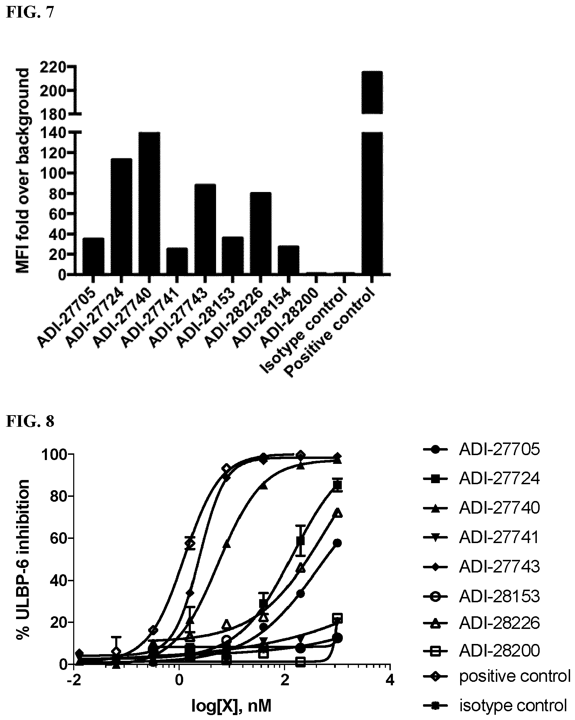

NKG2D-binding domains produced by all clones bound to EL4 cells expressing human and mouse NKG2D. Positive control antibodies (comprising heavy chain and light chain variable domains selected from SEQ ID NOs: 101-104, or anti-mouse NKG2D clones MI-6 and CX-5 available at eBioscience) gave the best FOB binding signal. The NKG2D-binding affinity for each clone was similar between cells expressing human NKG2D (FIG. 6) and mouse (FIG. 7) NKG2D.

### Example 2 - NKG2D-binding domains block natural ligand binding to NKG2D

### Competition With ULBP-6

Recombinant human NKG2D-Fc proteins were adsorbed to wells of a microplate, and the wells were blocked with bovine serum albumin to reduce non-specific binding. A saturating concentration of ULBP-6-His-biotin was added to the wells, followed by addition of the NKG2D-binding domain clones. After a 2-hour incubation, wells were washed and ULBP-6-His-biotin that remained bound to the NKG2D-Fc coated wells was detected by streptavidin-conjugated to horseradish peroxidase and TMB substrate. Absorbance was measured at 450 nM and corrected at 540 nM. After subtracting background, specific binding of NKG2D-binding domains to the NKG2D-Fc proteins was calculated from the percentage of ULBP-6-His-biotin that was blocked from binding to the NKG2D-Fc proteins in wells. The positive control antibody (comprising heavy chain and light chain variable domains selected from SEQ ID NOs: 101-104) and various NKG2D-binding domains blocked ULBP-6 binding to NKG2D, while isotype control showed little competition with ULBP-6 (FIG. 8).

### ULBP-6 sequence is represented by SEQ ID NO: 108

MAAAAIPALLLCLPLLFLLFGWSRARRDDPHSLCYDITVIPKFRPGPRWCAVQ
GQVDEKTFLHYDCGNKTVTPVSPLGKKLNVTMAWKAQNPVLREVVDILTEQ
LLDIQLENYTPKEPLTLQARMSCEQKAEGHSSGSWQFSIDGQTFLLFDSEKRM
WTTVHPGARKMKEKWENDKDVAMSFHYISMGDCIGWLEDFLMGMDSTLEP
SAGAPLAMSSGTTQLRATATTLILCCLLIILPCFILPGI (SEQ ID NO:108)

### Competition With MICA

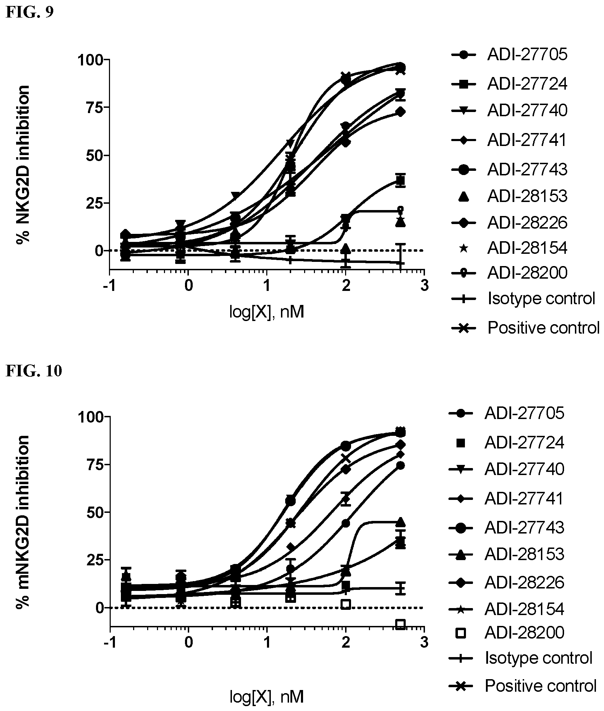

Recombinant human MICA-Fc proteins were adsorbed to wells of a microplate, and the wells were blocked with bovine serum albumin to reduce non-specific binding. NKG2D-Fc-biotin was added to wells followed by NKG2D-binding domains. After incubation and washing, NKG2D-Fc-biotin that remained bound to MICA-Fc coated wells was detected using streptavidin-HRP and TMB substrate. Absorbance was measured at 450 nM and corrected at 540 nM. After subtracting background, specific binding of NKG2D-binding domains to the NKG2D-Fc proteins was calculated from the percentage of NKG2D-Fc-biotin that was blocked from binding to the MICA-Fc coated wells. The positive control antibody (comprising heavy chain and light chain variable domains selected from SEQ ID NOs: 101-104) and various NKG2D-binding domains blocked MICA binding to NKG2D, while isotype control showed little competition with MICA (FIG. 9).

### Competition With Rae-1 delta

Recombinant mouse Rae-1delta-Fc (purchased from R&D Systems) was adsorbed to wells of a microplate, and the wells were blocked with bovine serum albumin to reduce non-specific binding. Mouse NKG2D-Fc-biotin was added to the wells followed by NKG2D-binding domains. After incubation and washing, NKG2D-Fc-biotin that remained bound to Rae-1delta-Fc coated wells was detected using streptavidin-HRP and TMB substrate. Absorbance was measured at 450 nM and corrected at 540 nM. After subtracting background, specific binding of NKG2D-binding domains to the NKG2D-Fc proteins was calculated from the percentage of NKG2D-Fc-biotin that was blocked from binding to the Rae-1delta-Fc coated wells. The positive control (comprising heavy chain and light chain variable domains selected from SEQ ID NOs: 101-104, or anti-mouse NKG2D clones MI-6 and CX-5 available at eBioscience) and various NKG2D-binding domain clones blocked Rae-1delta binding to mouse NKG2D, while the isotype control antibody showed little competition with Rae-1delta (FIG. 10).

### Example 3 - NKG2D-binding domain clones activate NKG2D

Nucleic acid sequences of human and mouse NKG2D were fused to nucleic acid sequences encoding a CD3 zeta signaling domain to obtain chimeric antigen receptor (CAR) constructs. The NKG2D-CAR constructs were then cloned into a retrovirus vector using Gibson assembly and transfected into expi293 cells for retrovirus production. EL4 cells were infected with viruses containing NKG2D-CAR together with 8 µg/mL polybrene. 24 hours after infection, the expression levels of NKG2D-CAR in the EL4 cells were analyzed by flow cytometry, and clones which express high levels of the NKG2D-CAR on the cell surface were selected.

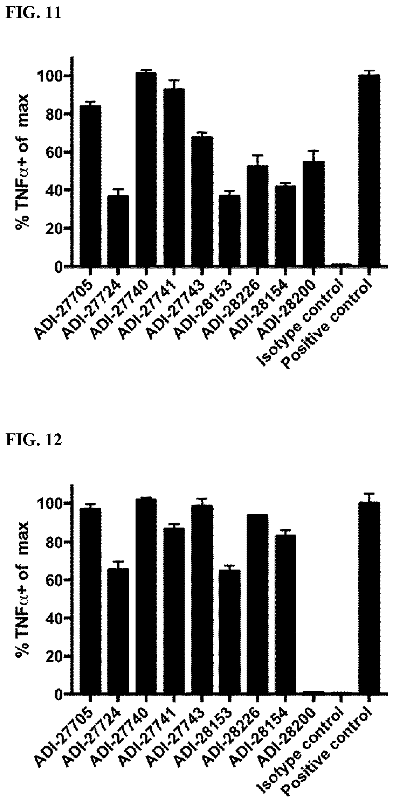

To determine whether NKG2D-binding domains activate NKG2D, they were adsorbed to wells of a microplate, and NKG2D-CAR EL4 cells were cultured on the antibody fragment-coated wells for 4 hours in the presence of brefeldin-A and monensin. Intracellular TNF-α production, an indicator for NKG2D activation, was assayed by flow cytometry. The percentage of TNF-α positive cells was normalized to the cells treated with the positive control. All NKG2D-binding domains activated both human NKG2D (FIG. 11) and mouse NKG2D (FIG. 12).

### Example 4 - NKG2D-binding domains activate NK cells

### Primary human NK cells

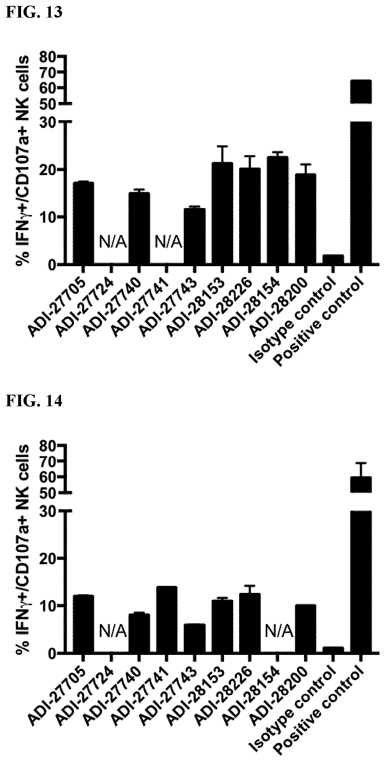

Peripheral blood mononuclear cells (PBMCs) were isolated from human peripheral blood buffy coats using density gradient centrifugation. NK cells (CD3⁻ CD56⁺) were isolated using negative selection with magnetic beads from PBMCs, and the purity of the isolated NK cells was typically >95%. Isolated NK cells were then cultured in media containing 100 ng/mL IL-2 for 24-48 hours before they were transferred to the wells of a microplate to which the NKG2D-binding domains were adsorbed, and cultured in the media containing fluorophore-conjugated anti-CD107a antibody, brefeldin-A, and monensin. Following culture, NK cells were assayed by flow cytometry using fluorophore-conjugated antibodies against CD3, CD56 and IFN-γ. CD107a and IFN-γ staining were analyzed in CD3⁻ CD56⁺ cells to assess NK cell activation. The increase in CD107a/IFN-γ double-positive cells is indicative of better NK cell activation through engagement of two activating receptors rather than one receptor. NKG2D-binding domains and the positive control *(e.g.,* heavy chain variable domain represent by SEQ ID NO: 101 or SEQ ID NO: 103, and light chain variable domain represented by SEQ ID NO: 102 or SEQ ID NO: 104) showed a higher percentage of NK cells becoming CD107a⁺ and IFN-γ⁺ than the isotype control (FIG. 13 & FIG. 14 represent data from two independent experiments, each using a different donor's PBMC for NK cell preparation).

### Primary mouse NK cells

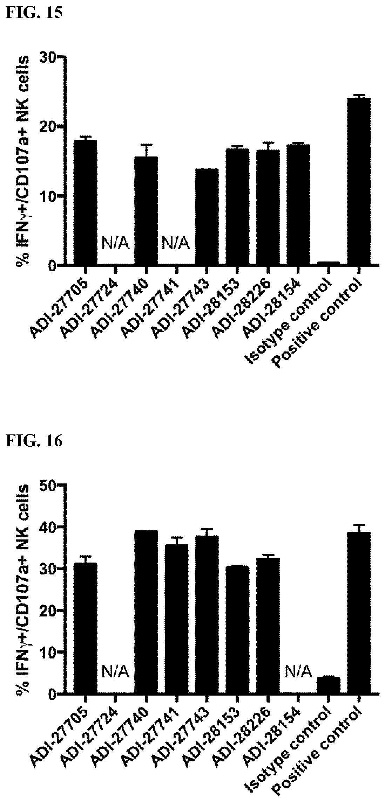

Spleens were obtained from C57B1/6 mice and crushed through a 70 µm cell strainer to obtain single cell suspension. Cells were pelleted and resuspended in ACK lysis buffer (purchased from Thermo Fisher Scientific #A1049201; 155 mM ammonium chloride, 10 mM potassium bicarbonate, 0.01 mM EDTA) to remove red blood cells. The remaining cells were cultured with 100 ng/mL hIL-2 for 72 hours before being harvested and prepared for NK cell isolation. NK cells (CD3⁻NK1.1⁺) were then isolated from spleen cells using a negative depletion technique with magnetic beads with typically >90% purity. Purified NK cells were cultured in media containing 100 ng/mL mIL-15 for 48 hours before they were transferred to the wells of a microplate to which the NKG2D-binding domains were adsorbed, and cultured in the media containing fluorophore-conjugated anti-CD107a antibody, brefeldin-A, and monensin. Following culture in NKG2D-binding domain-coated wells, NK cells were assayed by flow cytometry using fluorophore-conjugated antibodies against CD3, NK1.1 and IFN-γ. CD107a and IFN-γ staining were analyzed in CD3⁻NK1.1' cells to assess NK cell activation. The increase in CD107a/IFN-γ double-positive cells is indicative of better NK cell activation through engagement of two activating receptors rather than one receptor. NKG2D-binding domains and the positive control (selected from anti-mouse NKG2D clones MI-6 and CX-5 available at eBioscience) showed a higher percentage of NK cells becoming CD107a⁺ and IFN-γ⁺ than the isotype control (FIG. 15 & FIG. 16 represent data from two independent experiments, each using a different mouse for NK cell preparation).

### Example 5 - NKG2D-binding domains enable cytotoxicity of target tumor cells

Human and mouse primary NK cell activation assays demonstrated increased cytotoxicity markers on NK cells after incubation with NKG2D-binding domains. To address whether this translates into increased tumor cell lysis, a cell-based assay was utilized where each NKG2D-binding domain was developed into a monospecific antibody. The Fc region was used as one targeting arm, while the Fab fragment regions (NKG2D-binding domain) acted as another targeting arm to activate NK cells. THP-1 cells, which are of human origin and express high levels of Fc receptors, were used as a tumor target and a Perkin Elmer DELFIA Cytotoxicity Kit was used. THP-1 cells were labeled with BATDA reagent, and resuspended at 10⁵/mL in culture media. Labeled THP-1 cells were then combined with NKG2D antibodies and isolated mouse NK cells in wells of a microtiter plate at 37 °C for 3 hours. After incubation, 20 µL of the culture supernatant was removed, mixed with 200 µL of Europium solution and incubated with shaking for 15 minutes in the dark. Fluorescence was measured over time by a PheraStar plate reader equipped with a time-resolved fluorescence module (Excitation 337 nM, Emission 620 nM) and specific lysis was calculated according to the kit instructions.

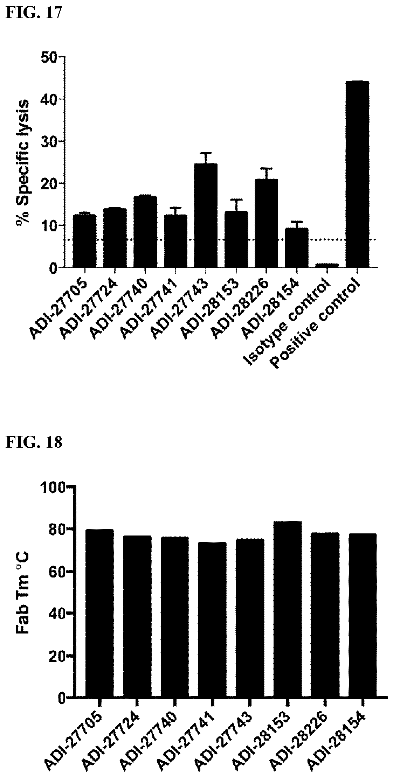

The positive control, ULBP-6 - a natural ligand for NKG2D - conjugated to Fc, showed increased specific lysis of THP-1 target cells by mouse NK cells. NKG2D antibodies also increased specific lysis of THP-1 target cells, while isotype control antibody showed reduced specific lysis. The dotted line indicates specific lysis of THP-1 cells by mouse NK cells without antibody added (FIG. 17).

### Example 6 - NKG2D antibodies show high thermostability

Melting temperatures of NKG2D-binding domains were assayed using differential scanning fluorimetry. The extrapolated apparent melting temperatures are high relative to typical IgG1 antibodies (FIG. 18).

### Example 7 - Synergistic activation of human NK cells by cross-linking NKG2D and CD16

### Primary human NK cell activation assay

Peripheral blood mononuclear cells (PBMCs) were isolated from peripheral human blood buffy coats using density gradient centrifugation. NK cells were purified from PBMCs using negative magnetic beads (StemCell # 17955). NK cells were >90% CD3⁻ CD56⁺ as determined by flow cytometry. Cells were then expanded 48 hours in media containing 100 ng/mL hIL-2 (Peprotech #200-02) before use in activation assays. Antibodies were coated onto a 96-well flat-bottom plate at a concentration of 2 µg/mL (anti-CD 16, Biolegend # 302013) and 5 µg/mL (anti-NKG2D, R&D #MAB139) in 100 µL sterile PBS overnight at 4 °C followed by washing the wells thoroughly to remove excess antibody. For the assessment of degranulation IL-2-activated NK cells were resuspended at 5 × 10⁵ cells/mL in culture media supplemented with 100 ng/mL human IL-2 (hIL2) and 1 µg/mL APC-conjugated anti-CD107a mAb (Biolegend # 328619). 1×10⁵ cells/well were then added onto antibody coated plates. The protein transport inhibitors Brefeldin A (BFA, Biolegend # 420601) and Monensin (Biolegend # 420701) were added at a final dilution of 1:1000 and 1:270, respectively. Plated cells were incubated for 4 hours at 37 °C in 5% CO₂. For intracellular staining of IFN-γ, NK cells were labeled with anti-CD3 (Biolegend #300452) and anti-CD56 mAb (Biolegend # 318328), and subsequently fixed, permeabilized and labeled with anti-IFN-γ mAb (Biolegend # 506507). NK cells were analyzed for expression of CD107a and IFN-γ by flow cytometry after gating on live CD56⁺CD3⁻cells.

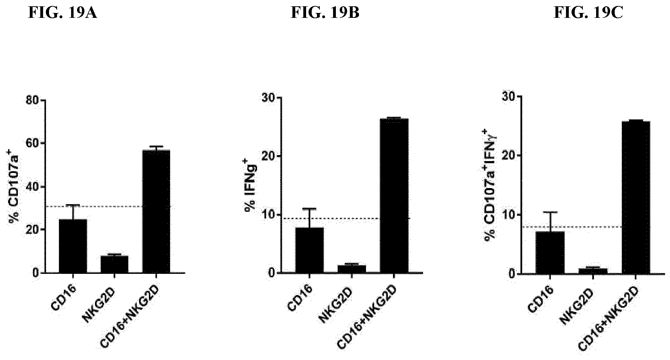

To investigate the relative potency of receptor combination, crosslinking of NKG2D or CD16, and co-crosslinking of both receptors by plate-bound stimulation was performed. As shown in Figure 19 (FIGs. 19A-19C), combined stimulation of CD16 and NKG2D resulted in highly elevated levels of CD107a (degranulation) (FIG. 19A) and/or IFN-γ production (FIG. 19B). Dotted lines represent an additive effect of individual stimulations of each receptor.

CD107a levels and intracellular IFN-γ production of IL-2-activated NK cells were analyzed after 4 hours of plate-bound stimulation with anti-CD16, anti-NKG2D or a combination of both monoclonal antibodies. Graphs indicate the mean (n = 2) ± Sd. FIG. 19A demonstrates levels of CD107a; FIG. 19B demonstrates levels of IFN-γ; FIG. 19C demonstrates levels of CD107a and IFN-γ. Data shown in FIGs. 19A-19C are representative of five independent experiments using five different healthy donors.

### Example 8 - Trispecific binding protein (TriNKET)-mediated enhanced cytotoxicity of target cells

### Assessment of TriNKET or mAb binding to cell expressed human cancer antigens

Human cancer cell lines expressing EPCAM were used to assess tumor antigen binding of TriNKETs derived from EPCAM targeting clone MT110 in F4 and F3' formats. The human cell lines H747, HCC827 and HCT116 were used to assess binding of TriNKETs and mAb to cell expressed EPCAM. TriNKETs or mAb were diluted and incubated with the respective cells. Binding was detected using a fluorophore conjugated anti-human IgG secondary antibody. Cells were analyzed by flow cytometry, binding MFI to cell expressed EPCAM was normalized to human recombinant IgG1 stained controls to obtain fold over background values.

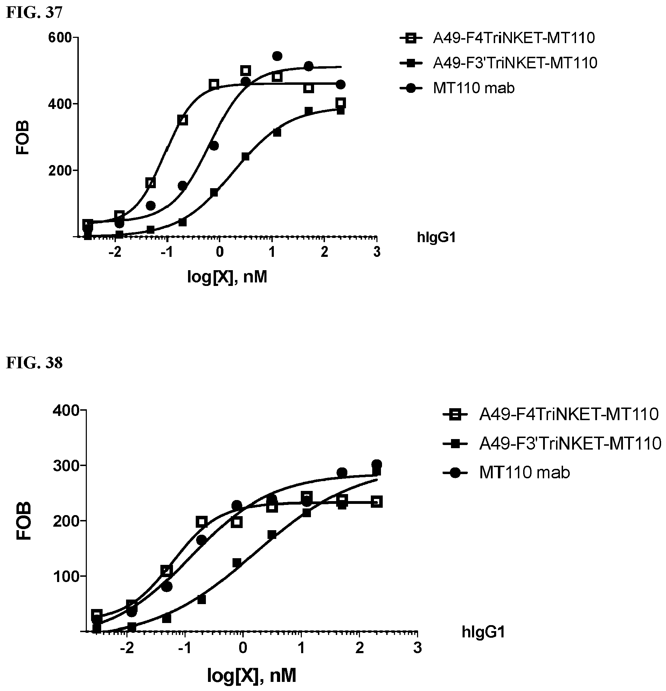

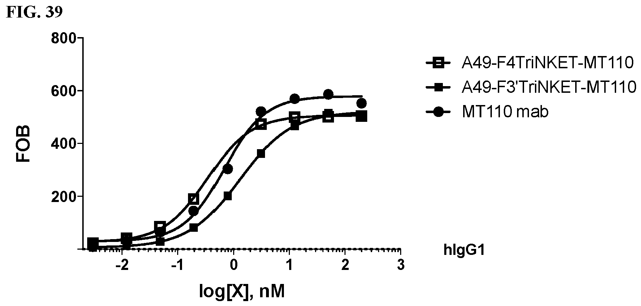

FIG. 37 shows binding of trispecific binding proteins (TriNKETs) of the present disclosure (A49-F4-TriNKET-MT110 and A49-F3'-TriNKET-MT110) and parental monoclonal antibody (mAb) to EpCAM expressing H747 human colorectal cancer cells. FIG. 38 shows binding of trispecific binding proteins (TriNKETs) of the present disclosure (A49-F4-TriNKET-MT110 and A49-F3'-TriNKET-MT110) and parental monoclonal antibody (mAb) to EpCAM expressing HCC827 human lung cancer cells. FIG. 39 shows binding of trispecific binding proteins (TriNKETs) of the present disclosure (A49-F4-TriNKET-MT110 and A49-F3'-TriNKET-MT110) and parental monoclonal antibody (mAb) to EpCAM expressing HCT116 human colorectal cancer cells. Overall binding was stronger with F4-TriNKET compared to F3'-TriNKET that incorporate MT110 EPCAM binder.

### Primary human NK cell cytotoxicity assay

PBMCs were isolated from human peripheral blood buffy coats using density gradient centrifugation. Isolated PBMCs were washed and prepared for NK cell isolation. NK cells were isolated using a negative selection technique with magnetic beads. Purity of isolated NK cells achieved was typically greater than 90% CD3⁻ CD56⁺. Isolated NK cells were incubated overnight without cytokine, and used the following day in cytotoxicity assays.

### DELFIA cytotoxicity assay

Human cancer cell lines expressing a target of interest were harvested from culture, washed with HBS, and resuspended in growth media at 10⁶ cells/mL for labeling with BATDA reagent (Perkin Elmer, AD0116). Manufacturer instructions were followed for labeling of the target cells. After labeling, cells were washed 3 times with HBS and resuspended at 0.5x10⁵ cells/mL in culture media. To prepare the background wells, an aliquot of the labeled cells was put aside, and the cells were spun out of the media. 100 µL of the media was carefully added to wells in triplicate to avoid disturbing the pelleted cells. 100 µL of BATDA-labeled cells were added to each well of the 96-well plate. Wells were saved for spontaneous release from target cells and prepared for lysis of target cells by addition of 1% Triton-X. Monoclonal antibodies or TriNKETs against the tumor target of interest were diluted in culture media, and 50 µL of diluted mAb or TriNKET was added to each well. Rested NK cells were harvested from culture, washed, and resuspended at 1.0x10⁵-2.0x10⁶ cell/mL in culture media, depending on the desired effector to target cell ratio. 50 µL of NK cells were added to each well of the plate to provide a total of 200 µL culture volume. The plate was incubated at 37 °C with 5% CO₂ for 2-4 hours before developing the assay.

After culturing for 2-3 hours, the plate was removed from the incubator and the cells were pelleted by centrifugation at 200xg for 5 minutes. 20 µL of culture supernatant was transferred to a clean microplate provided from the manufacturer, and 200 µL of room temperature Europium solution was added to each well. The plate was protected from light and incubated on a plate shaker at 250 rpm for 15 minutes. The plate was read using a SpectraMax^{®} i3X instrument (Molecular Devices), and percent specific lysis was calculated (% Specific lysis = (Experimental release - Spontaneous release) / (Maximum release - Spontaneous release)) x 100).

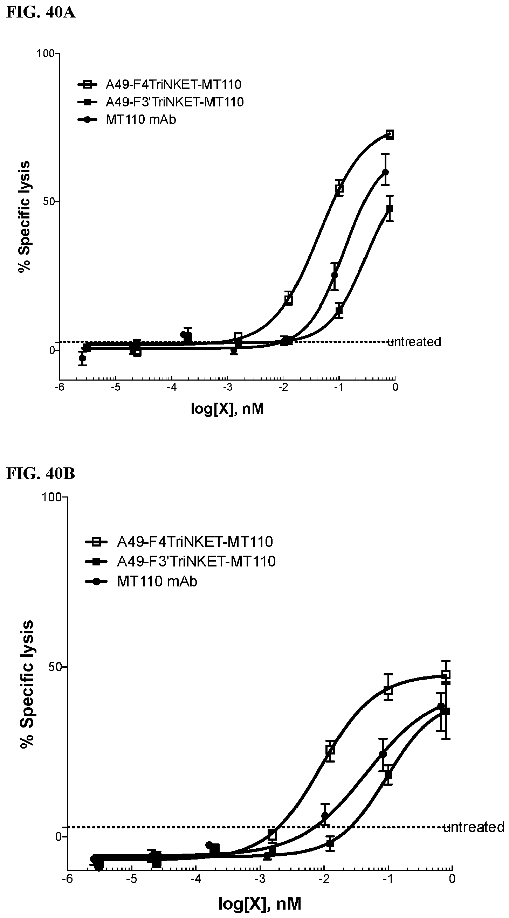

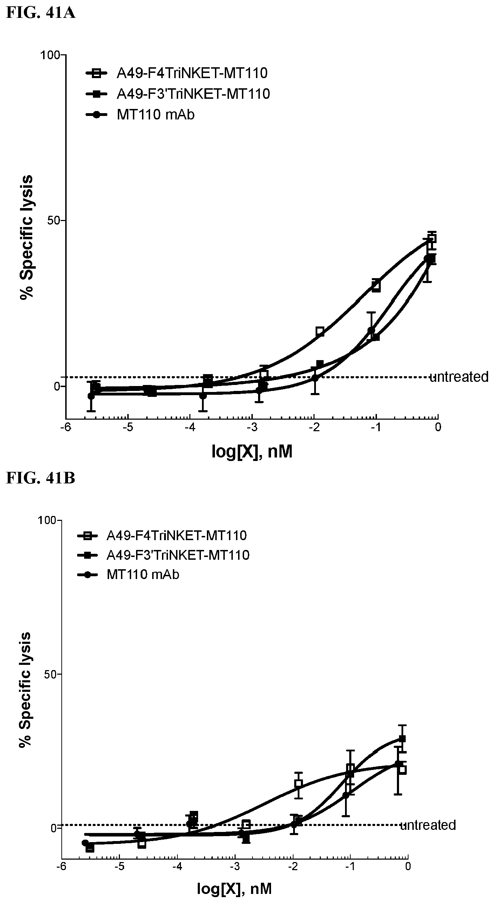

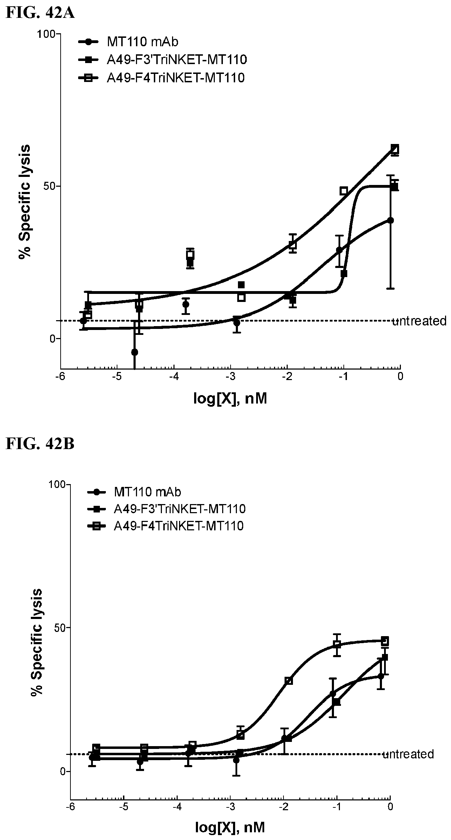

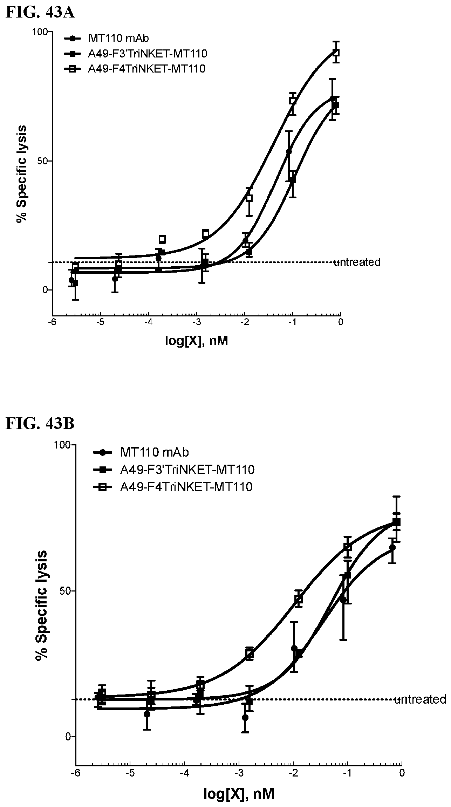

FIG. 40A and FIG. 40B TriNKET-mediated cytotoxicity of rested human NK cells from two different healthy donors against H747 human cancer cells. FIG. 41A and FIG. 41B TriNKET-mediated cytotoxicity of rested human NK cells from two different healthy donors against HCC827 human cancer cells. FIG. 42A and FIG. 42B TriNKET-mediated cytotoxicity of rested human NK cells from two different healthy donors against MCF7 human cancer cells. FIG. 43A and FIG. 43B TriNKET-mediated cytotoxicity of rested human NK cells from two different healthy donors against HCT116 human cancer cells. EPCAM-targeting F4-TriNKET killed target cells more effectively than the parental mAb targeting EPCAM. F4-TriNKET also killed target cells more potently than F3'-TriNKET, which may be a reflection of the stronger binding of F4-TriNKET to target cells.

### INCORPORATION BY REFERENCE

The entire disclosure of each of the patent documents and scientific articles referred to herein is incorporated by reference for all purposes.

### EQUIVALENTS

The invention may be embodied in other specific forms without departing from the spirit or essential characteristics thereof. The foregoing embodiments are therefore to be considered in all respects illustrative rather than limiting the invention described herein. Scope of the invention is thus indicated by the appended claims rather than by the foregoing description, and all changes that come within the meaning and range of equivalency of the claims are intended to be embraced therein.

### Embodiments of the Invention

1. A protein comprising:
   (a) a first antigen-binding site that binds Natural killer group 2 member D (NKG2D);
   (b) a second antigen-binding site that binds an antigen selected from the group consisting of: EpCAM, Cancer Antigen 125 (CA125), sodium/phosphate cotransporter 2B (NaPi2b), Nectin cell adhesion molecule 4 (Nectin4), Fucosyl-GM1 (monosialotetrahexosylganglioside), disintegrin and metalloproteinase domain-containing protein 8 (ADAM8), disintegrin and metalloproteinase domain-containing protein 9 (ADAM9), solute carrier family 44 member 4 (SLC44A4), and sialylated Lewis a antigen (CA19-9); and
   (c) an antibody Fc domain or a portion thereof sufficient to bind cluster of differentiation 16 (CD16), or a third antigen-binding site that binds CD16.
2. A protein comprising:
   (a) a first antigen-binding site that binds NKG2D;
   (b) a second antigen-binding site that binds EpCAM; and
   (c) an antibody Fc domain or a portion thereof sufficient to bind CD16, or a third antigen-binding site that binds CD16.
3. A protein comprising:
   (a) a first antigen-binding site that binds NKG2D;
   (b) a second antigen-binding site that binds a tumor associated antigen selected from Cancer Antigen 125 (CA125), sodium/phosphate cotransporter 2B (NaPi2b), Nectin cell adhesion molecule 4 (Nectin4), Fucosyl-GM1 (monosialotetrahexosylganglioside), disintegrin and metalloproteinase domain-containing protein 8 (ADAM8), disintegrin and metalloproteinase domain-containing protein 9 (ADAM9), solute carrier family 44 member 4 (SLC44A4), and sialylated Lewis a antigen (CA19-9); and
   (c) an antibody Fc domain or a portion thereof sufficient to bind CD16, or a third antigen-binding site that binds CD16.
4. A protein comprising:
   (a) a first antigen-binding site that binds NKG2D;
   (b) a second antigen-binding site that binds a tumor associated antigen selected from sodium-dependent phosphate transport protein 2b (NaPi2b); and
   (c) an antibody Fc domain or a portion thereof sufficient to bind CD16, or a third antigen-binding site that binds CD16.
5. A protein comprising:
   (a) a first antigen-binding site that binds NKG2D;
   (b) a second antigen-binding site that binds a tumor associated antigen Nectin4; and
   (c) an antibody Fc domain or a portion thereof sufficient to bind CD16, or a third antigen-binding site that binds CD16.
6. A protein comprising:
   (a) a first antigen-binding site that binds NKG2D;
   (b) a second antigen-binding site that binds a multiple myeloma associated antigen Fucosyl-GM1; and
   (c) an antibody Fc domain or a portion thereof sufficient to bind CD16, or a third antigen-binding site that binds CD16.
7. A protein comprising:
   (a) a first antigen-binding site that binds NKG2D;
   (b) a second antigen-binding site that binds a T-cell associated tumor antigen selected from SLC44A4; and
   (c) an antibody Fc domain or a portion thereof sufficient to bind CD16, or a third antigen-binding site that binds CD16.
8. The protein of any one of embodiments 1-7, wherein the first antigen-binding site binds to NKG2D in humans, non-human primates, and rodents.
9. The protein of any one of embodiments 1-8, wherein the first antigen-binding site comprises a heavy chain variable domain and a light chain variable domain.
10. A protein according to embodiment 9, wherein the heavy chain variable domain and the light chain variable domain are present on the same polypeptide.
11. A protein according to embodiment 9 or 10, wherein the second antigen-binding site comprises a heavy chain variable domain and a light chain variable domain.
12. A protein according to embodiment 11, wherein the heavy chain variable domain and the light chain variable domain of the second antigen-binding site are present on the same polypeptide.
13. A protein according to embodiment 11 or 12, wherein the light chain variable domain of the first antigen-binding site has an amino acid sequence identical to the amino acid sequence of the light chain variable domain of the second antigen-binding site.
14. A protein comprising:
   (a) a first antigen-binding site comprising an Fab fragment that binds NKG2D;
   b) a second antigen-binding site comprising a single-chain variable fragment (scFv) that binds EpCAM; and
   (c) an antibody Fc domain or a portion thereof sufficient to bind CD16, or a third antigen-binding site that binds CD16.
15. The protein of embodiment 14, wherein the scFv is linked to the antibody Fc domain or a portion thereof sufficient to bind CD 16, or the third antigen-binding site that binds CD16, via a hinge comprising Ala-Ser or Gly-Ala-Ser, wherein the scFv comprises a heavy chain variable domain and a light chain variable domain.
16. The protein of embodiment 15, wherein the scFv is linked to the antibody Fc domain.
17. The protein of embodiment 14 or 15, wherein the heavy chain variable domain of the scFv forms a disulfide bridge with the light chain variable domain of the scFv.
18. The protein of embodiment 17, wherein the disulfide bridge is formed between C44 from the heavy chain variable domain and C100 from the light chain variable domain.
19. The protein of embodiment 18, wherein the scFv is linked to the antibody Fc domain, wherein the light chain variable domain of the scFv is positioned at the N-terminus of the heavy chain variable domain of the scFv, and is linked to the heavy chain variable domain of the scFv via a flexible linker (GlyGlyGlyGlySer)₄ ((G4S)₄), and the Fab is linked to the antibody Fc domain.
20. A protein according to any one of embodiments 15-19, wherein the heavy chain variable domain of the scFv is linked to the light chain variable domain of the scFv via a flexible linker.
21. The protein of embodiment 20, wherein the flexible linker comprises (GlyGlyGlyGlySer)₄ ((G4S)₄).
22. A protein according to any one of embodiments 15-21, wherein the heavy chain variable domain of the scFv is positioned at the N-terminus or the C-terminus of the light chain variable domain of the scFv.
23. The protein of embodiment 22, wherein the light chain variable domain of the scFv is positioned at the N-terminus of the heavy chain variable domain of the scFv.
24. A protein according to any one of embodiments 14 to 23, wherein the Fab fragment is linked to the antibody Fc domain or a portion thereof sufficient to bind CD 16 or the third antigen-binding site that binds CD16.
25. The protein of embodiment 24, wherein the heavy chain portion of the Fab fragment comprises a heavy chain variable domain and a CH1 domain, and wherein the heavy chain variable domain is linked to the CH1 domain.
26. A protein according to embodiment 24 or 25, wherein the Fab fragment is linked to the antibody Fc domain.
27. A protein according to any one of embodiments 14 to 26 comprising a sequence selected from SEQ ID NO:208 and SEQ ID NO:209.
28. A protein according to any one of embodiments 15-27 comprising an scFv linked to an antibody Fc domain, wherein the scFv linked to the antibody Fc domain is represented by a sequence selected from SEQ ID N0:210 and SEQ ID NO:211.
29. A protein according to any one of embodiments 15-27 comprising a sequence selected from SEQ ID NO:212 and SEQ ID NO:213.
30. A protein according to any one of embodiments 15-26 comprising a sequence at least 90% identical to an amino acid sequence selected from SEQ ID NO:210 and SEQ ID NO:211.
31. A protein according to any one of embodiments 15-26 comprising a sequence at least 95% identical to an amino acid sequence selected from SEQ ID NO:210 and SEQ ID NO:211.
32. A protein according to any one of embodiments 15-26 comprising a sequence at least 99% identical to an amino acid sequence selected from SEQ ID NO:210 and SEQ ID NO:211.
33. A protein according to any one of embodiments 15-32 comprising a sequence at least 90% identical to an amino acid sequence selected from SEQ ID NO:212 and SEQ ID NO:213.
34. A protein according to any one of embodiments 15-32 comprising a sequence at least 95% identical to an amino acid sequence selected from SEQ ID NO:212 and SEQ ID NO:213.
35. A protein according to any one of embodiments 15-32 comprising a sequence at least 99% identical to an amino acid sequence selected from SEQ ID NO:212 and SEQ ID NO:213.
36. A protein comprising:
   (a) a first antigen-binding site comprising a single-chain variable fragment (scFv) that binds NKG2D;
   (b) a second antigen-binding site that binds EpCAM; and
   (c) an antibody Fc domain or a portion thereof sufficient to bind CD16, or a third antigen-binding site that binds CD16.
37. A protein according to embodiment 36 further comprising an additional antigen-binding site that binds EpCAM
38. The protein according to embodiment 36 or 37, wherein the second antigen-binding site that binds EpCAM is an Fab fragment.
39. The protein according to embodiment 37 or 38, wherein the second and the additional antigen-binding site that bind EpCAM are Fab fragments.
40. The protein according to embodiment 36 or 37, wherein the second and the additional antigen-binding site that bind EpCAM are scFvs.
41. The protein according to any one of embodiments 36-40, wherein the heavy chain variable domain of the scFv that binds NKG2D is positioned at the N-terminus or the C-terminus of the light chain variable domain of the scFv.
42. The protein according to embodiment 41, wherein the light chain variable domain is positioned at the N-terminus of the heavy chain variable domain of the scFv that binds NKG2D.
43. The protein according to any one of embodiments 36-42, wherein the scFv that binds to NKG2D is linked to the antibody Fc domain or a portion thereof sufficient to bind CD16, or a third antigen-binding site that binds CD16.
44. The protein according to embodiment 43, wherein the scFv that binds to NKG2D is linked to the antibody Fc domain or a portion thereof sufficient to bind CD 16, or a third antigen-binding site that binds CD16 *via* a hinge comprising Ala-Ser or Gly-Ala-Ser.
45. The protein according to embodiment 43, wherein the scFv that binds to NKG2D is linked to the C-terminus of the antibody Fc domain or a portion thereof sufficient to bind CD16, or a third antigen-binding site that binds CD16 *via* a flexible linker comprising SGSGGGGS (SEQ ID NO: 207).
46. The protein according to embodiment 45, wherein the C-terminus of the antibody Fc domain is linked to the N-terminus of the light chain variable domain of the scFv that binds NKG2D.
47. The protein according to any one of embodiments 36-46, wherein within the scFv that binds NKG2D, a disulfide bridge is formed between the heavy chain variable domain of the scFv and the light chain variable domain of the scFv.
48. The protein according to embodiment 47, wherein the disulfide bridge is formed between C44 from the heavy chain variable domain and C100 from the light chain variable domain.
49. The protein according to any one of embodiments 36-48, wherein, within the scFv that binds NKG2D, the heavy chain variable domain is linked to the light chain variable domain via a flexible linker.
50. The protein according to embodiment 49, wherein the flexible linker comprises (GlyGlyGlyGlySer)₄ (G4S)₄).
51. The protein according to any one of embodiments 40 to 50, wherein the second and the additional antigen-binding site scFvs are linked to the antibody Fc domain or a portion thereof sufficient to bind CD 16, or the third antigen-binding site that binds CD 16, *via* a hinge comprising Ala-Ser.
52. The protein according to any one of embodiments 40 to 51, wherein the second and the additional antigen-binding site scFvs are linked to the antibody Fc domain *via* a hinge comprising Ala-Ser.
53. The protein according to embodiment 51 or 52, wherein a disulfide bridge is formed between the heavy chain variable domain and the light chain variable domain of the second antigen-binding site and/or the additional antigen-binding site.
54. The protein according to embodiment 53, wherein the disulfide bridge is formed between C44 from the heavy chain variable domain and C100 from the light chain variable domain.
55. The protein according to any one of embodiments 36 to 54, wherein the scFv that binds NKG2D comprises a light chain variable domain positioned at the N-terminus of a heavy chain variable domain, wherein the light chain variable domain is linked to the heavy chain variable domain of the scFv via a flexible linker (GlyGlyGlyGlySer)₄ (G4S)₄), and the scFv that binds NKG2D is linked to the antibody Fc domain *via* a hinge comprising Ala-Ser or Gly-Ala-Ser.
56. A protein comprising an amino acid sequence of SEQ ID NO:203.
57. A protein comprising an amino acid sequence of SEQ ID NO:203 and SEQ ID NO:204.
58. A protein comprising an amino acid sequence at least 90% identical to an amino acid sequence of SEQ ID NO:203.
59. A protein comprising an amino acid sequence at least 95% identical to an amino acid sequence of SEQ ID NO:203.
60. A protein comprising an amino acid sequence at least 99% identical to an amino acid sequence of SEQ ID NO:203.
61. A protein according to any one of the preceding embodiments, wherein the first antigen-binding site comprises a heavy chain variable domain at least 90% identical to an amino acid sequence selected from: SEQ ID NO:1, SEQ ID NO:41, SEQ ID NO:49, SEQ ID NO:57, SEQ ID NO:59, SEQ ID NO:61, SEQ ID NO:69, SEQ ID NO:77, SEQ ID NO:85, and SEQ ID NO:93.
62. A protein according to any one of embodiments 1-60, wherein the first antigen-binding site comprises a heavy chain variable domain at least 90% identical to SEQ ID NO:41 and a light chain variable domain at least 90% identical to SEQ ID NO:42.
63. A protein according to any one of embodiments 1-60, wherein the first antigen-binding site comprises a heavy chain variable domain at least 90% identical to SEQ ID NO:49 and a light chain variable domain at least 90% identical to SEQ ID NO:50.
64. A protein according to any one of embodiments 1-60, wherein the first antigen-binding site comprises a heavy chain variable domain at least 90% identical to SEQ ID NO:57 and a light chain variable domain at least 90% identical to SEQ ID NO:58.
65. A protein according to any one of embodiments 1-60, wherein the first antigen-binding site comprises a heavy chain variable domain at least 90% identical to SEQ ID NO:59 and a light chain variable domain at least 90% identical to SEQ ID NO:60.
66. A protein according to any one of embodiments 1-60, wherein the first antigen-binding site comprises a heavy chain variable domain at least 90% identical to SEQ ID NO:61 and a light chain variable domain at least 90% identical to SEQ ID NO:62.
67. A protein according to any one of embodiments 1-60, wherein the first antigen-binding site comprises a heavy chain variable domain at least 90% identical to SEQ ID NO:69 and a light chain variable domain at least 90% identical to SEQ ID NO:70.
68. A protein according to any one of embodiments 1-60, wherein the first antigen-binding site comprises a heavy chain variable domain at least 90% identical to SEQ ID NO:77 and a light chain variable domain at least 90% identical to SEQ ID NO:78.
69. A protein according to any one of embodiments 1-60, wherein the first antigen-binding site comprises a heavy chain variable domain at least 90% identical to SEQ ID NO:85 and a light chain variable domain at least 90% identical to SEQ ID NO:86.
70. A protein according to any one of embodiments 1-60, wherein the first antigen-binding site comprises a heavy chain variable domain at least 90% identical to SEQ ID NO:93 and a light chain variable domain at least 90% identical to SEQ ID NO:94.
71. A protein according to any one of embodiments 1-60, wherein the first antigen-binding site comprises a heavy chain variable domain at least 90% identical to SEQ ID NO: 101 and a light chain variable domain at least 90% identical to SEQ ID NO: 102.
72. A protein according to any one of embodiments 1-60, wherein the first antigen-binding site comprises a heavy chain variable domain at least 90% identical to SEQ ID NO: 103 and a light chain variable domain at least 90% identical to SEQ ID NO: 104.
73. The protein of any one of embodiments 1-10, wherein the first antigen-binding site is a single-domain antibody.
74. The protein of embodiment 73, wherein the single-domain antibody is a V_{H}H fragment or a V_{NAR} fragment.
75. A protein of any one of embodiments 1-10 or 73-74, wherein the second antigen-binding site comprises a heavy chain variable domain and a light chain variable domain.
76. A protein of embodiment 75, wherein the heavy chain variable domain and the light chain variable domain of the second antigen-binding site are present on the same polypeptide.
77. A protein of any of embodiments 1, 2, or 61-72, wherein the second antigen-binding site binds EpCAM, the heavy chain variable domain of the second antigen-binding site comprises an amino acid sequence at least 90% identical to SEQ ID NO: 115 and the light chain variable domain of the second antigen-binding site comprises an amino acid sequence at least 90% identical to SEQ ID NO: 119.
78. A protein of embodiment 77, wherein the heavy chain variable domain of the second antigen-binding site comprises an amino acid sequence including:
   a heavy chain CDR1 sequence identical to the amino acid sequence of SEQ ID NO:116;
   a heavy chain CDR2 sequence identical to the amino acid sequence of SEQ ID NO: 117; and
   a heavy chain CDR3 sequence identical to the amino acid sequence of SEQ ID NO: 118.
79. A protein of embodiment 78, wherein the light chain variable domain of the second antigen-binding site comprises an amino acid sequence including:
   a light chain CDR1 sequence identical to the amino acid sequence of SEQ ID NO: 120;
   a light chain CDR2 sequence identical to the amino acid sequence of SEQ ID NO:121;
   and a light chain CDR3 sequence identical to the amino acid sequence of SEQ ID NO: 122.
80. A protein of any one of embodiments 1, 2, or 61-72, wherein the second antigen-binding site binds EpCAM, the heavy chain variable domain of the second antigen-binding site comprises an amino acid sequence at least 90% identical to SEQ ID NO: 123 and the light chain variable domain of the second antigen-binding site comprises an amino acid sequence at least 90% identical to SEQ ID NO: 127.
81. A protein of embodiment 80, wherein the heavy chain variable domain of the second antigen-binding site comprises an amino acid sequence including:
   a heavy chain CDR1 sequence identical to the amino acid sequence of SEQ ID NO: 124;
   a heavy chain CDR2 sequence identical to the amino acid sequence of SEQ ID NO: 125; and
   a heavy chain CDR3 sequence identical to the amino acid sequence of SEQ ID NO: 126.
82. A protein according to embodiment 81, wherein the light chain variable domain of the second antigen-binding site comprises an amino acid sequence including:
   a light chain CDR1 sequence identical to the amino acid sequence of SEQ ID NO: 128;
   a light chain CDR2 sequence identical to the amino acid sequence of SEQ ID NO: 129; and
   a light chain CDR3 sequence identical to the amino acid sequence of SEQ ID NO:130.
83. A protein of any one of embodiments 1, 2, or 61-72, wherein the second antigen-binding site binds EpCAM, the heavy chain variable domain of the second antigen-binding site comprises an amino acid sequence at least 90% identical to SEQ ID NO: 131 and the light chain variable domain of the second antigen-binding site comprises an amino acid sequence at least 90% identical to SEQ ID NO: 135.
84. A protein of embodiment 83, wherein the heavy chain variable domain of the second antigen-binding site comprises an amino acid sequence including:
   a heavy chain CDR1 sequence identical to the amino acid sequence of SEQ ID NO:132;
   a heavy chain CDR2 sequence identical to the amino acid sequence of SEQ ID NO: 133; and
   a heavy chain CDR3 sequence identical to the amino acid sequence of SEQ ID NO:134.
85. A protein of embodiment 84, wherein the light chain variable domain of the second antigen-binding site comprises an amino acid sequence including:
   a light chain CDR1 sequence identical to the amino acid sequence of SEQ ID NO:136;
   a light chain CDR2 sequence identical to the amino acid sequence of SEQ ID NO: 137; and
   a light chain CDR3 sequence identical to the amino acid sequence of SEQ ID NO:138.
86. A protein of any one of embodiments 1, 2, or 61-72, wherein the second antigen-binding site binds EpCAM, the heavy chain variable domain of the second antigen-binding site comprises an amino acid sequence at least 90% identical to SEQ ID NO: 139 and the light chain variable domain of the second antigen-binding site comprises an amino acid sequence at least 90% identical to SEQ ID NO: 143.
87. A protein of embodiment 86, wherein the heavy chain variable domain of the second antigen-binding site comprises an amino acid sequence including:
   a heavy chain CDR1 sequence identical to the amino acid sequence of SEQ ID NO: 140;
   a heavy chain CDR2 sequence identical to the amino acid sequence of SEQ ID NO: 141; and
   a heavy chain CDR3 sequence identical to the amino acid sequence of SEQ ID NO: 142.
88. A protein of embodiment 87, wherein the light chain variable domain of the second antigen-binding site comprises an amino acid sequence including:
   a light chain CDR1 sequence identical to the amino acid sequence of SEQ ID NO: 144;
   a light chain CDR2 sequence identical to the amino acid sequence of SEQ ID NO: 145; and
   a light chain CDR3 sequence identical to the amino acid sequence of SEQ ID NO: 146.
89. A protein of any one of embodiments 1, 3, or 61-72, wherein the second antigen-binding site binds CA125, the heavy chain variable domain of the second antigen-binding site comprises an amino acid sequence at least 90% identical to SEQ ID NO: 155 and the light chain variable domain of the second antigen-binding site comprises an amino acid sequence at least 90% identical to SEQ ID NO: 159.
90. A protein of any one of embodiments 1, 3, or 61-72, wherein the second antigen-binding site binds CA125, the heavy chain variable domain of the second antigen-binding site comprises an amino acid sequence at least 90% identical to SEQ ID NO: 163 and the light chain variable domain of the second antigen-binding site comprises an amino acid sequence at least 90% identical to SEQ ID NO: 167.
91. A protein of any one of embodiments 1, 4, or 61-72, wherein the second antigen-binding site binds NaPi2b, the heavy chain variable domain of the second antigen-binding site comprises an amino acid sequence at least 90% identical to SEQ ID NO: 171 and the light chain variable domain of the second antigen-binding site comprises an amino acid sequence at least 90% identical to SEQ ID NO: 175.
92. A protein of any one of embodiments 1, 5, or 61-72, wherein the second antigen-binding site binds Nectin4, the heavy chain variable domain of the second antigen-binding site comprises an amino acid sequence at least 90% identical to SEQ ID NO: 179 and the light chain variable domain of the second antigen-binding site comprises an amino acid sequence at least 90% identical to SEQ ID NO:183.
93. A protein of any one of embodiments 1, 6, or 61-72, wherein the second antigen-binding site binds fucosyl-GM1, the heavy chain variable domain of the second antigen-binding site comprises an amino acid sequence at least 90% identical to SEQ ID NO: 187 and the light chain variable domain of the second antigen-binding site comprises an amino acid sequence at least 90% identical to SEQ ID NO: 191.
94. A protein of any one of embodiments 1, 7, or 61-72, wherein the second antigen-binding site binds SLC44A4, the heavy chain variable domain of the second antigen-binding site comprises an amino acid sequence at least 90% identical to SEQ ID NO: 195 and the light chain variable domain of the second antigen-binding site comprises an amino acid sequence at least 90% identical to SEQ ID NO: 199.
95. A protein according to any one of embodiments 1-94, wherein the antibody Fc domain comprises hinge and CH2 domains of a human IgG1 antibody.
96. A protein of embodiment 95, wherein the Fc domain comprises an amino acid sequence at least 90% identical to amino acids 234-332 of a human IgG1 antibody.
97. A protein of embodiment 96, wherein the Fc domain comprises amino acid sequence at least 90% identical to the Fc domain of human IgG1 and differs at one or more positions selected from the group consisting of Q347, Y349, L351, S354, E356, E357, K360, Q362, S364, T366, L368, K370, N390, K392, T394, D399, S400, D401, F405, Y407, K409, T411, K439.
98. A protein according to any one of embodiments 1-96, wherein the protein binds to NKG2D with a K_{D} of 10 nM or weaker affinity.
99. A formulation comprising a protein according to any one of the preceding embodiments and a pharmaceutically acceptable carrier.
100. A cell comprising one or more nucleic acids expressing a protein according to any one of embodiments 1-98.
101. A method of directly and/or indirectly enhancing tumor cell death, the method comprising exposing a tumor and natural killer cells to a protein according to any one of embodiments 1-98.
102. A method of treating cancer, wherein the method comprises administering a protein according to any one of embodiments 1-98 or a formulation according to embodiment 99 to a patient.
103. The method of embodiment 102, wherein when the second binding site binds EpCAM, the cancer is selected from the group consisting of head and neck cancer, ovarian cancer, bladder cancer, breast cancer, colorectal cancer, prostate cancer, gastric cancer, liver cancer, esophageal cancer, and lung cancer.

## Claims

1. A protein comprising:
(a) a first antigen-binding site that binds Natural killer group 2 member D (NKG2D);
(b) a second antigen-binding site that binds an antigen selected from the group consisting of: EpCAM, Cancer Antigen 125 (CA125), sodium/phosphate cotransporter 2B (NaPi2b), Fucosyl-GM1 (monosialotetrahexosylganglioside), disintegrin and metalloproteinase domain-containing protein 8 (ADAM8), disintegrin and metalloproteinase domain-containing protein 9 (ADAM9), solute carrier family 44 member 4 (SLC44A4), and sialylated Lewis a antigen (CA19-9); and
(c) an antibody Fc domain or a portion thereof sufficient to bind cluster of differentiation 16 (CD16), or a third antigen-binding site that binds CD16.

2. A protein comprising:
i)
(a) a first antigen-binding site that binds NKG2D;
(b) a second antigen-binding site that binds EpCAM; and
(c) an antibody Fc domain or a portion thereof sufficient to bind CD16, or a third antigen-binding site that binds CD16,
ii)
(a) a first antigen-binding site that binds NKG2D;
(b) a second antigen-binding site that binds a tumor associated antigen selected from Cancer Antigen 125 (CA125), sodium/phosphate cotransporter 2B (NaPi2b), Fucosyl-GM1 (monosialotetrahexosylganglioside), disintegrin and metalloproteinase domain-containing protein 8 (ADAM8), disintegrin and metalloproteinase domain-containing protein 9 (ADAM9), solute carrier family 44 member 4 (SLC44A4), and sialylated Lewis a antigen (CA19-9); and
(c) an antibody Fc domain or a portion thereof sufficient to bind CD16, or a third antigen-binding site that binds CD16,
iii)
(a) a first antigen-binding site that binds NKG2D;
(b) a second antigen-binding site that binds a tumor associated antigen selected from sodium-dependent phosphate transport protein 2b (NaPi2b); and
(c) an antibody Fc domain or a portion thereof sufficient to bind CD16, or a third antigen-binding site that binds CD 16,
iv)
(a) a first antigen-binding site that binds NKG2D;
(b) a second antigen-binding site that binds a multiple myeloma associated antigen Fucosyl-GM1; and
(c) an antibody Fc domain or a portion thereof sufficient to bind CD16, or a third antigen-binding site that binds CD16, or
v)
(a) a first antigen-binding site that binds NKG2D;
(b) a second antigen-binding site that binds a T-cell associated tumor antigen SLC44A4; and
(c) an antibody Fc domain or a portion thereof sufficient to bind CD16, or a third antigen-binding site that binds CD16.

3. The protein of any one of claims 1-2, wherein the first antigen-binding site binds to NKG2D in humans and non-human primates.

4. The protein of any one of claims 1-3, wherein the first antigen-binding site comprises a heavy chain variable domain and a light chain variable domain, optionally wherein the heavy chain variable domain and the light chain variable domain are present on the same polypeptide.

5. A protein according to claim 4, wherein the second antigen-binding site comprises a heavy chain variable domain and a light chain variable domain, optionally wherein
the heavy chain variable domain and the light chain variable domain of the second antigen-binding site are present on the same polypeptide, and/or
the light chain variable domain of the first antigen-binding site has an amino acid sequence identical to the amino acid sequence of the light chain variable domain of the second antigen-binding site.

6. A protein comprising:
(a) a first antigen-binding site comprising an Fab fragment that binds NKG2D;
b) a second antigen-binding site comprising a single-chain variable fragment (scFv) that binds EpCAM; and
(c) an antibody Fc domain or a portion thereof sufficient to bind CD16, or a third antigen-binding site that binds CD 16.

7. The protein of claim 6, wherein the scFv is linked to the antibody Fc domain or a portion thereof sufficient to bind CD 16, or the third antigen-binding site that binds CD 16, via a hinge comprising Ala-Ser or Gly-Ala-Ser, wherein the scFv comprises a heavy chain variable domain and a light chain variable domain, optionally wherein the scFv is linked to the antibody Fc domain.

8. The protein of claim 7, wherein the heavy chain variable domain of the scFv forms a disulfide bridge with the light chain variable domain of the scFv,
optionally wherein the disulfide bridge is formed between C44 from the heavy chain variable domain and C100 from the light chain variable domain,
further optionally wherein the scFv is linked to the antibody Fc domain, wherein the light chain variable domain of the scFv is positioned at the N-terminus of the heavy chain variable domain of the scFv, and is linked to the heavy chain variable domain of the scFv via a flexible linker (GlyGlyGlyGlySer)4 ((G4S)4), and the Fab is linked to the antibody Fc domain.

9. A protein according to any one of claims 7-8, wherein the heavy chain variable domain of the scFv is linked to the light chain variable domain of the scFv via a flexible linker, optionally wherein the flexible linker comprises (GlyGlyGlyGlySer)4 ((G4S)4).

10. A protein according to any one of claims 7-9, wherein the heavy chain variable domain of the scFv is positioned at the N-terminus or the C-terminus of the light chain variable domain of the scFv, optionally wherein the light chain variable domain of the scFv is positioned at the N-terminus of the heavy chain variable domain of the scFv.

11. A protein according to any one of claims 6-10, wherein the Fab fragment is linked to the antibody Fc domain or a portion thereof sufficient to bind CD16 or the third antigen-binding site that binds CD16, optionally wherein the heavy chain portion of the Fab fragment comprises a heavy chain variable domain and a CH1 domain, and wherein the heavy chain variable domain is linked to the CH1 domain, and/or wherein the Fab fragment is linked to the antibody Fc domain.

12. A protein according to any one of claims 6-11 comprising an amino acid sequence selected from SEQ ID NO:208 and SEQ ID NO:209.

13. A protein according to any one of claims 7-12, comprising:
a) an scFv linked to an antibody Fc domain, wherein the scFv linked to the antibody Fc domain is represented by an amino acid sequence selected from SEQ ID NO:210 and SEQ ID NO:211, or
b) an amino acid sequence at least 90%, 95% or 99% identical to an amino acid sequence selected from SEQ ID NO:210 and SEQ ID NO:211.

14. A protein according to any one of claims 7-12, comprising:
a) a sequence selected from SEQ ID NO:212 and SEQ ID NO:213 or
b) an amino acid sequence at least 90%, 95% or 99% identical to an amino acid sequence selected from SEQ ID NO:212 and SEQ ID NO:213.

15. A protein comprising:
(a) a first antigen-binding site comprising a single-chain variable fragment (scFv) that binds NKG2D;
(b) a second antigen-binding site that binds EpCAM; and
(c) an antibody Fc domain or a portion thereof sufficient to bind CD16, or a third antigen-binding site that binds CD 16.

16. A protein according to claim 15 further comprising an additional antigen-binding site that binds EpCAM

17. The protein according to claim 15 or 16, wherein the second antigen-binding site that binds EpCAM is an Fab fragment.

18. The protein according to claim 16 or 17, wherein the second and the additional antigen-binding site that bind EpCAM are Fab fragments.

19. The protein according to claim 16 or 17, wherein the second and the additional antigen-binding site that bind EpCAM are scFvs.

20. The protein according to any one of claims 15-19, wherein the heavy chain variable domain of the scFv that binds NKG2D is positioned at the N-terminus or the C-terminus of the light chain variable domain of the scFv, optionally wherein the light chain variable domain is positioned at the N-terminus of the heavy chain variable domain of the scFv that binds NKG2D.

21. The protein according to any one of claims 15-20, wherein the scFv that binds to NKG2D is linked to the antibody Fc domain or a portion thereof sufficient to bind CD16, or a third antigen-binding site that binds CD16,
optionally wherein
a) the scFv that binds to NKG2D is linked to the antibody Fc domain or a portion thereof sufficient to bind CD16, or a third antigen-binding site that binds CD16 via a hinge comprising Ala-Ser or Gly-Ala-Ser,
b) the scFv that binds to NKG2D is linked to the C-terminus of the antibody Fc domain or a portion thereof sufficient to bind CD 16, or a third antigen-binding site that binds CD 16 via a flexible linker comprising SGSGGGGS (SEQ ID NO: 207), or
c) the C-terminus of the antibody Fc domain is linked to the N-terminus of the light chain variable domain of the scFv that binds NKG2D.

22. The protein according to any one of claims 15-21, wherein within the scFv that binds NKG2D, a disulfide bridge is formed between the heavy chain variable domain of the scFv and the light chain variable domain of the scFv, optionally wherein the disulfide bridge is formed between C44 from the heavy chain variable domain and C100 from the light chain variable domain.

23. The protein according to any one of claims 15-22, wherein, within the scFv that binds NKG2D, the heavy chain variable domain is linked to the light chain variable domain via a flexible linker, optionally wherein the flexible linker comprises (GlyGlyGlyGlySer)4 (G4S)4).

24. The protein according to any one of claims 19-23, wherein the second and the additional antigen-binding site scFvs are linked to the antibody Fc domain or a portion thereof sufficient to bind CD16, or the third antigen-binding site that binds CD16, via a hinge comprising Ala-Ser.

25. The protein according to any one of claims 19-24, wherein the second and the additional antigen-binding site scFvs are linked to the antibody Fc domain via a hinge comprising Ala-Ser.

26. The protein according to claim 24 or 25, wherein a disulfide bridge is formed between the heavy chain variable domain and the light chain variable domain of the second antigen-binding site and/or the additional antigen-binding site, optionally wherein the disulfide bridge is formed between C44 from the heavy chain variable domain and C100 from the light chain variable domain.

27. The protein according to any one of claims 15-26, wherein the scFv that binds NKG2D comprises a light chain variable domain positioned at the N-terminus of a heavy chain variable domain, wherein the light chain variable domain is linked to the heavy chain variable domain of the scFv via a flexible linker (GlyGlyGlyGlySer)4 (G4S)4), and the scFv that binds NKG2D is linked to the antibody Fc domain via a hinge comprising Ala-Ser or Gly-Ala-Ser.

28. A protein comprising an amino acid sequence
i) of SEQ ID NO:203,
ii) of SEQ ID NO:203 and SEQ ID NO:204, or
iii) at least 90%, 95% or 99% identical to an amino acid sequence of SEQ ID NO:203.

29. A protein according to any one of the preceding claims, wherein the first antigen-binding site comprises a heavy chain variable domain amino acid sequence at least 90% identical to an amino acid sequence selected from: SEQ ID NO:85, SEQ ID NO: 1, SEQ ID NO:41, SEQ ID NO:49, SEQ ID NO:57, SEQ ID NO:59, SEQ ID NO:61, SEQ ID NO:69, SEQ ID NO: 77, and SEQ ID NO: 93.

30. A protein according to any one of claims 1-29, wherein
a) the first antigen-binding site comprises a heavy chain variable domain amino acid sequence at least 90% identical to SEQ ID NO:85 and a light chain variable domain amino acid sequence at least 90% identical to SEQ ID NO:86,
b) the first antigen-binding site comprises a heavy chain variable domain amino acid sequence at least 90% identical to SEQ ID NO:41 and a light chain variable domain amino acid sequence at least 90% identical to SEQ ID NO:42,
c) the first antigen-binding site comprises a heavy chain variable domain amino acid sequence at least 90% identical to SEQ ID NO:49 and a light chain variable domain amino acid sequence at least 90% identical to SEQ ID NO:50,
d) the first antigen-binding site comprises a heavy chain variable domain amino acid sequence at least 90% identical to SEQ ID NO:57 and a light chain variable domain amino acid sequence at least 90% identical to SEQ ID NO:58,
e) the first antigen-binding site comprises a heavy chain variable domain amino acid sequence at least 90% identical to SEQ ID NO:59 and a light chain variable domain amino acid sequence at least 90% identical to SEQ ID NO:60,
f) the first antigen-binding site comprises a heavy chain variable domain amino acid sequence at least 90% identical to SEQ ID NO:61 and a light chain variable domain amino acid sequence at least 90% identical to SEQ ID NO:62,
g) the first antigen-binding site comprises a heavy chain variable domain amino acid sequence at least 90% identical to SEQ ID NO:69 and a light chain variable domain amino acid sequence at least 90% identical to SEQ ID NO:70,
h) the first antigen-binding site comprises a heavy chain variable domain amino acid sequence at least 90% identical to SEQ ID NO:77 and a light chain variable domain amino acid sequence at least 90% identical to SEQ ID NO:78,
i) the first antigen-binding site comprises a heavy chain variable domain amino acid sequence at least 90% identical to SEQ ID NO:93 and a light chain variable domain amino acid sequence at least 90% identical to SEQ ID NO:94,
j) the first antigen-binding site comprises a heavy chain variable domain amino acid sequence at least 90% identical to SEQ ID NO: 101 and a light chain variable domain amino acid sequence at least 90% identical to SEQ ID NO: 102 or
k) the first antigen-binding site comprises a heavy chain variable domain amino acid sequence at least 90% identical to SEQ ID NO: 103 and a light chain variable domain amino acid sequence at least 90% identical to SEQ ID NO: 104.

31. The protein of any one of claims 1-4, wherein the first antigen-binding site is a single-domain antibody, optionally wherein the single-domain antibody is a V_{H}H fragment or a V_{NAR} fragment.

32. A protein of any one of claims 1-4 or 31, wherein the second antigen-binding site comprises a heavy chain variable domain and a light chain variable domain, optionally wherein the heavy chain variable domain and the light chain variable domain of the second antigen-binding site are present on the same polypeptide.

33. A protein of any of claims 1, 2i) or 30, wherein:
a) the second antigen-binding site binds EpCAM, the heavy chain variable domain of the second antigen-binding site comprises an amino acid sequence at least 90% identical to SEQ ID NO:115 and the light chain variable domain of the second antigen-binding site comprises an amino acid sequence at least 90% identical to SEQ ID NO:119,
optionally wherein the heavy chain variable domain of the second antigen-binding site comprises an amino acid sequence including:
a heavy chain CDR1 sequence identical to the amino acid sequence of SEQ ID NO: 116;
a heavy chain CDR2 sequence identical to the amino acid sequence of SEQ ID NO: 117; and
a heavy chain CDR3 sequence identical to the amino acid sequence of SEQ ID NO: 118,
further optionally wherein the light chain variable domain of the second antigen-binding site comprises an amino acid sequence including:
a light chain CDR1 sequence identical to the amino acid sequence of SEQ ID NO: 120;
a light chain CDR2 sequence identical to the amino acid sequence of SEQ ID NO: 121;
and a light chain CDR3 sequence identical to the amino acid sequence of SEQ ID NO: 122,
b) the second antigen-binding site binds EpCAM, the heavy chain variable domain of the second antigen-binding site comprises an amino acid sequence at least 90% identical to SEQ ID NO: 123 and the light chain variable domain of the second antigen-binding site comprises an amino acid sequence at least 90% identical to SEQ ID NO: 127,
optionally wherein the heavy chain variable domain of the second antigen-binding site comprises an amino acid sequence including:
a heavy chain CDR1 sequence identical to the amino acid sequence of SEQ ID NO: 124;
a heavy chain CDR2 sequence identical to the amino acid sequence of SEQ ID NO: 125; and
a heavy chain CDR3 sequence identical to the amino acid sequence of SEQ ID NO: 126,
further optionally wherein the light chain variable domain of the second antigen-binding site comprises an amino acid sequence including:
a light chain CDR1 sequence identical to the amino acid sequence of SEQ ID NO: 128;
a light chain CDR2 sequence identical to the amino acid sequence of SEQ ID NO: 129; and
a light chain CDR3 sequence identical to the amino acid sequence of SEQ ID NO: 130,
c) the second antigen-binding site binds EpCAM, the heavy chain variable domain of the second antigen-binding site comprises an amino acid sequence at least 90% identical to SEQ ID NO: 131 and the light chain variable domain of the second antigen-binding site comprises an amino acid sequence at least 90% identical to SEQ ID NO:135,
optionally wherein the heavy chain variable domain of the second antigen-binding site comprises an amino acid sequence including:
a heavy chain CDR1 sequence identical to the amino acid sequence of SEQ ID NO: 132;
a heavy chain CDR2 sequence identical to the amino acid sequence of SEQ ID NO: 133; and
a heavy chain CDR3 sequence identical to the amino acid sequence of SEQ ID NO: 134,
further optionally wherein the light chain variable domain of the second antigen-binding site comprises an amino acid sequence including:
a light chain CDR1 sequence identical to the amino acid sequence of SEQ ID NO:136;
a light chain CDR2 sequence identical to the amino acid sequence of SEQ ID NO: 137; and
a light chain CDR3 sequence identical to the amino acid sequence of SEQ ID NO: 138, or
d) the second antigen-binding site binds EpCAM, the heavy chain variable domain of the second antigen-binding site comprises an amino acid sequence at least 90% identical to SEQ ID NO:139 and the light chain variable domain of the second antigen-binding site comprises an amino acid sequence at least 90% identical to SEQ ID NO:143,
optionally wherein the heavy chain variable domain of the second antigen-binding site comprises an amino acid sequence including:
a heavy chain CDR1 sequence identical to the amino acid sequence of SEQ ID NO: 140;
a heavy chain CDR2 sequence identical to the amino acid sequence of SEQ ID NO: 141; and
a heavy chain CDR3 sequence identical to the amino acid sequence of SEQ ID NO: 142,
further optionally wherein the light chain variable domain of the second antigen-binding site comprises an amino acid sequence including:
a light chain CDR1 sequence identical to the amino acid sequence of SEQ ID NO: 144;
a light chain CDR2 sequence identical to the amino acid sequence of SEQ ID NO: 145; and
a light chain CDR3 sequence identical to the amino acid sequence of SEQ ID NO: 146.

34. A protein of any one of claims 1, 2ii) or 30, wherein the second antigen-binding site binds CA125, the heavy chain variable domain of the second antigen-binding site comprises an amino acid sequence at least 90% identical to SEQ ID NO: 155 and the light chain variable domain of the second antigen-binding site comprises an amino acid sequence at least 90% identical to SEQ ID NO:159,
and/or
wherein the second antigen-binding site comprises:
a heavy chain CDR1 comprising the amino acid sequence of SEQ ID NO: 156;
a heavy chain CDR2 comprising the amino acid sequence of SEQ ID NO: 157;
a heavy chain CDR3 comprising the amino acid sequence of SEQ ID NO: 158;
a light chain CDR1 comprising the amino acid sequence of SEQ ID NO: 160;
a light chain CDR2 comprising the amino acid sequence of SEQ ID NO: 161; and
a light chain CDR3 comprising the amino acid sequence of SEQ ID NO: 162.

35. A protein of any one of claims 1, 2ii) or 30, wherein the second antigen-binding site binds CA125, the heavy chain variable domain of the second antigen-binding site comprises an amino acid sequence at least 90% identical to SEQ ID NO: 163 and the light chain variable domain of the second antigen-binding site comprises an amino acid sequence at least 90% identical to SEQ ID NO: 167,
and/or
wherein the second antigen-binding site comprises:
a heavy chain CDR1 comprising the amino acid sequence of SEQ ID NO: 164;
a heavy chain CDR2 comprising the amino acid sequence of SEQ ID NO: 165;
a heavy chain CDR3 comprising the amino acid sequence of SEQ ID NO: 166;
a light chain CDR1 comprising the amino acid sequence of SEQ ID NO: 168;
a light chain CDR2 comprising the amino acid sequence of SEQ ID NO: 169; and
a light chain CDR3 comprising the amino acid sequence of SEQ ID NO: 170.

36. A protein of any one of claims 1, 2iii) or 30, wherein the second antigen-binding site binds NaPi2b, the heavy chain variable domain of the second antigen-binding site comprises an amino acid sequence at least 90% identical to SEQ ID NO: 171 and the light chain variable domain of the second antigen-binding site comprises an amino acid sequence at least 90% identical to SEQ ID NO: 175,
and/or
wherein the second antigen-binding site comprises:
a heavy chain CDR1 comprising the amino acid sequence of SEQ ID NO: 172;
a heavy chain CDR2 comprising the amino acid sequence of SEQ ID NO: 173;
a heavy chain CDR3 comprising the amino acid sequence of SEQ ID NO: 174;
a light chain CDR1 comprising the amino acid sequence of SEQ ID NO: 176;
a light chain CDR2 comprising the amino acid sequence of SEQ ID NO: 177; and
a light chain CDR3 comprising the amino acid sequence of SEQ ID NO: 178.

37. A protein of any one of claims 1, 2iv) or 30, wherein the second antigen-binding site binds fucosyl-GM1, the heavy chain variable domain of the second antigen-binding site comprises an amino acid sequence at least 90% identical to SEQ ID NO: 187 and the light chain variable domain of the second antigen-binding site comprises an amino acid sequence at least 90% identical to SEQ ID NO: 191,
and/or
wherein the second antigen-binding site comprises:
a heavy chain CDR1 comprising the amino acid sequence of SEQ ID NO: 188;
a heavy chain CDR2 comprising the amino acid sequence of SEQ ID NO: 189;
a heavy chain CDR3 comprising the amino acid sequence of SEQ ID NO: 190;
a light chain CDR1 comprising the amino acid sequence of SEQ ID NO: 192;
a light chain CDR2 comprising the amino acid sequence of SEQ ID NO: 193; and
a light chain CDR3 comprising the amino acid sequence of SEQ ID NO: 194.

38. A protein of any one of claims 1, 2v) or 30, wherein the second antigen-binding site binds SLC44A4, the heavy chain variable domain of the second antigen-binding site comprises an amino acid sequence at least 90% identical to SEQ ID NO: 195 and the light chain variable domain of the second antigen-binding site comprises an amino acid sequence at least 90% identical to SEQ ID NO:199,
and/or
wherein the second antigen-binding site comprises:
a heavy chain CDR1 comprising the amino acid sequence of SEQ ID NO: 196;
a heavy chain CDR2 comprising the amino acid sequence of SEQ ID NO: 197;
a heavy chain CDR3 comprising the amino acid sequence of SEQ ID NO: 198;
a light chain CDR1 comprising the amino acid sequence of SEQ ID NO:200;
a light chain CDR2 comprising the amino acid sequence of SEQ ID NO:201; and
a light chain CDR3 comprising the amino acid sequence of SEQ ID NO:202.

39. A protein according to any one of the preceding, wherein the antibody Fc domain comprises hinge and CH2 domains of a human IgG1 antibody, optionally wherein the Fc domain comprises an amino acid sequence at least 90% identical to amino acids 234-332 of a human IgG1 antibody, and further optionally wherein the Fc domain comprises an amino acid sequence at least 90% identical to the Fc domain of human IgG1 and differs at one or more positions selected from the group consisting of Q347, Y349, L351, S354, E356, E357, K360, Q362, S364, T366, L368, K370, N390, K392, T394, D399, S400, D401, F405, Y407, K409, T411, and K439.

40. A protein according to any one of the preceding claims, wherein the protein binds to NKG2D with a K_{D} of 10 nM or weaker affinity.

41. A formulation comprising a protein according to any one of the preceding claims and a pharmaceutically acceptable carrier.

42. A cell comprising one or more nucleic acids encoding a protein according to any one of claims 1-40.

43. A protein according to any one of claims 1-40,
a) for use in therapy, or
b) for use in a method of directly and/or indirectly enhancing tumor cell death, the method comprising exposing a tumor cell and a natural killer cell to the protein.

44. A protein according to any one of claims 1-40, or a formulation according to claim 44, for use in a method of treating cancer, wherein the method comprises administering the protein or the formulation to a patient.

45. The protein or the formulation for the use of claim 44, wherein:
a) when the second binding site binds EpCAM, the cancer is selected from the group consisting of head and neck cancer, ovarian cancer, bladder cancer, breast cancer, colorectal cancer, prostate cancer, gastric cancer, liver cancer, esophageal cancer, and lung cancer,
b) the cancer is selected from the group consisting of bladder cancer, breast cancer, ovarian cancer, pancreatic cancer, colorectal cancer, and lung cancer, or
c) the cancer is selected from the group consisting of ovarian cancer, endometrial cancer, pancreatic cancer, lung cancer, thyroid cancer, bladder cancer, breast cancer, colorectal cancer, small cell lung cancer, neuroblastoma, liver cancer, renal cancer, melanoma, cervical cancer, prostate cancer, osteosarcoma, brain cancer, gastric cancer, and cholangiocarcinoma.
